(19) Europäisches Patentamt
European Patent Office
Office européen des brevets

(11) EP 1 530 637 B1

(12) **EUROPEAN PATENT SPECIFICATION**

(45) Date of publication and mention of the grant of the patent:
**10.02.2010 Bulletin 2010/06**

(21) Application number: **03711261.2**

(22) Date of filing: **26.02.2003**

(51) Int Cl.:
***C12N 15/12*** *(2006.01)* ***C12N 5/10*** *(2006.01)*
***C12P 21/02*** *(2006.01)* ***C12N 9/56*** *(2006.01)*

(86) International application number:
**PCT/US2003/005861**

(87) International publication number:
**WO 2003/072746 (04.09.2003 Gazette 2003/36)**

(54) **SUBTILISIN CARLSBERG PROTEINS WITH REDUCED IMMUNOGENICITY**

SUBTILISIN-CARLSBERG-PROTEINE MIT VERMINDERTER IMMUNOGENITÄT

PROTEINES DE LA SUBTILISINE (CARLSBERG) PRESENTANT UNE ANTIGENICITE REDUITE

(84) Designated Contracting States:
**AT BE BG CH CY CZ DE DK EE ES FI FR GB GR HU IE IT LI LU MC NL PT SE SI SK TR**

(30) Priority: **26.02.2002 US 360057 P**
**30.05.2002 US 384777 P**

(43) Date of publication of application:
**18.05.2005 Bulletin 2005/20**

(73) Proprietor: **GENENCOR INTERNATIONAL, INC.**
**Palo Alto, California 94304 (US)**

(72) Inventor: **HARDING, Fiona, A**
**Santa Clara, CA 95050 (US)**

(74) Representative: **Kiddle, Simon John et al**
**Mewburn Ellis LLP**
**33 Gutter Lane**
**London**
**EC2V 8AS (GB)**

(56) References cited:
**WO-A2-99/53038** **WO-A2-99/53078**
**WO-A2-02/069232** **US-A- 5 677 163**

- **STICKLER M M ET AL: "CD4+ T-CELL EPITOPE DETERMINATION USING UNEXPOSED HUMAN DONOR PERIPHERAL BLOOD MONONUCLEAR CELLS" JOURNAL OF IMMUNOTHERAPY, LIPPINCOTT WILLIAMS & WILKINS, HAGERSTOWN, MD, US, vol. 23, no. 6, November 2000 (2000-11), pages 654-660, XP008040520 ISSN: 1524-9557**
- **STICKLER M.M. ET AL.: 'CD4 T-CELL determination using unexposed huamn donor peripheral blood mononuclear cells' JOURNAL OF IMMUNOTHERAPY vol. 23, no. 6, 05 July 2000, pages 654 - 660, XP008040520**

EP 1 530 637 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

### FIELD OF THE INVENTION

The present invention relates to methods for the identification of $CD4^+$ T-cell epitopes in subtilisin Carlsberg proteins. The present invention also provides for the production of altered peptides which, when incorporated into a wild-type subtilisin Carlsberg protein produce an altered immunog enic response, which is a lowered immunogenic response in humans. In particular, the present inv ention provides means, including methods and compositions suitable for reducing the immunogenicity of ALCALASE® enzyme.

### BACKGROUND OF THE INVENTION

Proteins used in industrial, pharmaceutical and commercial applications are of increasing prevalence and importance. However, this has resulted in the sensitization of numerous individuals to these proteins, resulting in the widespread occurrence of allergic reactions to these proteins. For example, some proteases are associated with hypersensitivity reactions in certain individuals. As a result, despite the usefulness of proteases in industry (*e.g.*, in laundry detergents, cosmetics, textile treatment etc.), as well as the extensive research performed in the field to provide improved proteases (*e.g.*, variant subtilisin Carlsberg enzymes with more effective stain removal under typical laundry conditions), the use of proteases in industry has been problematic.

Much work has been done to alleviate these problems. Strateg ies explored to reduce immunogenic potential of protease use include improved production processes which reduce potential contact by controlling and minimizing workplace concentrations of dust particles and/or aerosol carry ing airborne protease, improved granulation processes which reduce the amount of dust or aerosol actually produced from the protease product, and improved recovery processes to reduce the level of potentially allergenic/immunogenic contaminants in the final product. However, efforts to reduce the allergenicity/immunogenicity of proteases themselves have been relatively unsuccessful. Alternatively, efforts have been made to mask epitopes in protease which are recognized by immunoglobulin E (IgE) in hypersensitive individuals (See, PCT Publication No. WO 92/10755; WO 94/10191; WO 96/17929; WO 99/49056; and WO 01/07578), or to enlarge or change the nature of the antigenic determinants by attaching polymers or peptides/proteins to the problematic protease.

While some studies have provided methods of reducing the allergenicity/immunogenicity of certain proteins and identification of epitopes which cause allergic reactions in some individuals, the assays used to identify these epitopes generally involve measurement of IgE and IgG in the sera of those who have been previously exposed to the antigen. However, once an Ig reaction has been initiated, sensitization has already occurred. Accordingly, there is a need to identify proteins which produce an enhanced immunologic response, as well as a need to produce proteins which produce a reduced immunologic response.

WO 99/53038 and WO 99/53078 describe a method of T-cell epitope mapping and reducing the immunogenicity of a protein. WO 02/069232 also describes a method for identification of T-cell epitopes and use for preparing molecules with reduced immunogenicity.

### SUMMARY OF THE INVENTION

The present invention relates to methods for the identification of $CD4^+$ T-cell epitopes in subtilisin Carlsberg proteins. The present invention provides for the production of altered peptides which when incorporated into a wild-type subtilisin Carlsberg protein produce an altered immunogenic response, which is a lowered immunogenic response in humans. In particular, the present invention provides means, including methods and compositions suitable for reducing the immunogenicity of ALCALASE® enzyme. The present invention also provides for the production of altered peptides which when incorporated into a wild-type subtilisin Carlsberg protein sequence, are no longer capable of initiating the $CD4^+$ T-cell response or at least reduce the allergic response. In particular, the present invention provides means, including methods and compositions suitable for reducing the immunogenicity of a subtilisin as defined in the claims.

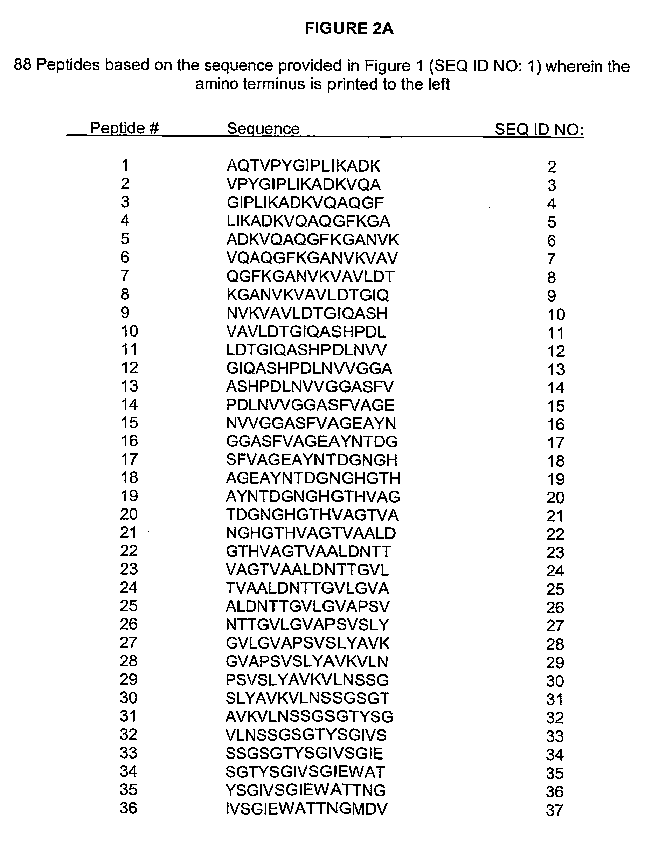

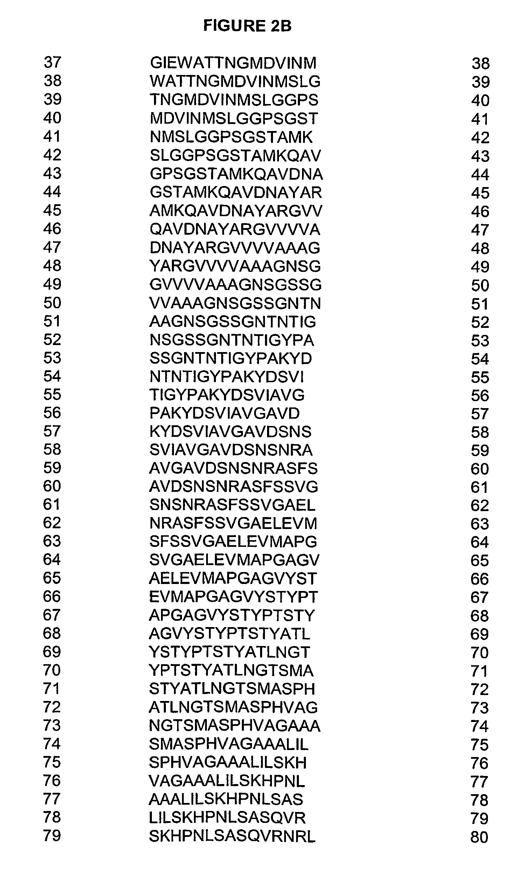

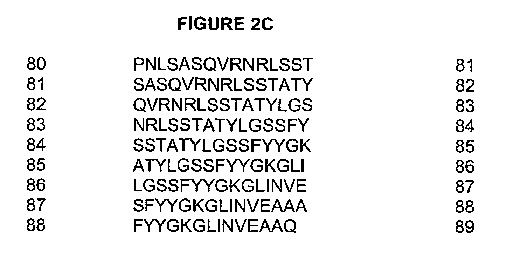

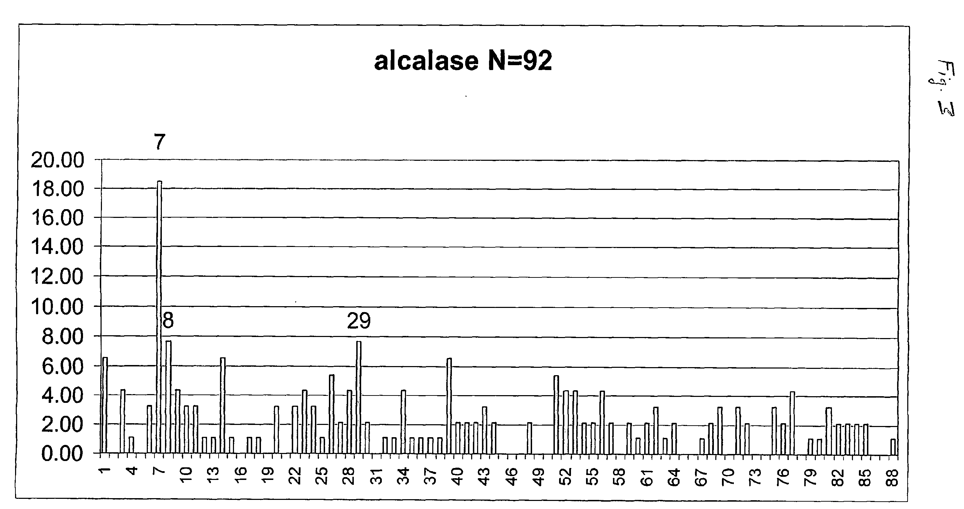

Described herein are T-cell epitopes.of the ALCALASE® enzyme. These epitopes are in various sequences set forth herein (See, Figures 2 and 3) and include but are not limited to peptide number 5 (SEQ ID NO. 6); peptide number 26 (SEQ ID NO: 27); peptide number 37 (SEQ ID NO: 38); peptide number 50 (SEQ ID NO: 51); peptide number 51 (SEQ ID NO: 52) and peptide number 79 (SEQ ID NO: 80). The present invention provides altered sequences of the identified epitopes which are suitable for substitution into the ALCALASE@ enzyme.

Also described herein are assay systems for identification of T-cell epitopes and T-cell non-epitopes, including but not limited to methods having the steps of combining differentiated dendritic cells with human $CD4^+$ and/or $CD8^+$ T-cells and with a peptide of interest (*e.g.*, peptides derived from the ALCALASE® enzyme). More specifically, peptides of interest that produce a reduced immunogenic response are provided, wherein a T-cell epitope is recognized comprising

the steps of: (a) obtaining from a single blood source a solution of dendritic cells and a solution of $CD4^+$ and/or $CD8^+$ T-cells; (b) promoting differentiation of the dendritic cells; (c) combining the solution of differentiated dendritic cells, $CD4^+$ T-cells and/or $CD8^+$ T-cells with a peptide of interest (*e.g.*, a peptide comprising at least a portion of the ALCALASE®); and (d) measuring the proliferation of the T-cells in step (c). (See *e.g.*, WO99/53038; and Strickler et al., J. Immunother., 23:654 - 660 [2000])

Also described herein are a series of peptide oligomers which correspond to all or parts of the ALCALASE® enzyme. For example, a peptide library is produced covering the relevant portion or all of the ALCALASE® enzyme. One manner of producing the peptides is to introduce overlap into the peptide library, for example, producing a first peptide which corresponds to amino acid sequence 1-15 of the ALCALASE® enzyme, a second peptide which corresponds to amino acid sequence 4-18 of the ALCALASE® enzyme, a third peptide which corresponds to amino acid sequence 7-21 of the ALCALASE@ enzyme, a fourth peptide which corresponds to amino acid sequence 10-24 of the ALCALASE® enzyme, etc. until representative peptides corresponding to the entire ALCALASE@ enzyme molecule are created. By analyzing each of the peptides individually in the assay provided herein, it is possible to precisely identify the location of epitopes recognized by T-cells. In the example above, the greater reaction of one specific peptide as compared to its neighbors facilitates identification of the epitope anchor region to within three amino acids. After determining the location of these epitopes, it is possible to alter one or more of the amino acids within each epitope. The modified epitope(s) may then be reincorporated into the backbone of the wild-type subtilisin Carlsberg protein. The resulting modified subtilisin Carlsberg proteins produces a different T-cell response, preferably a reduced T-cell response, as compared to that produced by the original wild-type protein. Also described herein are means for the identification of proteins which naturally have desired low T-cell epitope potency and may find use in their naturally occurring forms.

Various *in vitro* and *in vivo* assays known in the art may be used to ascertain the reduced immunogenic response of modified proteins according to the invention. *In vivo* assays include, but are not limited to HLA class II responses and more specifically to HLA-DR3/DQ2 mouse T-cell responses. Suitable *in vitro* assays include, but are not limited to human peripheral blood mononuclear cell (PBMC) assays (See *e.g.*, Herman et al., J. Immunol., 163:6275-6282 [1999]; Sondergaard et al., Immunol. Rev., 172: 335-343 [1999]; Taneja and David, Immunol. Rev., 169:67-79 [1999]; Taurog et al., Immunol. Rev. 169:209 -223 [1999]; Cosgrove, et al., Cell 66:1051-1066 [1991]; and Grusby et al., Proc. Natl. Acad. Sci., 90:3913-3917 [1993]).

The present invention further provides modified subtilisin Carlsberg compositions with reduced immunogenicity. In particular, the present invention provides ALCALASE@ enzyme compositions that comprise epitopes described herein that reduce the immunogenic response to the ALCALASE® enzyme.

Described herein are methods for identifying at least one T-cell epitope of a microbial subtilisin, comprising the steps of: (i) obtaining from a from a single human blood source, a solution of dendritic cells and a solution of naïve CD4+ and/or CD8+ T-cells; (ii) differentiating the dendritic cells to produce a solution of differentiated dendritic cells; (iii) combining the solution of differentiated dendritic cells and naïve CD4+ and/or CD8+ T-cells with peptide fragments of the subtilisin Carlsberg; and (iv) measuring proliferation of the T-cells in step (iii). The microbial subtilisin Carlsberg may be derived from a member of the genus *Bacillus.* In particular, the *Bacillus* may be selected from the group consisting of *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis*. Microbial subtilisin Carlsberg comprises at least a portion of the sequence set forth in SEQ ID NO:1.

Described herein are methods for reducing the immunogenicity of a microbial subtilisin Carlsberg, comprising the steps of: (a) identifying at least one T-cell epitope in the protein by (i) contacting an adherent monocyte-derived dendritic cell that has been differentiated by exposure to at least one cytokine *in vitro,* with at least one peptide comprising a T-cell epitope; and (ii) contacting the dendritic cell and peptide with a naïve T-cell, wherein the naïve T-cell has been obtained from the same source as the adherent monocyte-derived dendritic cell, and whereby the T-cell proliferates in response to the peptide; and (b) modifying the subtilisin Carlsberg to neutralize the T-cell epitope to produce a variant protein, such that the variant protein induces less than or substantially equal to the baseline proliferation of the naïve T-cells. The microbial subtilisin Carlsberg may be derived from a member of the genus *Bacillus.* In particular, the *Bacillus* may be selected from the group consisting of *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis*. Microbial subtilisin Carlsberg comprises at least a portion of the sequence set forth in SEQ ID NO: 1. The epitope of the microbial subtilisin Carlsberg may be modified by substituting the amino acid sequence of the T-cell epitope with an analogous sequence from a homolog of the microbial subtilisin, wherein the substitution substantially mimics the major tertiary structure attributes of the T-cell epitope. The microbial subtilisin Carlsberg may be modified by altering at least one epitope selected from the group consisting of SEQ ID NO:2, SEQ ID NO:90, SEQ ID NO:15, SEQ ID NO:30, and SEQ ID NO:40. The epitope may be modified by substituting an amino acid sequence for a residue corresponding to at least one of the epitopes. The epitope may be modified by deleting an amino acid sequence for a residue corresponding to at least one of the epitopes. The epitope may be modified by adding an amino acid to at least one of the epitopes.

The present invention also provides modified subtilisin Carlsberg enzymes as defined in the claims (*i.e.*, variant subtilisin Carlsberg enzymes). In some preferred embodiments, these variant subtilisin Carlsberg enzymes comprise at least one alteration in at least one epitope comprising an amino acid sequence selected from the group consisting of SEQ ID NO:2, SEQ ID NO:90, SEQ ID NO:15 and SEQ ID NO:30. Indeed, the present invention provides numerous variant subtilisin Carlsberg enzymes. These variant enzymes exhibit reduced immunogenicity/allergenicity as compared to wild-type subtilisin Carlsberg enzymes. These variant enzymes find use in numerous products and methods, ranging from personal/consumer care items to industrial production and use.

## BRIEF DESCRIPTION OF THE FIGURES

Figure 1 provides the mature protein sequence of subtilisin Carlsberg as found in the ALCALASE® enzyme (SEQ ID NO: 1). In this Figure, the mature polypeptide start at position 106 is indicated by the underlined residues (AQTVP).
Figure 2 provides the amino acid sequences of peptide numbers 1- 88 corresponding to SEQ ID NOS: 2 - 89, respectively, of the peptides synthesized based on the sequence of SEQ ID NO: 1.
Figure 3 provides the assay results for the peptides set forth in Figure 2.

## DESCRIPTION OF THE INVENTION

The present invention relates to methods for the identification of $CD4^+$ T-cell epitopes in subtilisin Carlsberg proteins. The present invention also provides for the production of altered peptides which, when incorporated into a wild-type subtilisin Carlsberg protein produce an altered immunogenic response, preferably a low immunogenic response in humans. In particular, the present invention provides means, including methods and compositions suitable for reducing the immunogenicity of ALCALASE® enzyme. The present invention also provides for the production of altered peptides which when incorporated into a wild-type subtilisin Carlsberg protein sequence, are no longer capable of initiating the $CD4^+$ T-cell response or at least reduce the allergic response. In particular, the present invention provides means, including methods and compositions suitable for reducing the immunogenicity of a wild-type subtilisin Carlsberg.

### Definitions

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionaries of many of the terms used in the invention. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular "a", "an" and "the" includes the plural reference unless the context clearly indicates otherwise.

As used herein, "the genus *Bacillus*" includes all members known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus*, and *B. thuringiensis*. It is recognized that the genus *Bacillus* continues to undergo taxonomical reorganization. Thus, it is intended that the genus include species that have been reclassified, including but not limited to such organisms as *B. stearothermophilus*, which is now named "*Geobacillus stearothermophilus*." The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus*.

The term "wild-type subtilisin Carlsberg" as used herein includes: subtilisin Carlsberg proteins having similar catalytic activity to ALCALASE® and having at least about 80%, 85%, 90%, 95%, 97%, 98% or 99% amino acid sequence identity to SEQ ID NO: 1, and preferably at least 90% amino acid identity to SEQ ID NO: 1.

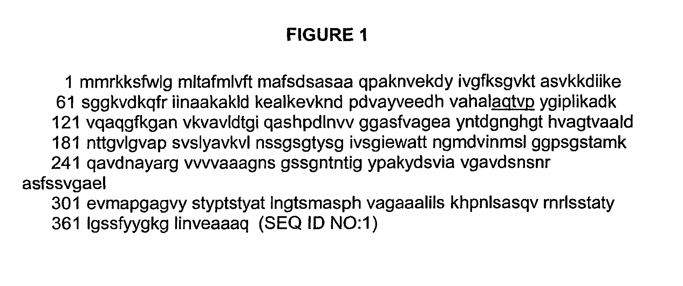

The term "ALCALASE®" as used herein refers to the serine protease, subtilisin Carlsberg derived from *Bacillus licheniformis* having SwissProt Accession number P00780. The total protein has 379 amino acid residues. The preprotein includes 105 amino acid residues and the mature protein comprises 274 amino acid residues (See, Figure 1, SEQ ID NO: 1; *See* also, Jacobs et al., Nucleic Acids Res., 13: 8913-8926 [1985]; and Smith et al., J. Biol. Chem., 243: 2184-2191 [1968]).

As used herein, the terms "modified wild-type subtilisin Carlsberg"; "modified ALCALASE®" and "modified

variant" refer to a protein in which at least one significant T-cell epitope of the wild-type subtilisin Carlsberg and particularly ALCALASE@ has been altered.

As used herein, "wild-type" and "native" proteins are those found in nature. The terms "wild-type sequence," and "wild-type gene" are used interchangeably herein, to refer to a sequence that is native or naturally occurring in a host cell.. In some embodiments, the wild-type sequence refers to a sequence of interest that is the starting point of a protein engineering project. The genes encoding the naturally-occurring (*i.e.*, precursor) protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.

"Recombinant," "recombinant subtilisin" and "recombinant protease" refer to a protein, subtilisin or protease in which the DNA sequence encoding the protein, subtilisin or protease is modified to produce a variant (or mutant) DNA sequence which encodes the substitution, deletion or insertion of one or more amino acids in the naturally-occurring amino acid sequence. Suitable methods to produce such modification, and which may be combined with those disclosed herein, include, but are not limited to those disclosed in US Patent 4,760,025 (US RE 34,606), US Patent 5,204,015 and US Patent 5,185,258.

"Non-human subtilisins" and the DNA sequences encoding them are obtained from many prokaryotic and eukaryotic organisms. Suitable examples of prokaryotic organisms include Gram-negative organisms (*e.g.*, *E. coli* and *Pseudomonas sp.*), as well as Gram-positive bacteria (*e.g.*, *Micrococcus sp.* and *Bacillus sp.*). Examples of eukaryotic organisms from which subtilisins and their genes may be obtained include fungi such as *Saccharomyces cerevisiae* and *Aspergillus sp*.

The term "sample" as used herein is used in its broadest sense. However, in preferred embodiments, the term is used in reference to a sample (*e.g.*, an aliquot) that comprises a peptide (*e.g.*, a peptide within a pepset, that comprises a sequence of a protein of interest) that is being analyzed, identified, modified, and/or compared with other peptides. Thus, in most cases, this term is used in reference to material that includes a protein or peptide that is of interest.

As used herein, "background level" and "background response" refer to the average percent of responders to any given peptide in the dataset for any tested protein. This value is determined by averaging the percent responders for all peptides in the set, as compiled for all the tested donors. As an example, a 3% background response would indicate that on average there would be three positive (SI greater than 2.95) responses for any peptide in a dataset when tested on 100 donors.

As used herein, "antigen presenting cell" ("APC") refers to a cell of the immune system that presents antigen on its surface, such that the antigen is recognizable by receptors on the surface of T-cells. Antigen presenting cells include, but are not limited to dendritic cells, interdigitating cells, activated B-cells and macrophages.

As used herein, the terms "T lymphocyte" and "T-cell," encompass any cell within the T lymphocyte lineage from T-cell precursors (including Thy1 positive cells which have not rearranged the T cell receptor genes) to mature T cells (*i.e.*, single positive for either CD4 or CD8, surface TCR positive cells).

As used herein, "$CD4^+$ T-cell" and "CD4 T-cell" refer to helper T-cells, while "$CD8^+$ T-cell" and CD8 T-cell" refer to cytotoxic T-cells.

"T-cell proliferation," as used herein, refers to the number of T-cells produced during the incubation of T-cells with the antigen presenting cells, with or without the presence of antigen.

"Baseline T-cell proliferation," as used herein, refers to the degree of T-cell proliferation that is normally seen in an individual in response to exposure to antigen presenting cells in the absence of peptide or protein antigen. For the purposes herein, the baseline T-cell proliferation level is determined on a per sample basis for each individual as the proliferation of T-cells in response to antigen presenting cells in the absence of antigen.

As used herein, "T-cell epitope" refers to a feature of a peptide or protein which is recognized by a T-cell receptor in the initiation of an immunogenic response to the peptide comprising that antigen (*i.e.*, the immunogen). Although it is not intended that the present invention be limited to any particular mechanism, it is generally believed that recognition of a T-cell epitope by a T-cell is via a mechanism wherein T-cells recognize peptide fragments of antigens which are bound to Class I or Class II MHC (*i.e.*, HLA) molecules expressed on antigen-presenting cells (*See e.g.*, Moeller, Immunol. Rev., 98:187 [1987]).

As used herein, "altered T-cell epitope," refers to an epitope amino acid sequence which differs from the precursor peptide or peptide of interest, such that the variant peptide of interest produces different immunogenic responses in a human or another animal. It is contemplated that an altered immunogenic response encompasses altered immunogenicity and/or allergenicity (*i.e.*, an either increased or decreased overall immunogenic response). In some embodiments, the altered T-cell epitope comprises substitution and/or deletion of an amino acid selected from those residues within the identified epitope. In alternative embodiments, the altered T-cell epitope comprises an addition of one or more residues within the epitope.

As used herein, a "weakly significant T-cell epitope" refers to an epitope wherein the response rate within the

tested donor pool is greater than the background response rate, but less than three times the background rate.

As used herein, the terms "B lymphocyte" and "B-cell" encompasses any cell within the B-cell lineage from B-cell precursors, such as pre-B-cells ($B220^+$ cells which have begun to rearrange Ig heavy chain genes), to mature B-cells and plasma cells.

As used herein, "B-cell proliferation," refers to the number of B-cells produced during the incubation of B-cells with the antigen presenting cells, with or without the presence of antigen.

As used herein, "baseline B-cell proliferation," as used herein, refers to the degree of B-cell proliferation that is normally seen in an individual in response to exposure to antigen presenting cells in the absence of peptide or protein antigen. For the purposes herein, the baseline B-cell proliferation level is determined on a per sample basis for each individual as the proliferation of B-cells in the absence of antigen.

As used herein, "B-cell epitope," refers to a feature of a peptide or protein which is recognized by a B-cell receptor in the immunogenic response to the peptide comprising that antigen (*i.e.*, the immunogen).

As used herein, "altered B-cell epitope," refers to an epitope amino acid sequence which differs from the precursor peptide or peptide of interest, such that the variant peptide of interest produces different (*i.e.*, altered) immunogenic responses in a human or another animal. It is contemplated that an altered immunogenic response encompasses altered immunogenicity and/or allergenicity (*i.e.*, an either increased or decreased overall immunogenic response). In some embodiments, the altered B-cell epitope comprises substitution and/or deletion of an amino acid selected from those residues within the identified epitope. In alternative embodiments, the altered B-cell epitope comprises an addition of one or more residues within the epitope.

As used herein, the term "significant epitope" refers to an epitope (*i.e.*, a T-cell or B-cell epitope) wherein the response rate within the tested donor pool is equal to or greater than about three times the background response rate.

As used herein "altered immunogenic response," refers to an increased or reduced immunogenic response. Proteins and peptides exhibit an "increased immunogenic response" when the T-cell and/or B-cell response they evoke is greater than that evoked by a parental (*e.g.*, precursor) protein or peptide (*e.g.*, the protein of interest). The net result of this higher response is an increased antibody response directed against the variant protein or peptide. Proteins and peptides exhibit a "reduced immunogenic response" when the T-cell and/or B-cell response they evoke is less than that evoked by a parental (*e.g.*, precursor) protein or peptide. In preferred embodiments, the net result of this lower response is a reduced antibody response directed against the variant protein or peptide. In some preferred embodiments, the parental protein is a wild-type protein or peptide.

As used herein, an "*in vivo* reduction in immunogenicity" refers to an exhibited decrease in the immunogenic response as determined by an assay that occurs at least in part, within a living organism, (*e.g.*, requires the use of an living animal). Exemplary "*in vivo*" assays include determination of altered immunogenic responses in mouse models.

As used herein, an "*in vitro* reduction in immunogenicity" refers an exhibited decrease in the immunogenic response as determined by an assay that occurs in an artificial environment outside of a living organism (*i.e.*, does not require use of a living animal). Exemplary *in vitro* assays include testing the proliferative responses by human peripheral blood mononuclear cells to a peptide of interest.

As used herein, "protein of interest," refers to a protein (*e.g.*, protease) which is being analyzed, identified and/or modified. Naturally-occurring, as well as recombinant proteins find use in the present invention. As used herein, "protein" refers to any composition comprised of amino acids and recognized as a protein by those of skill in the art. The terms "protein," "peptide" and polypeptide are used interchangeably herein. Amino acids may be referred to by their complete names (*e.g.*, alanine) or by the accepted one letter (*e.g.*, A), or three letter (*e.g.*, ala) abbreviations. Wherein a peptide is a portion of a protein, those skill in the art understand the use of the term in context. The term "protein" encompasses mature forms of proteins, as well as the pro- and prepro-forms of related proteins. Prepro forms of proteins comprise the mature form of the protein having a prosequence operably linked to the amino terminus of the protein, and a "pre-" or "signal" sequence operably linked to the amino terminus of the prosequence. In preferred embodiments, the protein is a subtilisin Carlsberg.

As used herein, "wild-type" and "native" proteins are those found in nature. The terms "wild-type sequence," and "wild-type gene" are used interchangeably herein, to refer to a sequence that is native or naturally occurring in a host cell. In some embodiments, the wild-type sequence refers to a sequence of interest that is the starting point of a protein engineering project.

As used herein, "protease" refers to naturally-occurring proteases, as well as recombinant proteases. Proteases are carbonyl hydrolases which generally act to cleave peptide bonds of proteins or peptides. Naturally-occurring proteases include, but are not limited to such examples as $\alpha$-aminoacylpeptide hydrolase, peptidylamino acid hydrolase, acylamino hydrolase, serine carboxypeptidase, metallocarboxypeptidase, thiol proteinase, carboxylproteinase and metalloproteinase. Serine, metallo, thiol and acid proteases are included, as well as endo and exo-proteases. Indeed, in some preferred embodiments, serine proteases such as chymotrypsin and subtilisin find use. Both of these serine proteases have a catalytic triad comprising aspartate, histidine and serine. In the subtilisin proteases, the relative order of these amino acids reading from the carboxy terminus is aspartate-histidine-serine, while in the chymotrypsin proteases, the

relative order of these amino acids reading from the carboxy terminus is histidine-asparate-serine. Although subtilisins are typically obtained from bacterial, fungal or yeast sources, "subtilisin" as used herein, refers to a serine protease having the catalytic triad of the subtilisin proteases defined above. Additionally, human subtilisins are proteins of human origin having subtilisin catalytic activity, for example the kexin family of human derived proteases. Subtilisins are well known by those skilled in the art for example, *Bacillus amyloliquefaciens* subtilisin (BPN'), *Bacillus lentus* subtilisin, *Bacillus subtilis* subtilisin, *Bacillus licheniformis* subtilisin (*See e.g.,* US Patent No. 4,760,025 (RE 34,606), US Patent No. 5,204,015, US Patent No. 5,185,258, European Patent No. 0 328 299, and WO89/06279). In some preferred embodiments of the present invention, the term is used in reference to a subtilisin, while in particularly preferred embodiments, the term refers to the ALCALASE® enzyme.

As used herein, "protein" refers to any composition comprised of amino acids and recognized as a protein by those of skill in the art. The terms "protein," "peptide" and polypeptide are used interchangeably herein. Wherein a peptide is a portion of a protein, those skill in the art understand the use of the term in context. The term "protein" encompasses mature forms of proteins, as well as the pro- and prepro-forms of related proteins. Prepro forms of proteins comprise the mature form of the protein having a prosequence operably linked to the amino terminus of the protein, and a "pre-" or "signal" sequence operably linked to the amino terminus of the prosequence. As used herein, functionally similar proteins are considered to be "related proteins." In some embodiments, these proteins are derived from a different genus and/or species (*e.g., B. subtilis* subtilisin and *B. lentus* subtilisin), including differences between classes of organisms (*e.g.*, a bacterial subtilisin and a fungal subtilisin). In additional embodiments, related proteins are provided from the same species. Indeed, it is not intended that the present invention be limited to related proteins from any source(s).

As used herein, the term "derivative" refers to a protein (*e.g.*, a protease) which is derived from a precursor protein (*e.g.*, the native protease) by addition of one or more amino acids to either or both the C- and N-terminal end (s), substitution of one or more amino acids at one or a number of different sites in the amino acid sequence, and/or deletion of one or more amino acids at either or both ends of the protein or at one or more sites in the amino acid sequence, and/or insertion of one or more amino acids at one or more sites in the amino acid sequence. The preparation of a protease derivative is preferably achieved by modifying a DNA sequence which encodes for the native protein, transformation of that DNA sequence into a suitable host, and expression of the modified DNA sequence to form the derivative protease.

One type of related (and derivative) proteins are "variant proteins." In preferred embodiments, variant proteins differ from a parent protein and one another by a small number of amino acid residues. The number of differing amino acid residues may be one or more, preferably 1, 2, 3, 4, 5, 10, 15, 20, 30, 40, 50, or more amino acid residues. In one preferred embodiment, the number of different amino acids between variants is between 1 and 10. In particularly preferred embodiments, related proteins and particularly variant proteins comprise at least 50%, 60%, 65%. 70%, 75%, 80%, 85%, 90%, 95%, 97%, 98%, or 99% amino acid sequence identity. Additionally, a related protein or a variant protein as used herein, refers to a protein that differs from another related protein or a parent protein in the number of prominent regions. For example, in some embodiments, variant proteins have 1, 2, 3, 4, 5, or 10 corresponding prominent regions which differ from the parent protein. In one embodiment, the prominent corresponding region of a variant produces only a background level of immunogenic response.

As used herein, "corresponding to," refers to a residue at the enumerated position in a protein or peptide, or a residue that is analogous, homologous, or equivalent to an enumerated residue in another protein or peptide.

As used herein, "corresponding region" generally refers to an analogous position within related proteins or a parent protein.

As used herein, the term "analogous sequence" refers to a sequence within a protein that provides similar function, tertiary structure, and/or conserved residues as the protein of interest. In particularly preferred embodiments, the analogous sequence involves sequence(s) at or near an epitope. For example, in epitope regions that contain an alpha helix or a beta sheet structure, the replacement amino acids in the analogous sequence preferably maintain the same specific structure. The term also refers to nucleotide sequences, as well as amino acid sequences.

As used herein, "homologous protein" refers to a protein (*e.g.*, protease) that has similar catalytic action, structure, antigenic, and/or immunogenic response as the protein (*i.e.*, protease) of interest. It is not intended that a homolog and a protein (*e.g.*, protease) of interest be necessarily related evolutionarily. Thus, it is intended that the term encompass the same functional protein obtained from different species. In some preferred embodiments, it is desirable to identify a homolog that has a tertiary and/or primary structure similar to the protein of interest, as replacement for the epitope in the protein of interest with an analogous segment from the homolog will reduce the disruptiveness of the change. Thus, in most cases, closely homologous proteins provide the most desirable sources of epitope substitutions. Alternatively, it is advantageous to look to human analogs for a given protein.

In preferred embodiments, "homolog," as used herein means an enzyme which has similar catalytic action, structure and/or use as ALCALASE® enzyme. In some preferred embodiments, the ALCALASE® enzyme homologs of the present invention have tertiary and/or primary structures substantially similar to wild-type ALCALASE® enzyme. A significant ALCALASE® enzyme epitope may be replaced with an analogous segment from a homologous enzyme.

This type of replacement may reduce the disruptiveness of the change in the parent subtilisin. In most cases, closely homologous proteins provide the most desirable source of epitope substitutions.

As used herein, "homologous genes" refers to at least a pair of genes from different, but usually related species, which correspond to each other and which are identical or very similar to each other. The term encompasses genes that are separated by speciation (*i.e.*, the development of new species) (*e.g.*, orthologous genes), as well as genes that have been separated by genetic duplication (*e.g.*, paralogous genes).

As used herein, "ortholog" and "orthologous genes" refer to genes in different species that have evolved from a common ancestral gene (*i.e.*, a homologous gene) by speciation. Typically, orthologs retain the same function in during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

The degree of homology between sequences may be determined using any suitable method known in the art (*See e.g.*, Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]).

For example, PILEUP is a useful program to determine sequence homology levels. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle, (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps. Another example of a useful algorithm is the BLAST algorithm, described by Altschul *et al.*, (Altschul et al., J. Mol. Biol., 215:403-410, [1990]; and Karlin et al., Proc. Natl. Acad. Sci. USA 90:5873-5787 [1993]). One particularly useful BLAST program is the WU-BLAST-2 program (See, Altschul et al., Meth. Enzymol." 266:460-480 [1996]). parameters "W," "T," and "X" determine the sensitivity and speed of the alignment. The BLAST program uses as defaults a wordlength (W) of 11, the BLOSUM62 scoring matrix (See, Henikoff and Henikoff, Proc. Natl. Acad. Sci. USA 89:10915 [1989]) alignments (B) of 50, expectation (E) of 10, M'5, N'-4, and a comparison of both strands.

As used herein, "percent (%) nucleic acid sequence identity" is defined as the percentage of nucleotide residues in a candidate sequence that are identical with the nucleotide residues of the sequence.

As used herein, the term "hybridization" refers to the process by which a strand of nucleic acid joins with a complementary strand through base pairing, as known in the art.

As used herein, "maximum stringency" refers to the level of hybridization that typically occurs at about Tm-5°C (5°C below the Tm of the probe); "high stringency" at about 5°C to 10°C below Tm; "intermediate stringency" at about 10°C to 20°C below Tm; and "low stringency" at about 20°C to 25°C below Tm. As will be understood by those of skill in the art, a maximum stringency hybridization can be used to identify or detect identical polynucleotide sequences while an intermediate or low stringency hybridization can be used to identify or detect polynucleotide sequence homologs.

The phrases "substantially similar and "substantially identical" in the context of two nucleic acids or polypeptides typically means that a polynucleotide or polypeptide comprises a sequence that has at least 75% sequence identity, preferably at least 80%, more preferably at least 90%, still more preferably 95%, most preferably 97%, sometimes as much as 98% and 99% sequence identity, compared to the reference (*i.e.*, wild-type) sequence. Sequence identity may be determined using known programs such as BLAST, ALIGN, and CLUSTAL using standard parameters. (*See e.g.*, Altschul, et al., J. Mol. Biol. 215:403-410 [1990]; Henikoff et al., Proc. Natl Acad Sci. USA 89:10915 [1989]; Karin et al., Proc. Natl Acad. Sci USA 90:5873 [1993]; and Higgins et al., Gene 73:237 - 244 [1988]). Software for performing BLAST analyses is publicly available through the National Center for Biotechnology Information. Also, databases may be searched using FASTA (Pearson et al., Proc. Natl. Acad. Sci. USA 85:2444-2448 [1988]).

In some embodiments, "equivalent residues" are defined by determining homology at the level of tertiary structure for a precursor protein (*i.e.*, protein of interest) whose tertiary structure has been determined by x-ray crystallography. Equivalent residues are defined as those for which the atomic coordinates of two or more of the main chain atoms of a particular amino acid residue of the precursor protein and another protein are within 0.13nm and preferably 0.1 nm after alignment. Alignment is achieved after the best model has been oriented and positioned to give the maximum overlap of atomic coordinates of non-hydrogen protein atoms of the protein. In most embodiments, the best model is the crystallographic model giving the lowest R factor for experimental diffraction data at the highest resolution available.

In some embodiments, modification is preferably made to the "precursor DNA sequence" which encodes the

amino acid sequence of the precursor enzyme, but can be by the manipulation of the precursor protein. In the case of residues which are not conserved, the replacement of one or more amino acids is limited to substitutions which produce a variant which has an amino acid sequence that does not correspond to one found in nature. In the case of conserved residues, such replacements should not result in a naturally-occurring sequence. Derivatives provided by the present invention further include chemical modification(s) that change the characteristics of the protease. In some preferred embodiments, the protein gene is ligated into an appropriate expression plasmid. The cloned protein gene is then used to transform or transfect a host cell in order to express the protein gene. This plasmid may replicate in hosts in the sense that it contains the well-known elements necessary for plasmid replication or the plasmid may be designed to integrate into the host chromosome. The necessary elements are provided for efficient gene expression (*e.g.*, a promoter operably linked to the gene of interest). In some embodiments, these necessary elements are supplied as the gene's own homologous promoter if it is recognized, (*i.e.*, transcribed, by the host), a transcription terminator (a polyadenylation region for eukaryotic host cells) which is exogenous or is supplied by the endogenous terminator region of the protein gene. In some embodiments, a selection gene such as an antibiotic resistance gene that enables continuous cultural maintenance of plasmid-infected host cells by growth in antimicrobial-containing media is also included.

The present invention encompasses proteases having altered immunogenicity that are equivalent to those that are derived from the particular microbial strain mentioned. Being "equivalent," means that the proteases are encoded by a polynucleotide capable of hybridizing to the polynucleotide having the sequence as shown in any one of those shown in Figure 1, under conditions of medium to high stringency and still retaining the altered immunogenic response to human T-cells. Being "equivalent" means that the protease comprises at least 55%, at least 65%, at least 70%, at least 75%, at least 80%, at least 85%, at least 90%, at least 95%, at least 97% or at least 99% identity to the epitope sequences and the variant proteases having such epitopes (*e.g.*, having the amino acid sequence modified).

As used herein, the terms "hybrid proteases" and "fusion proteases " refer to proteins that are engineered from at least two different or "parental" proteins. In preferred embodiments, these parental proteins are homologs of one another. For example, in some embodiments, a preferred hybrid protease or fusion protein contains the N-terminus of a protein and the C-terminus of a homolog of the protein. In some preferred embodiment, the two terminal ends are combined to correspond to the full-length active protein. In alternative preferred embodiments, the homologs share substantial similarity but do not have identical T-cell epitopes. Therefore, in one embodiment, the present invention provides a protease of interest having one or more T-cell epitopes in the C-terminus, but in which the C-terminus is replaced with the C-terminus of a homolog having a less potent T-cell epitope, or fewer or no T-cell epitopes in the C-terminus. Thus, the skilled artisan understands that by being able to identify T-cell epitopes among homologs, a variety of variants producing different immunogenic responses can be formed. Moreover, it is understood that internal portions, and more than one homolog can be used to produce the variants of the present invention.

The variants of the present invention include the mature forms of protein variants, as well as the pro- and prepro- forms of such protein variants. The prepro- forms are the preferred construction since this facilitates the expression, secretion and maturation of the protein variants.

As used herein, "prosequence" refers to a sequence of amino acids bound to the N-terminal portion of the mature form of a protein which when removed results in the appearance of the "mature" form of the protein. Many proteolytic enzymes are found in nature as translational proenzyme products and, in the absence of post-translational processing, are expressed in this fashion. A preferred prosequence for producing protein variants such as protease variants is the putative prosequence of *Bacillus licheniformis* subtilisin Carlsberg, although other prosequences find use in the present invention.

As used herein, "signal sequence" and "presequence" refer to any sequence of amino acids bound to the N-terminal portion of a protein or to the N-terminal portion of a pro-protein which may participate in the secretion of the mature or pro forms of the protein. This definition of signal sequence is a functional one and is intended to include all those amino acid sequences encoded by the N-terminal portion of the protein gene which participate in the effectuation of the secretion of protein under native conditions. The present invention utilizes such sequences to effect the secretion of the protein variants described herein.

As used herein, a "prepro" form of a protein variant consists of the mature form of the protein having a prosequence operably linked to the amino terminus of the protein and a "pre" or "signal" sequence operably linked to the amino terminus of the prosequence.

The term "regulatory element" as used herein refers to a genetic element that controls some aspect of the expression of nucleic acid sequences. For example, a promoter is a regulatory element which facilitates the initiation of transcription of an operably linked coding region. Additional regulatory elements include splicing signals, polyadenylation signals and termination signals.

As used herein, "expression vector" refers to a DNA construct containing a DNA sequence that is operably linked to a suitable control sequence capable of effecting the expression of the DNA in a suitable host. Such control sequences include a promoter to effect transcription, an optional operator sequence to control such transcription, a sequence encoding suitable mRNA ribosome binding sites and sequences which control termination of transcription

and translation. The vector may be a plasmid, a phage particle, or simply a potential genomic insert. Once transformed into a suitable host, the vector may replicate and function independently of the host genome, or may, in some instances, integrate into the genome itself. In the present specification, "plasmid" and "vector" are sometimes used interchangeably as the plasmid is the most commonly used form of vector at present. However, the invention is intended to include such other forms of expression vectors that serve equivalent functions and which are, or become, known in the art.

As used herein, "host cells" are generally prokaryotic or eukaryotic hosts that preferably have been manipulated by the methods known in the art (*See e.g.*, US Patent 4,760,025 (RE 34,606)) to render them incapable of secreting enzymatically active endoprotease. A preferred host cell for expressing protein is the *Bacillus* strain BG2036 which is deficient in enzymatically active neutral protein and alkaline protease (subtilisin). The construction of strain BG2036 is described in detail in US Patent 5,264,366. Other host cells for expressing protein include *Bacillus subtilis* 1168 (also described in US Patent 4,760,025 (RE 34,606) and US Patent 5,264,366), as well as any suitable *Bacillus* strain, including those within the species of *B. licheniformis, B. lentus,* and other *Bacillus* species, etc.

Host cells are transformed or transfected with vectors constructed using recombinant DNA techniques known in the art. Transformed host cells are capable of either replicating vectors encoding the protein variants or expressing the desired protein variant. In the case of vectors which encode the pre- or prepro-form of the protein variant, such variants, when expressed, are typically secreted from the host cell into the host cell medium.

The term "introduced" in the context of inserting a nucleic acid sequence into a cell, means transformation, transduction or transfection. Means of transformation include protoplast transformation, calcium chloride precipitation, electroporation, naked DNA and the like as known in the art. (See, Chang and Cohen (1979) Mol. Gen. Genet. 168:111 - 115; Smith et al., (1986) Appl. and Env. Microbiol. 51:634; and the review article by Ferrari et al., (1989) Genetics, pages 57 - 72 in Bacillus ed. C. Harwood, Plenum Publishing Corporation).

In embodiments involving proteases, variant protease activity can be determined and compared with the protease of interest by examining the interaction of the protease with various commercial substrates, including, but not limited to casein, keratin, elastin, and collagen. Indeed, protease activity can be determined by any suitable method known in the art. Exemplary assays to determine protease activity include, but are not limited to, succinyl-Ala-Ala-Pro-Phe-para nitroanilide (SAAPFpNA) (citation) assay; and 2,4,6-trinitrobenzene sulfonate sodium salt (TNBS) assay. In the SAAPFpNA assay, proteases cleave the bond between the peptide and p-nitroaniline to give a visible yellow colour absorbing at 405 nm. In the TNBS color reaction method, the assay measures the enzymatic hydrolysis of the substrate into polypeptides containing free amino groups. These amino groups react with TNBS to form a yellow colored complex. Thus, the more deeply colored the reaction, the more activity is measured. The yellow color can be determined by various analyzers or spectrophotometers known in the art.

Other characteristics of the variant proteases can be determined by methods known to those skilled in the art. Exemplary characteristics include, but are not limited to thermal stability, alkaline stability, and stability of the particular protease in various substrate or buffer solutions or product formulations.

When combined with the enzyme stability assay procedures disclosed herein, mutants obtained by random mutagenesis can be identified which demonstrate either increased or decreased alkaline or thermal stability while maintaining enzymatic activity.

Alkaline stability can be measured either by known procedures or by the methods described herein. A substantial change in alkaline stability is evidenced by at least about a 5% or greater increase or decrease (in most embodiments, it is preferably an increase) in the half-life of the enzymatic activity of a mutant when compared to the precursor protein.

Thermal stability can be measured either by known procedures or by the methods described herein. A substantial change in thermal stability is evidenced by at least about a 5% or greater increase or decrease (in most embodiments, it is preferably an increase) in the half-life of the catalytic activity of a mutant when exposed to a relatively high temperature and neutral pH as compared to the precursor protein.

As used herein, "personal care products" means products used in the cleaning of hair, skin, scalp, teeth, including, but not limited to shampoos, body lotions, shower gels, topical moisturizers, toothpaste, and/or other topical cleansers. In some particularly preferred embodiments, these products are utilized by humans, while in other embodiments, these products find use with non-human animals (*e.g.*, in veterinary applications).

As used herein, "skin care compositions" means products used in topical application for cleaning and/or moisturizing skin. Such compositions include, but are not limited to moisturizing body washes, shower gels, body lotions, moisturizing facial creams, make-up removers, and lotions.

As used herein, "cleaning compositions" are compositions that can be used to remove undesired compounds from substrates, such as fabric, dishes, contact lenses, other solid substrates, hair (shampoos), skin (soaps and creams), teeth (mouthwashes, toothpastes) etc.

The term "cleaning composition materials," as used herein, refers to any liquid, solid or gaseous material selected for the particular type of cleaning composition desired and the form of the product (*e.g.*, liquid; granule; spray composition), which materials are also compatible with the protease enzyme used in the composition. The specific selection of cleaning composition materials are readily made by considering the surface, item or fabric to be cleaned,

and the desired form of the composition for the cleaning conditions during use (*e.g.*, through the wash detergent use).

As used herein the term "hard surface cleaning composition," refers to detergent compositions for cleaning hard surfaces such as floors, walls, bathroom tile, and the like. Such compositions are provided in any form, including but not limited to solids, liquids, emulsions, etc.

As used herein, "dishwashing composition" refers to all forms for compositions for cleaning dishes, including but not limited to, granular and liquid forms.

As used herein, "fabric cleaning composition" refers to all forms of detergent compositions for cleaning fabrics, including but not limited to, granular, liquid and bar forms. As used herein, "fabric" refers to any textile material.

As used herein, the term "compatible," means that the cleaning composition materials do not reduce the proteolytic activity of the protease enzyme to such an extent that the protease is not effective as desired during normal use situations. Specific cleaning composition materials are exemplified in detail hereinafter.

As used herein, "effective amount of protease enzyme" refers to the quantity of protease enzyme (*e.g.*, subtilisin Carlsberg) necessary to achieve the enzymatic activity necessary in the specific application (*e.g.*, personal care product, cleaning composition, etc.). Such effective amounts are readily ascertained by one of ordinary skill in the art and are based on many factors, such as the particular enzyme used, the cleaning application, the specific composition of the cleaning composition, and whether a liquid or dry (*e.g.*, granular, bar) composition is required, and the like.

As used herein, "non-fabric cleaning compositions" encompass hard surface cleaning compositions, dishwashing compositions, oral cleaning compositions, denture cleaning compositions, and personal cleansing compositions.

As used herein, "oral cleaning compositions" refers to dentifrices, toothpastes, toothgels, toothpowders, mouthwashes, mouth sprays, mouth gels, chewing gums, lozenges, sachets, tablets, biogels, prophylaxis pastes, dental treatment solutions, and the like.

As used herein, "pharmaceutically acceptable" means that drugs, medicaments and/or inert ingredients which the term describes are suitable for use in contact with the tissues of humans and other animals without undue toxicity, incompatibility, instability, irritation, allergic response, and the like, commensurate with a reasonable benefit/risk ratio.

Many of the protein variants of the present invention are useful in formulating various detergent compositions. A number of known compounds are suitable surfactants useful in compositions comprising the protein mutants of the invention. These include nonionic, anionic, cationic, anionic or zwitterionic detergents (*See e.g.*, US Patent No 4,404,128 and US Patent No. 4,261,868). A suitable detergent formulation is that described in US Patent 5,204,015. Those in the art are familiar with the different formulations which find use as cleaning compositions. In addition to typical cleaning compositions, it is readily understood that the protein variants of the present invention find use in any purpose that native or wild-type proteins are used. Thus, these variants can be used, for example, in bar or liquid soap applications, dishcare formulations, surface cleaning applications, contact lens cleaning solutions or products, peptide hydrolysis, waste treatment, textile applications, as fusion-cleavage enzymes in protein production, etc. Indeed, it is not intended that the variants of the present invention be limited to any particular use. For example, the variants of the present invention may comprise, in addition to decreased allergenicity/immunogenicity, enhanced performance in a detergent composition (as compared to the precursor). As used herein, enhanced performance in a detergent is defined as increasing cleaning of certain enzyme sensitive stains (*e.g.*, grass or blood), as determined by usual evaluation after a standard wash cycle.

Proteins, particularly proteases of the invention can be formulated into known powdered and liquid detergents having pH between 6.5 and 12.0 at levels of about .01 to about 5% (preferably 0.1% to 0.5%) by weight. In some embodiments, these detergent cleaning compositions further include other enzymes such as proteases, amylases, cellulases, lipases or endoglycosidases, as well as builders and stabilizers.

The addition of proteins to conventional cleaning compositions does not create any special use limitations. In other words, any temperature and pH suitable for the detergent are also suitable for the present compositions, as long as the pH is within the above range, and the temperature is below the described protein's denaturing temperature. In addition, proteins of the invention find use in cleaning compositions without detergents, again either alone or in combination with builders and stabilizers.

In one embodiment, the present invention provides compositions for the treatment of textiles that includes variant proteins of the present invention. The composition can be used to treat for example silk or wool (*See e.g.*, U.S. Reissue Patent No. 216,034; European Patent No. 134,267; US Patent No. 4,533,359; and European Patent No. 344,259). These variants can be screened for proteolytic activity and their suitability for these applications using methods well known in the art.

As indicated above, the proteins of the present invention exhibit modified immunogenic responses (*e.g.*, antigenicity and/or immunogenicity) when compared to the native proteins encoded by their precursor DNAs. In some preferred embodiments, the proteins (*e.g.*, proteases) exhibit reduced allergenicity/immunogenicity. Those of skill in the art readily recognize that the uses of the proteases of this invention will be determined, in large part, on the immunological properties of the proteins. For example, proteases that exhibit reduced immunogenic responses can be used in cleaning compositions. An effective amount of one or more protease variants described herein find use in compositions useful for cleaning a variety of surfaces in need of proteinaceous stain removal. Such cleaning compositions include detergent

compositions for cleaning hard surfaces, detergent compositions for cleaning fabrics, dishwashing compositions, oral cleaning compositions, and denture cleaning compositions.

An effective amount of one or more related and/or variant proteins with reduced allergenicity/immunogenicity, ranked according to the methods of the present invention find use in various compositions that are applied to keratinous materials such as nails and hair, including but not limited to those useful as hair spray compositions, hair shampoo and/or conditioning compositions, compositions applied for the purpose of hair growth regulation, and compositions applied to the hair and scalp for the purpose of treating seborrhea, dermatitis, and/or dandruff.

An effective amount of one or more protease variant(s) described herein find use in included in compositions suitable for topical application to the skin or hair. These compositions can be in the form of creams, lotions, gels, and the like, and may be formulated as aqueous compositions or may be formulated as emulsions of one or more oil phases in an aqueous continuous phase.

In addition, the related and/or variant proteins with reduced allergenicity/immunogenicity find use in other applications, including pharmaceutical applications, drug delivery applications, and other health care applications.

## Skin Care Active

In some embodiments, the compositions provided by the present invention comprise a skin care active at a level from about 0.1 % to about 20%, preferably from about 1% to about 10%, more preferably from about 2% to about 8%, by weight. Non-limiting examples of suitable skin care actives for use herein include a vitamin $B_3$ component, panthenol, vitamin E, vitamin E acetate, retinol, retinyl propionate, retinyl palmitate, retinoic acid, vitamin C, theobromine, α-hydroxyacid, farnesol, phytantriol, salicylic acid, palmityl peptapeptide-3 and mixtures thereof.

## B3 Compound

As used herein, "vitamin $B_3$ compound" means a compound having the formula:

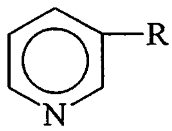

wherein R is - $CONH_2$ (*i.e.*, niacinamide), - COOH (*i.e.*, nicotinic acid) or - $CH_2OH$ (*i.e.*, nicotinyl alcohol); derivatives thereof; and salts of any of the foregoing. Exemplary derivatives of the foregoing vitamin $B_3$ compounds include nicotinic acid esters, including non-vasodilating esters of nicotinic acid, nicotinyl amino acids, nicotinyl alcohol esters of carboxylic acids, nicotinic acid N-oxide and niacinamide N-oxide.

Suitable esters of nicotinic acid include nicotinic acid esters of $C_1$-$C_{22}$, preferably $C_1$-$C_{16}$, more preferably $C_1$-$C_6$ alcohols. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (*i.e.*, the majority of the general population would not experience a visible flushing response, although such compounds may cause vasodilation not visible to the naked eye). Non-vasodilating esters of nicotinic acid include tocopherol nicotinate and inositol hexanicotinate; tocopherol nicotinate is preferred. A more complete description of vitamin $B_3$ compounds is given in WO 98/22085. Preferred vitamin $B_3$ compounds are niacinamide and tocopherol nicotinate.

## Retinoids

Another suitable skin care active is a retinoid. As used herein, "retinoid" includes all natural and/or synthetic analogs of Vitamin A or retinol-like compounds which possess the biological activity of Vitamin A in the skin as well as the geometric isomers and stereoisomers of these compounds. When a retinoid is included in the compositions herein, it typically comprises from or about 0.005% to or about 2%, more preferably 0.01% to about 2% retinoid. Retinol is preferably used in an amount of from or about 0.01% to or about 0.15%; retinol esters are preferably used in an amount of from about 0.01% to about 2% (*e.g.*, about 1%).

The retinoid is preferably retinol, retinol esters (*e.g.*, $C_2$ - $C_{22}$ alkyl esters of retinol, including retinyl palmitate, retinyl acetate, retinyl propionate), retinal, and/or retinoic acid (including all-trans retinoic acid and/or 13-cis-retinoic acid), more preferably retinoids other than retinoic acid. These compounds are well known in the art and are commercially

available from a number of sources (*e.g.*, Sigma Chemical Company (St. Louis, MO), and Boehringer Mannheim (Indianapolis, IN)). Preferred retinoids include retinol, retinyl palmitate, retinyl acetate, retinyl propionate, retinal, retinoic acid and combinations thereof. More preferred retinoids include retinol, retinoic propionate, retinoic acid and retinyl palmitate. The retinoid may be included as the substantially pure material, or as an extract obtained by suitable physical and/or chemical isolation from natural (*e.g.*, plant) sources.

**Carriers**

It is further contemplated that the compositions of the present invention will find use in safe and effective amounts of a dermatologically acceptable carrier, suitable for topical application to the skin and/or hair within which the essential materials and optional other materials are incorporated to enable the essential materials and optional components to be delivered to the skin or hair at an appropriate concentration. Thus, the carrier acts as a diluent, dispersant, solvent, or the like for the essential components which ensures that they can be applied to and distributed evenly over the selected target at an appropriate concentration.

The type of carrier utilized in the present invention depends on the type of product form desired for the composition. It is not intended that the present invention be limited to a carrier of any particular form, although it is most commonly a solid, semi-solid or liquid. Suitable carriers are liquid or semi-solid, such as creams, lotions, gels, sticks, ointments, pastes and mousses. Preferably the carrier is in the form of a lotion, cream or a gel, more preferably one which has a sufficient thickness or yield point to prevent the particles from sedimenting. The carrier can itself be inert or it can possess dermatological benefits of its own. The carrier may be applied directly to the skin and/or hair, or it may be applied via a woven or non-woven wipe or cloth. It may also be in the form of a patch, mask, or wrap. It may also be aerosolized or otherwise sprayed onto the skin and/or hair. The carrier should also be physically and chemically compatible with the essential components described herein, and should not unduly impair stability, efficacy or other use benefits associated with the compositions of the present invention.

Preferred carriers contain a dermatologically acceptable, hydrophilic diluent. Suitable hydrophilic diluents include water, organic hydrophilic diluents such as $C_1$ - $C_4$ monohydric alcohols and low molecular weight glycols and polyols, including propylene glycol, polyethylene glycol (*e.g.* of MW 200-600), polypropylene glycol (*e.g.* of MW 425-2025), glycerol, butylene glycol, 1,2,4-butanetriol, sorbitol esters, 1,2,6-hexametriol, ethanol, iso-propanol, sorbitol esters, ethoxylated ethers, propoxylated ethers and combinations thereof. The diluent is preferably liquid. Water is a preferred diluent. The composition preferably comprises at least about 20% of the hydrophilic diluent.

Suitable carriers may also comprise an emulsion comprising a hydrophilic phase, especially an aqueous phase, and a hydrophobic phase (*e.g.*, a lipid, oil or oily material). As well known to those skilled in the art, the hydrophilic phase is dispersed in the hydrophobic phase, or vice versa, to form respectively hydrophilic or hydrophobic dispersed and continuous phases, depending on the composition ingredients. In emulsion technology, the well-known term "dispersed phase" means that the phase exists as small particles or droplets that are suspended in and surrounded by a continuous phase. The dispersed phase is also known as the internal or discontinuous phase. The emulsion may be or comprise (*e.g.*, in a triple or other multi-phase emulsion) an oil-in-water emulsion or a water-in-oil emulsion such as a water-in-silicone emulsion. Oil-in-water emulsions typically comprise from about 1 % to about 60% (preferably about 1 % to about 30%) of the dispersed hydrophobic phase and from about 1 % to about 99% (preferably from about 40% to about 90%) of the continuous hydrophilic phase; water-in-oil emulsions typically comprise from about 1 % to about 98% (preferably from about 40% to about 90%) of the dispersed hydrophilic phase and from about 1% to about 50% (preferably about 1% to about 30%) of the continuous hydrophobic phase.

**Humectants**

In some embodiments, the compositions of the present invention comprise humectants which are preferably present at a level of from about 0.01% to about 20%, more preferably from about 0.1 % to about 15% and especially from about 0.5% to about 10%. Preferred humectants include, but are not limited to, compounds selected from polyhydric alcohols, urea, D or DL panthenol, calcium pantothenate, royal jelly, panthetine, pantotheine, panthenyl ethyl ether, pangamic acid, pyridoxin, pantoyl lactose Vitamin B complex, hexane - 1, 2, 6, - triol, guanidine or its derivatives, and mixtures thereof.

Suitable polyhydric alcohols for use herein include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, erythritol, threitol, pentaerythritol, xylitol, glucitol, mannitol, hexylene glycol, butylene glycol (*e.g.*, 1,3-butylene glycol), hexane triol (*e.g.*, 1,2,6-hexanetriol), trimethylol propane, neopentyl glycol, glycerine, ethoxylated glycerine, propane-1,3 diol, propoxylated glycerine and mixtures thereof. The alkoxylated derivatives of any of the above polyhydric alcohols are also suitable for use herein. Preferred polyhydric alcohols of the present invention are selected from glycerine, butylene glycol, propylene glycol, dipropylene glycol, polyethylene glycol,

hexane triol, ethoxylated glycerine and propoxylated glycerine, and mixtures thereof.

Suitable humectants useful herein are sodium 2-pyrrolidone-5-carboxylate (NaPCA), guanidine; glycolic acid and glycolate salts (*e.g.* ammonium and quaternary alkyl ammonium); lactic acid and lactate salts (*e.g.* ammonium and quaternary alkyl ammonium); aloe vera in any of its variety of forms (*e.g.*, aloe vera gel); hyaluronic acid and derivatives thereof (*e.g.*, salt derivatives such as sodium hyaluronate); lactamide monoethanolamine; acetamide monoethanolamine; urea; panthenol and derivatives thereof; and mixtures thereof.

At least part (up to about 5% by weight of composition) of a humectant can be incorporated in the form of an admixture with a particulate cross-linked hydrophobic acrylate or methacrylate copolymer, itself preferably present in an amount of from about 0.1 % to about 10%, which can be added either to the aqueous or disperse phase. This copolymer is particularly valuable for reducing shine and controlling oil while helping to provide effective moisturization benefits and is described in further detail by WO96/03964, incorporated herein by reference.

**Emollients**

In some embodiments, the oil in water emulsion embodiments of the present invention comprise from about 1% to about 20%, preferably from about 1.5% to about 15%, more preferably from about 0.1 % to about 8%, and even more preferably from about 0.5% to about 5% of a dermatologically acceptable emollient. Emollients tend to lubricate the skin, increase the smoothness and suppleness, prevent or relieve dryness, and/or protect the skin. Emollients are typically water-immiscible, oily or waxy materials and emollients with high molecular weights can confer tacky properties to a topical composition. A wide variety of suitable emollients are known and may be used herein. For example, Sagarin, Cosmetics, Science and Technology, 2nd Edition, Vol. 1, pp. 32-43 (1972), contains numerous examples of materials suitable for use as emollients. In addition, all emollients discussed in application WO 00/24372 should be considered as suitable for use in the present invention although preferred examples are outlined in further detail below:

i) Straight and branched chain hydrocarbons having from about 7 to about 40 carbon atoms, such as dodecane, squalane, cholesterol, hydrogenated polyisobutylene, isohexadecane, isoeicosane, isooctahexacontane, isohexapentacontahectane, and the $C_7$-$C_{40}$ isoparaffins, which are $C_7$-$C_{40}$ branched hydrocarbons. Suitable branched chain hydrocarbons for use herein are selected from isopentacontaoctactane, petrolatum, and mixtures thereof. Suitable for use herein are branched chain aliphatic hydrocarbons sold under the trade name Permethyl (RTM) and commercially available from Presperse Inc., South Plainfield, N.J.

ii) $C_1$-$C_{30}$ alcohol esters of $C_1$-$C_{30}$ carboxylic acids, C12-15 alkyl benzoates, and of $C_2$-$C_{30}$ dicarboxylic acids, for example, isononyl isononanoate, isostearyl neopentanoate. isodecyl octanoate, isodecyl isononanoate, tridecyl isononanoate, myristyl octanoate, octyl pelargonate, octyl isononanoate, myristyl myristate, myristyl neopentanoate, myristyl octanoate, isopropyl myristate, myristyl propionate, isopropyl stearate, isopropyl isostearate, methyl isostearate, behenyl behenate, dioctyl maleate, diisopropyl adipate, and diisopropyl dilinoleate and mixtures thereof.

iii) $C_1$-$C_{30}$ mono- and poly- esters of sugars and related materials. These esters are derived from a sugar or polyol moiety and one or more carboxylic acid moieties. Depending on the constituent acid and sugar, these esters can be in either liquid or solid form at room temperature. Examples include glucose tetraoleate, the galactose tetraesters of oleic acid, the sorbitol tetraoleate, sucrose tetraoleate, sucrose pentaoleate, sucrose hexaoleate, sucrose heptaoleate, sucrose octaoleate, sorbitol hexaester in which the carboxylic acid ester moieties are palmitoleate and arachidate in a 1:2 molar ratio, and the octaester of sucrose wherein the esterifying carboxylic acid moieties are laurate, linoleate and behenate in a 1:3:4 molar ratio. Other materials include cottonseed oil or soybean oil fatty acid esters of sucrose. Other examples of such materials are described in WO 96/16636. A particularly preferred material is known by the INCI name sucrose polycottonseedate.

iv) Vegetable oils and hydrogenated vegetable oils. Examples of vegetable oils and hydrogenated vegetable oils include safflower oil, coconut oil, cottonseed oil, menhaden oil, palm kernel oil, palm oil, peanut oil, soybean oil, rapeseed oil, linseed oil, rice bran oil, pine oil, sesame oil, sunflower seed oil, partially and fully hydrogenated oils from the foregoing sources, and mixtures thereof.

v) Soluble or colloidally-soluble moisturizing agents. Examples include hylaronic acid and starch-grafted sodium polyacrylates such as Sanwet (RTM) IM-1000, IM-1500 and IM-2500 available from Celanese Superabsorbent Materials, Portsmith, VA, and described in US Pat. No. 4,076,663.

Preferred emollients for use herein are isohexadecane, isooctacontane, petrolatum, isononyl isononanoate, isodecyl octanoate, isodecyl isononanoate, tridecyl isononanoate, myristyl octanoate, octyl isononanoate, myristyl myristate, methyl isostearate, isopropyl isostearate, C12-15 alkyl benzoates and mixtures thereof. Particularly preferred emollients for use herein are isohexadecane, isononyl isononanoate, methyl isostearate, isopropyl isostearate, petrolatum, or mixtures thereof.

## Emulsifiers/Surfactants

In some embodiments, the compositions of the present invention contain an emulsifier and/or surfactant, generally to help disperse and suspend the disperse phase within the continuous aqueous phase. A surfactant may also be useful if the product is intended for skin cleansing. For convenience hereinafter, emulsifiers are encompassed within the term "surfactants." Thus, "surfactant(s)" refer(s) to surface active agents whether used as emulsifiers or for other surfactant purposes such as skin cleansing. Known or conventional surfactants find use used in the compositions of the present invention, provided that the selected agent is chemically and physically compatible with essential components of the composition, and provides the desired characteristics. Suitable surfactants include non-silicone derived materials, and mixtures thereof. All surfactants discussed in application WO 00/24372 are considered as suitable for use in the present invention.

In some embodiments, the compositions of the present invention comprise from about 0.05% to about 15% of a surfactant or mixture of surfactants. The exact surfactant or surfactant mixture chosen will depend upon the pH of the composition and the other components present.

Among the nonionic surfactants that are useful herein are those that can be broadly defined as condensation products of long chain alcohols (*e.g.* $C_{8-30}$ alcohols), with sugar or starch polymers (*i.e.*, glycosides). Other useful nonionic surfactants include the condensation products of alkylene oxides with fatty acids (*i.e.*, alkylene oxide esters of fatty acids). These materials have the general formula $RCO(X)_nOH$ wherein R is a $C_{10-30}$ alkyl group, X is $-OCH_2CH_2-$ (*i.e.*, derived from ethylene glycol or oxide) or $-OCH_2CHCH_3-$(*i.e.*, derived from propylene glycol or oxide), and n is an integer from about 6 to about 200. Other nonionic surfactants are the condensation products of alkylene oxides with 2 moles of fatty acids (*i.e.*, alkylene oxide diesters of fatty acids). These materials have the general formula $RCO(X)_nOOCR$ wherein R is a $C_{10-30}$ alkyl group, X is $-OCH_2CH_2-$(*i.e.* derived from ethylene glycol or oxide) or $-OCH_2CHCH_3-$(*i.e.*, derived from propylene glycol or oxide), and n is an integer from about 6 to about 100. An emulsifier for use herein is most preferably a fatty acid ester blend based on a mixture of sorbitan fatty acid ester and sucrose fatty acid ester, especially a blend of sorbiton stearate and sucrose cocoate. This is commercially available from ICI under the trade name Arlatone 2121. Even further suitable examples include a mixture of cetearyl alcohols, cetearyl glucosides such as those available under the trade name Montanov 68 from Seppic and Emulgade PL68/50 available from Henkel.

In some embodiments, the hydrophilic surfactants useful herein alternatively or additionally include any of a wide variety of cationic, anionic, zwitterionic, and amphoteric surfactants such as are known in the art (See *e.g.*, US Patent No. 5,011,681, US Patent No. 4,421,769, and US Patent No. 3,755,560). A wide variety of anionic surfactants also find use in the compositions of the present invention (See *e.g.*, US Patent No. 3,929,678). Exemplary anionic surfactants include the alkoyl isethionates (*e.g.*, $C_{12}$ - $C_{30}$), alkyl and alkyl ether sulfates and salts thereof, alkyl and alkyl ether phosphates and salts thereof, alkyl methyl taurates (*e.g.*, $C_{12}$ - $C_{30}$), and soaps (*e.g.*, alkali metal salts, such as sodium or potassium salts) of fatty acids.

Amphoteric and zwitterionic surfactants also find use in the compositions of the present invention. Examples of amphoteric and zwitterionic surfactants which can be used in the compositions of the present invention are those which are broadly described as derivatives of aliphatic secondary and tertiary amines in which the aliphatic radical can be straight or branched chain and wherein one of the aliphatic substituents contains from about 8 to about 22 carbon atoms (preferably $C_8$ - $C_{18}$) and one contains an anionic water solubilizing group (*e.g.*, carboxy, sulfonate, sulfate, phosphate, or phosphonate). Examples include alkyl imino acetates, iminodialkanoates and aminoalkanoates, imidazolinium and ammonium derivatives. Other suitable amphoteric and zwitterionic surfactants include those selected from the group consisting of betaines, sultaines, hydroxysultaines, and branched and unbranched alkanoyl sarcosinates, and mixtures thereof.

In some embodiments, emulsions of the present invention further include a silicone containing emulsifier or surfactant. A wide variety of silicone emulsifiers find use in the present invention. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and polyether chains containing moieties derived from both ethylene oxide and propylene oxide. Other examples include alkyl-modified dimethicone copolyols (*i.e.*, compounds which contain $C_2$-$C_{30}$ pendant side chains). Still other useful dimethicone copolyols include materials having various cationic, anionic, amphoteric, and zwitterionic pendant moieties.

## Polymeric Thickening Agents

In some embodiments, the compositions of the present invention comprise at least one polymeric thickening agent. The polymeric thickening agents useful herein preferably have a number average molecular weight of greater than 20,000, more preferably greater than 50,000 and especially greater than 100,000. In some embodiments, the compositions of the present invention comprise from about 0.01% to about 10%, preferably from about 0.1 % to about 8% and most preferably from about 0.5% to about 5% by weight of the composition of the polymeric thickening agent, or mixtures thereof.

Preferred polymer thickening agents for use herein include non-ionic thickening agents and anionic thickening agents, or mixtures thereof. Suitable non-ionic thickening agents include polyacrylamide polymers, crosslinked poly(N-vinylpyrrolidones), polysaccharides, natural or synthetic gums, polyvinylpyrrolidone, and polyvinylalcohol. Suitable anionic thickening agents include acrylic acid/ethyl acrylate copolymers, carboxyvinyl polymers and crosslinked copolymers of alkyl vinyl ethers and maleic anhydride. Particularly preferred thickening agents for use herein are the non-ionic polyacrylamide polymers such as polyacrylamide and isoparaffin and laureth-7, available under the trade name Sepigel 305 from Seppic Corporation, and acrylic acid/ethyl acrylate copolymers and the carboxyvinyl polymers sold by the B.F. Goodrich Company under the trade mark of CARBOPOL™ resins, or mixtures thereof. In some embodiments, suitable CARBOPOL™ resins are hydrophobically modified. Additional suitable resins are described in WO98/22085. It is also contemplated that mixtures of these resins will find use in the present invention.

## Silicone Oil

In some embodiments, the present compositions comprise, at least one silicone oil phase. Silicone oil phase (s) generally comprises from about 0.1 % to about 20%, preferably from about 0.5% to about 10%, more preferably from about 0.5% to about 5%, of the composition. The, or each, silicone oil phase preferably comprises one or more silicone components.

In some embodiments, silicone components are fluids, including straight chain, branched and cyclic silicones. Suitable silicone fluids useful herein include silicones inclusive of polyalkyl siloxane fluids, polyaryl siloxane fluids, cyclic and linear polyalkylsiloxanes, polyalkoxylated silicones, amino and quaternary ammonium modified silicones, polyalkylaryl siloxanes or a polyether siloxane copolymer and mixtures thereof. The silicone fluids can be volatile or non-volatile. Silicone fluids generally have a weight average molecular weight of less than about 200,000. Suitable silicone fluids have a molecular weight of about 100,000 or less, preferably about 50,000 or less, most preferably about 10,000 or less. Preferably the silicone fluid is selected from silicone fluids having a weight average molecular weight in the range from about 100 to about 50,000 and preferably from about 200 to about 40,000. Typically, silicone fluids have a viscosity ranging from about 0.65 to about 600,000 $mm^2.s^{-1}$, preferably from about 0.65 to about 10,000 $mm^2.s^{-1}$ at 25°C. The viscosity can be measured by means of a glass capillary viscometer as set forth in Dow Corning Corporate Test Method CTM0004. Suitable polydimethyl siloxanes that find use in the present invention include those available, for example, from the General Electric Company as the SF and Viscasil (RTM) series and from Dow Corning as the Dow Corning 200 series. Also useful are essentially non-volatile polyalkylarylsiloxanes (*e.g.*, polymethylphenylsiloxanes), having viscosities of about 0.65 to 30,000 $mm^2.s^{-1}$ at 25°C. These siloxanes are available, for example, from the General Electric Company as SF 1075 methyl phenyl fluid or from Dow Corning as 556 Cosmetic Grade Fluid. Cyclic polydimethylsiloxanes suitable for use herein are those having a ring structure incorporating from about 3 to about 7 $(CH_3)_2SiO$ moieties.

Silicone gums also find use with the present invention. The term "silicone gum" herein means high molecular weight silicones having a weight average molecular weight in excess of about 200,000 and preferably from about 200,000 to about 4,000,000. The present invention includes non-volatile polyalkyl as well as polyaryl siloxane gums. In preferred embodiments, a silicone oil phase comprises a silicone gum or a mixture of silicones including the silicone gum. Typically, silicone gums have a viscosity at 25°C in excess of about 1,000,000 $mm^2s^{-1}$. The silicone gums include dimethicones as known in the art (*See e.g.*, US Patent No. 4,152,416), as well as the silicone gums described in General Electric Silicone Rubber Product Data Sheets SE 30, SE 33, SE 54 and SE 76. Specific examples of silicone gums include polydimethylsiloxane, (polydimethylsiloxane)-(methylvinylsiloxane) copolymer, poly(dimethylsiloxane)(diphenyl)(methylvinylsiloxane) copolymer and mixtures thereof. Preferred silicone gums for use herein are silicone gums having a molecular weight of from about 200,000 to about 4,000,000 selected from dimethiconol, dimethicone copolyol, dimethicone, and mixtures thereof.

A silicone phase herein preferably comprises a silicone gum incorporated into the composition as part of a silicone gum-fluid blend. When the silicone gum is incorporated as part of a silicone gum-fluid blend, the silicone gum preferably constitutes from about 5% to about 40%, especially from about 10% to 20% by weight of the silicone gum-fluid blend. Suitable silicone gum-fluid blends herein are mixtures consisting essentially of:

(i) a silicone having a molecular weight of from about 200,000 to about 4,000,000 selected from dimethiconol,

fluorosilicone and dimethicone and mixtures thereof; and

(ii) a carrier which is a silicone fluid, the carrier having a viscosity from about 0.65 $mm^2.s^{-1}$ to about 100 $mm^2.s^{-1}$,

wherein the ratio of i) to ii) is from about 10:90 to about 20:80 and wherein the silicone gum-based component has a final viscosity of from about 100 $mm^2.s^{-1}$ to about 100,000 $mm^2.s^{-1}$, preferably from 500 $mm^2.s^{-1}$ to about 10,000 $mm^2.s^{-1}$.

Further silicone components suitable for use in a silicone oil phase herein are crosslinked polyorganosiloxane polymers, optionally dispersed in a fluid carrier. In general, crosslinked polyorganosiloxane polymers, together with its carrier (if present) comprise 0.1 % to about 20%, preferably from about 0.5% to about 10%, more preferably from about 0.5% to about 5% of the composition. Such polymers comprise polyorganosiloxane polymers crosslinked by a crosslinking agent. Suitable crosslinking agents include those described in WO98/22085. Examples of suitable polyorganosiloxane polymers for use herein include methyl vinyl dimethicone, methyl vinyl diphenyl dimethicone, and methyl vinyl phenyl methyl diphenyl dimethicone.

Another class of silicone components suitable for use in a silicone oil phase herein includes polydiorganosiloxane-polyoxyalkylene copolymers containing at least one polydiorganosiloxane segment and at least one polyoxyalkylene segment. Suitable polydiorganosiloxane segments and copolymers thereof include those described in WO98/22085. Suitable polydiorganosiloxane-polyalkylene copolymers are available commercially under the trade names Belsil (RTM) from Wacker-Chemie GmbH, Munich, and Abil (RTM) from Th. Goldschmidt Ltd., England, for example Belsil (RTM) 6031 and Abil (RTM) B88183. A particularly preferred copolymer fluid blend for use herein includes Dow Corning DC3225C which has the CTFA designation Dimethicone/Dimethicone copolyol.

## Sunscreens

In still further embodiments, the present invention provides compositions comprising an organic sunscreen. In some embodiments, suitable sunscreens include UVA absorbing properties and/or UVB absorbing properties. The exact amount of the sunscreen active will vary depending upon the desired Sun Protection Factor (*i.e.*, the "SPF") of the composition, as well as the desired level of UV protection. The compositions of the present invention preferably comprise an SPF of at least 10, preferably at least 15. SPF is a commonly used measure of photoprotection of a sunscreen against erythema. The SPF is defined as a ratio of the ultraviolet energy required to produce minimal erythema on protected skin to that required to products the same minimal erythema on unprotected skin in the same individual (See, Fed. Reg., 43, No 166, pp. 38206-38269, August 25, 1978). Amounts of the sunscreen used are typically from about 2% to about 20%, more typically from about 4% to about 14%. Suitable sunscreens include, but are not limited to, those found in the Wenninger and McEwen (eds.), CTFA International Cosmetic Ingredient Dictionary and Handbook, 7th edition, volume 2 pp. 1672 (The Cosmetic, Toiletry, and Fragrance Association, Inc., Washington, D. C., 1997).

In some embodiments, compositions of the present invention comprise an UVA absorbing sunscreen actives which absorb UV radiation having a wavelength of from about 320nm to about 400nm. Suitable UVA absorbing sunscreen actives are selected from dibenzoylmethane derivatives, anthranilate derivatives such as methylanthranilate and homomethyl, 1-N-acetylanthranilate, and mixtures thereof. Examples of dibenzoylmethane sunscreen actives are described in US Patent No 4,387,089, as well as in Lowe and Shaath (eds), Sunscreens: Development. Evaluation, and Regulatory Aspects, Marcel Dekker, Inc (1990). The UVA absorbing sunscreen active is preferably present in an amount to provide broad- spectrum UVA protection either independently, or in combination with, other UV protective actives which may be present in the composition.

Suitable UVA sunscreen actives are dibenzoylmethane sunscreen actives and their derivatives. They include, but are not limited to, those selected from 2-methyldibenzoylmethane, 4-methyldibenzoylmethane, 4-isopropyldibenzoylmethane, 4-tert-butyldibenzoylmethane, 2,4-dimethyldibenzoylmethane, 2,5-dimethyldibenzoyl-methane, 4,4'-diisopropylbenzoylmethane, 4-(1,1-dimethylethyl)-4'-methoxydiben-zoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoyl-methane, 2,6-dimethyl-4'-tert-butyl-4'methoxydibenzoylmethane, and mixtures thereof. Preferred dibenzoyl sunscreen actives include those selected from 4-(1, 1-dimethylethyl)-4'-methoxydibenzoylmethane, 4-isopropyldibenzoylmethane, and mixtures thereof. A preferred sunscreen active is 4-(1, 1-dimethylethyl)-4'-methoxydibenzoylmethane.

The sunscreen active 4-(1, 1-dimethylethyl)-4'-methoxydibenzoylmethane, which is also known as butyl methoxydibenzoylmethane or Avobenzone, is commercially available under the names of PARSOL® 1789 from Givaudan Roure (International) S. A. (Basel, Switzerland) and EUSOLEX® 9020 from Merck & Co., Inc (Whitehouse Station, NJ). The sunscreen 4-isoproplydibenzoylmethane, which is also known as isopropyldibenzoylmethane, is commercially available from Merck under the name of EUSOLEX® 8020.

In further embodiments, the compositions of the present invention comprise a UVB sunscreen active that absorbs UV radiation having a wavelength of from about 290nm to about 320nm. The compositions comprise an amount of the UVB sunscreen active compound which is safe and effective to provide UVB protection either independently, or

in combination with, other UV protective actives which may be present in the compositions. In some embodiments, the compositions comprise from about 0.1% to abut 16%, more, preferably from about 0.1 % to about 12%, and most preferably from about 0.5% to about 8% by weight, of UVB absorbing organic sunscreen.

A variety of UVB sunscreen actives are suitable for use herein. Nonlimiting examples of such organic sunscreen actives include those described in US Patent No. 5,087,372, US Patent No. 5,073,371, US Patent No. 5,073,372, and Segarin et al., Cosmetics Science and Technology, at Chapter VIII, pages 189 et seq. Additional useful sunscreens include those described in US Patent No. 4,937,370, and US Patent No. 4,999,186. Preferred UVB sunscreen actives are selected from 2-ethylhexyl-2-cyano-3, 2-ethylhexyl N,N-dimethyl-p-aminobenzoate, p-aminobenzoic acid, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, 3 - diphenylacrylate (referred to as octocrylene), 2-phenylbenzimidazole-5-sulphonic acid (PBSA), cinnamates and their derivatives such as 2-ethylhexyl-p-methoxycinnamate and octyl-p-methoxycinnamate, TEA salicylate, octyldimethyl PABA, camphor derivatives and their derivatives, and mixtures thereof. Preferred organic sunscreen actives are 2-ethylhexyl-2-cyano-3, 3-diphenylacrylate (referred to as octocrylene), 2-phenyl-benzimidazole-5-sulphonic acid (PBSA), octyl-p-methoxycinnamate, and mixtures thereof. Salt and acid neutralized forms of the acidic sunscreens are also useful herein.

In some embodiments of the present invention, the compositions further include an agent useful in stabilizing the UVA sunscreen to prevent it from photo-degrading on exposure to UV radiation and thereby maintaining its UVA protection efficacy. A wide range of compounds have been cited as providing these stabilizing properties. It is contemplated that these compounds are chosen to complement both the UVA sunscreen and the composition as a whole. Suitable stabilizing agents include, but are not limited to, those described in US Patents Nos 5,972,316; 5,968,485; 5,935,556; 5,827,508 and WO 00/06110. Preferred examples of stabilizing agents for use in the present invention include 2-ethylhexyl-2-cyano-3, 3-diphenylacrylate (referred to as octocrylene), ethyl-2-cyano-3, 3-diphenylacrylate, 2-ethylhexyl-3, 3-diphenylacrylate, ethyl-3, 3-bis(4-methoxyphenyl)acrylate, and mixtures thereof. 2-ethylhexyl-2-cyano-3, 3-diphenylacrylate is most preferred.

In some embodiments, an agent is added to any of the compositions useful in the present invention to improve the skin, particularly those compositions with enhanced resistance to being washed off by water, or rubbed off. A preferred agent which provides this benefit is a copolymer of ethylene and acrylic acid (*See e.g.*, US Patent No. 4,663,157).

In addition to the organic sunscreens, in some embodiments, the compositions of the present invention additionally comprise inorganic physical sunblocks. Nonlimiting examples of suitable physical sunblocks are described in CTFA International Cosmetic Ingredient Dictionary, 6th Edition, 1995, pp. 1026-28 and 1103; and Sayre et al., J. Soc. Cosmet. Chem., 41:103-109 (1990). Preferred inorganic physical sunblocks include zinc oxide and titanium dioxide, and mixtures thereof.

When used, the physical sunblocks are present in an amount such that the present compositions are transparent on the skin (*i.e.*, non-whitening), preferably less than or equal to about 5%. When titanium dioxide is used, it can have an anatase, rutile, or amorphous structure. Physical sunblock particles (*e.g.*, titanium dioxide and zinc oxide), can be uncoated or coated with a variety of materials including but not limited to amino acids, aluminum compounds such as alumina, aluminum stearate, aluminum laurate, and the like; carboxylic acids and their salts egg stearic acid and its salts; phospholipids such as lecithin; organic silicone compounds; inorganic silicone compounds such as silica and silicates; and mixtures thereof. A preferred titanium dioxide is commercially available from Tayca (Japan) and is distributed by Tri-K Industries (Emerson, NJ) under the MT microionized series (*e.g.*, MT 100SAS). In some embodiments, the compositions of the present invention comprise from about 0.1 % to about 10%, more preferably from about 0.1 % to about 4%, and most preferably from about 0.5% to about 2.5%, by weight, of inorganic sunscreen.

## Antimicrobial and Antifungal Actives

In some embodiments, the compositions of the present invention comprise antimicrobial and/or antifungal actives. Non-limiting examples of antimicrobial and antifungal actives useful herein include, but are not limited to β-lactam drugs, quinolone drugs, ciprofloxacin, norfloxacin, tetracycline, erythromycin, amikacin, 2,4,4'-trichloro-2'-hydroxy diphenyl ether, 3,4,4'-trichlorobanilide, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amanfadine sulfate, octopirox, parachlorometa

xylenol, nystatin, tolnaftate, clotrimazole, cetylpyridinium chloride (CPC), piroctone olamine, selenium sulfide, ketoconazole, triclocarbon, triclosan, zinc pyrithione, itraconazole, asiatic acid, hinokitiol, mipirocin, clinacycin hydrochloride, benzoyl peroxide, benzyl peroxide, minocyclin, phenoxy isopropanol, and mixtures thereof, as well as those described in European Patent No. 0 680 745.

## Other Optional Ingredients

In some additional embodiments, a variety of optional ingredients such as neutralizing agents, perfumes, and coloring agents, find use in the compositions of the present invention. It is preferred that any additional ingredients enhance the skin softness/smoothness benefits of the product. In addition it is preferred that any such ingredients do not negatively impact the aesthetic properties of the product. Thus, high levels of proteins such as collagen and elastin are typically not preferred in compositions useful in the present invention.

In some embodiments, the compositions of the present invention also contain from about 0.01% to about 10%, preferably from about 0.1 % to about 5% of a panthenol moisturizer. In preferred embodiments, the panthenol moisturizer is selected from D-panthenol ([R]-2,4-dihydroxy-N-[3-hydroxypropyl)]-3,3-dimethylbutamide), DL-panthenol, calcium pantothenate, royal jelly, panthetine, pantotheine, panthenyl ethyl ether, pangamic acid, pyridoxin, and pantoyl lactose.

Neutralizing agents suitable for use in neutralizing acidic group containing hydrophilic gelling agents herein include sodium hydroxide, potassium hydroxide, ammonium hydroxide, monoethanolamine, triethanolamine, amino methyl propanol, tris-buffer and triethanolamine.

Other optional materials include keratolytic agents; water-soluble or solubilizable preservatives preferably at a level of from about 0.1 % to about 5%, such as Germall 115, methyl, ethyl, propyl and butyl esters of hydroxybenzoic acid, benzyl alcohol, DMDM hydantoin iodopropanyl butylcarbanate available under the trade name Glydant Plus from Lonza, EDTA, Euxyl (RTM) K400, Bromopol (2-bromo-2-nitropropane-1,3-diol) and phenoxypropanol; anti-bacterials such as Irgasan (RTM) and phenoxyethanol (preferably at levels of from 0.1 % to about 5%); soluble or colloidally-soluble moisturising agents such as hylaronic acid and starch-grafted sodium polyacrylates such as Sanwet (RTM) IM-1000, IM-1500 and IM-2500 available from Celanese Superabsorbent Materials, Portsmith, VA, and described in US Patent No. 4,076,663; vitamins such as vitamin A, vitamin C, vitamin E and derivatives thereof and building blocks thereof such as phytantriol and vitamin K and components thereof such as the fatty alcohol dodecatrienol; alpha and beta hydroxyacids; aloe vera; sphingosines and phytosphingosines, cholesterol; skin whitening agents; N-acetyl cysteine; coloring agents; antibacterial agents such as TCC/TCS, also known as triclosan and trichlorocarbon; perfumes and perfume solubilizers. Examples of alpha hydroxy acids include glycolic acid, lactic acid, malic acid, citric acid, glycolic acid in conjunction with ammonium glycolate, alpha-hydroxy ethanoic acid, alpha-hydroxyoctanoic acid, alpha-hydroxycaprylic acid, hydroxycaprylic acid, mixed fruit acid, tri-alpha hydroxy fruit acids, triple fruit acid, sugar cane extract, alpha hydroxy and botanicals, such as those comprising I-alpha hydroxy acid and glycomer in crosslinked fatty acids alpha nutrium. Preferred examples of alpha hydroxy acids are glycolic acid and lactic acid. It is preferred that alpha hydroxy acids are used in levels of up to 10%.

In some embodiments, a safe and effective amount of an anti-inflammatory agent is added to the compositions of the present invention, preferably from about 0.1 % to about 5%, more preferably from about 0.1 % to about 2%, of the composition. The anti-inflammatory agent enhances the skin appearance benefits of the present invention (*e.g.*, such agents contribute to a more uniform and acceptable skin tone or colour). The exact amount of anti-inflammatory agent to be used in the compositions will depend on the particular anti-inflammatory agent utilized since such agents vary widely in potency.

In further embodiments, compositions of the present invention further include an anti-oxidant/radical scavenger. The anti-oxidant/radical scavenger is especially useful for providing protection against UV radiation which can cause increased scaling or texture changes in the stratum corneum and against other environmental agents which can cause skin damage. Suitable amounts are from about 0.1 % to about 10%, more preferably from about 1% to about 5%, of the composition. Anti-oxidants/radical scavengers include compounds such as ascorbic acid (vitamin C) and its salts.

The inclusion of a chelating agent in some embodiments of the present invention, is especially useful for providing protection against UV radiation which can contribute to excessive scaling or skin texture changes and against other environmental agents which can cause skin damage. A suitable amount is from about 0.01% to about 1%, more preferably from about 0.05% to about 0.5%, of the composition. Exemplary chelators that are useful herein include those described in US Patent No. 5,487,884. Preferred chelators useful in compositions of the subject invention include ethylenediamine tetraacetic acid (EDTA), furildioxime, and derivatives thereof.

In still further embodiments, the compositions of the present invention also comprise a skin lightening agent. When used, the compositions preferably comprise from about 0.1 % to about 10%, more preferably from about 0.2% to about 5%, also preferably from about 0.5% to about 2%, of a skin lightening agent. Suitable skin lightening agents include those known in the art, including kojic acid, arbutin, ascorbic acid and derivatives thereof (*e.g.*, magnesium ascorbyl phosphate). Further skin lightening agents suitable for use herein also include those described in WO 95/34280 and

WO 95/23780; each incorporated herein by reference.

Other optional materials include water-soluble or solubilizable preservatives preferably at a level of from about 0.1 % to about 5%, such as Germall 115, methyl, ethyl, propyl and butyl esters of hydroxybenzoic acid, benzyl alcohol, DMDM hydantoin iodopropanyl butylcarbanate available under the trade name Glydant Plus (Lonza), EDTA, Euxyl (RTM) K400, Bromopol (2-bromo-2-nitropropane-1,3-diol) and phenoxypropanol; anti-bacterials such as Irgasan (RTM) and phenoxyethanol (preferably at levels of from 0.1 % to about 5%). Antibacterial agents such as TCC/TCS, also known as triclosan and trichlorocarbon are also useful in compositions of the present invention.

Other optional materials herein include pigments which, when water-insoluble, contribute to and are included in the total level of oil phase ingredients. Pigments suitable for use in the compositions of the present invention can be organic and/or inorganic. Also included within the term "pigment" are materials having a low colour or luster such as matte finishing agents, and also light scattering agents. Preferably, the compositions of the present invention comprise particulate materials having a refractive index of from about 1.3 to about 1.7, the particulate materials being dispersed in the composition and having a median particle size of from about 2 to about 30 μm. Preferably the particulates useful herein have relatively narrow distributions, by which is meant that more than 50% of the particles fall within 3 μm either side of the respective median value. It is also preferred that more than 50%, preferably more than 60%, and even more preferably more than 70% of particles fall within the size ranges prescribed for the respective median values. Suitable particulate materials include organic or organosilicone and preferably organosilicone polymers. Preferred particles are free-flowing, solid, materials. By "solid" is meant that the particles are not hollow. The void at the center of hollow particles can have an adverse effect on refractive index and therefore the visual effects of the particles on either skin or the composition. Suitable organic particulate materials include those made of polymethylsilsesquioxane, referenced above, polyamide, polythene, polyacrylonitrile, polyacrylic acid, polymethacrylic acid, polystyrene, polytetrafluoroethylene (PTFE) and poly(vinylidene chloride). Copolymers derived from monomers of the aforementioned materials can also be used. Inorganic materials include silica and boron nitride. Representative commercially available examples of useful particulate materials herein are Tospearl® 145 which has a median particle size of about 4.5 μm and EA-209® from Kobo which is an ethylene / acrylic acid copolymer having a median particle size of about 10 μm, Nylon-12 available under the trade name Orgasol 2002 from Elf Atochem, France, or mixtures thereof.

Further examples of suitable pigments include titanium dioxide, predispersed titanium dioxide from Kobo (*e.g.*, Kobo GWL75CAP), iron oxides, acyglutamate iron oxides, ultramarine blue, D&C dyes, carmine, and mixtures thereof. Depending upon the type of composition, a mixture of pigments will often find use. The preferred pigments for use herein from the viewpoint of moisturisation, skin feel, skin appearance and emulsion compatibility are treated pigments. The pigments can be treated with compounds such as amino acids, silicones, lecithin and ester oils.

Suitably, the pH of the compositions herein is in the range from about 6.1 to about 10.0, wherein the pH of the final composition is adjusted by addition of acidic, basic or buffer salts as necessary.

## Preparation of Compositions

The compositions of the present invention are prepared by standard techniques well known to those skilled in the art. In general, the aqueous phase and/ or the oil phase are prepared separately, with materials of similar phase partitioning being added in any order. If the final product is an emulsion, the two phases are then combined with vigorous stirring. Any ingredients in the formulation with high volatility, or which are susceptible to hydrolysis at high temperatures, can be added with gentle stirring towards the end of the process, post emulsification if applicable.

Proteases with reduced allergenicity/immunogenicity also find use in the treatment of textiles. "Textile treatment" comprises a process wherein textiles, individual yarns or fibers that can be woven, felted or knitted into textiles or garments are treated to produce a desired characteristic. Examples of such desired characteristics are "stone-washing," depilling, dehairing, desizing, softening, and other textile treatments well known to those of skill in the art.

## DETAILED DESCRIPTION OF THE INVENTION

The present invention relates to methods for the identification of $CD4^+$ T-cell epitopes in subtilisin Carlsberg proteins. The present invention also provides for the production of altered peptides which, when incorporated into a wild-type subtilisin Carlsberg protein produce an altered immunogenic response, which is a lowered immunogenic response in humans. In particular, the present invention provides means, including methods and compositions suitable for reducing the immunogenicity of ALCALASE@ enzyme. The present invention also provides for the production of altered peptides which when incorporated into a wild-type subtilisin Carlsberg protein sequence, are no longer capable of initiating the $CD4^+$T-cell response or at least reduce the allergic response. In particular, the present invention provides means, including methods and compositions suitable for reducing the immunogenicity of a wild-type subtilisin Carlsberg.

The present invention relates to T-cell epitopes of the ALCALASE® enzyme. These epitopes are provided in various sequences set forth herein (*See*, Figures 2 and 3) and include but are not limited to peptide number 1 (SEQ ID

NO.2); peptide number 7 (SEQ ID NO:8); peptide number 14 (SEQ ID NO:15); peptide umber29 (SEQ ID NO:30). In an embodiment of the present invention, altered sequences of the identified epitopes are suitable for substitution into the ALCALASE® enzyme.

The present invention relates to methods for the identification of CD4$^+$T-cell epitopes in wild-type subtilisin Carlsberg proteins. The invention provides for the production of peptides that are no longer capable of initiating the CD4$^+$T-cell response. In particular, the present invention provides means, including methods and compositions suitable for reducing the immunogenicity of ALCALASE® enzyme.

The development of an antibody to a protein requires a series of events that begin with a peptide segment derived from that protein being presented on the surface of an activated antigen presenting cell (APC). The peptide is associated with a specific protein on the surface of the antigen presenting cell, namely a protein in the major histocompatibility complex (MHC) (in humans, the MHC is referred to as the "human leukocyte antigen" (HLA) system). The bound peptide is capable of interacting with a second cell type, the T-cell. Specifically, the T-cell is of the subtype recognized by the expression of the CD4 protein on its surface (*i.e.*, CD4$^+$T-cell). If the interaction is successful, the specific CD4$^+$T-cell grows and divides (*i.e.*, proliferates) and becomes capable of interacting with an antibody-producing cell (*i.e.*, B-cell). If that interaction is successful, the B-cell proliferates and becomes a center for the production of antibodies that are specifically directed against the original protein. Thus the ultimate production of an antibody is dependent on the initial activation of a CD4$^+$ T-cell that is specific for a single peptide sequence (*i.e.*, an epitope). Using the compositions and methods described herein, it is possible to predict which peptides from a target protein, such as wild-type subtilisin Carlsberg proteins will be capable of the initial activation of specific CD4$^+$ T-cells.

In some preferred embodiments of the present invention, subtilisin Carlsberg proteins include: i) ALCALASE® enzyme (SEQ ID NO:1); ii) subtilisin Carlsberg proteins having similar catalytic activity to ALCALASE® enzyme and having at least about 80%, 85%, 90%, 95%, 97%, 98% or 99% amino acid sequence identity to SEQ ID NO:1, and preferably at least 90%, at least 95% or at least 97% amino acid sequence identity to SEQ ID NO:1.

Subtilisins are serine proteases (typically, bacterial and fungal) which generally act to cleave peptide bonds. While amino acid sequences of the subtilisins are not entirely homologous, the subtilisins exhibit the same or similar type of proteolytic activity and have a common amino acid sequence defining a catalytic triad which distinguishes them from the related class of serine proteases, chymotrypsin. The catalytic triad is, reading from amino to carboxy terminus, aspartate-histidine-serine. The wild-type subtilisin Carlsberg proteins and the modified proteins herein have this catalytic triad.

Subtilisin Carlsberg proteins having similar catalytic activity to the ALCALASE® enzyme as shown in P00780 and Figure 1 (SEQ ID NO:1), include those subtilisin Carlsberg proteins obtainable from *Bacillus licheniformis*, such as, but not limited to subtilisins having EMBL Accession code X91262, EMBL Accession code X91261, and EMBL Accession code X91260. The catalytic domains of these subtilisins have greater than 90% amino acid sequence identity, with the ALCALASE® enzyme. In a preferred embodiment, these wild-type subtilisin Carlsberg proteins have at least one and preferably between 1 and 6 significant epitopes in common with the identified significant ALCALASE@ enzyme epitopes of SEQ ID NO:1.

In some embodiments of the present invention, the variants of subtilisin Carlsberg proteins as defined in the claims are naturally occurring (*e.g.*, obtained from *Bacillus licheniformis* strains), while in other embodiments, they are genetically engineered variants (*i.e.*, recombinant proteins). These variants include recombinant proteins with alterations in one or more amino acid residues, wherein the altered amino acid residue is found in a position other than in a significant epitope. For example, in some embodiments, variants include one or more alterations to an amino acid residue located at a position corresponding to positions 1-15, 19-33, 40-54 and 85-99; of SEQ ID NO: 1. In other embodiments, variants include two or more amino acid alterations to amino acids located at positions corresponding to positions 1-15, 19-33, 40-54, and 85-99 of SEQ ID NO:1. In still further embodiments, the variants include between 2 and 10 alterations or 2 and 6 alterations to amino acid residues located at a position corresponding to positions 1-15, 19-33, 40-54 and 85-99 of SEQ ID NO:1. In some embodiments, the alterations include substitutions, deletions and/or insertions of an amino acid or amino acid sequence, wherein the alteration results in an altered phenotype of the enzyme. For example, in some embodiments, the alteration(s) results in enhanced stability, enhanced enzymatic activity, enhanced thermal stability, increased alkaline stability and/or other desired properties.

Alteration or modification to one or more significant epitopes resulting in a modified wild-type subtilisin Carlsberg protein, which includes a modified ALCALASE or modified variant subtilisin Carlsberg, may include a) modification of the epitope by substitution, deletion or insertion of one or more amino acids in the epitope or b) modification of the epitope by substitution with an analogous sequence from a homologous protein which analogous sequence produces a lesser immunogenic response due to T-cell recognition than the parent wild-type subtilisin Carlsberg.

In some embodiments of the present invention, a significant epitope is modified by the substitution, deletion and/or insertion of at least one, two three, four, five, six, seven and as many as fifteen amino acid residues in the epitope. For example, in one preferred embodiment, peptide number 7 (SEQ ID NO: 8; corresponding to amino acid residue positions 19-33 of SEQ ID NO:1) is altered. In these embodiments, the altered amino acid sequence comprises a

substitution of one or more positions of 19, 20, 21, 22, 23, 24, 25, 26 27 28, 29, 30, 31, 32, and/or 33 (*e.g.* positions 19 - 33). Substitutions are made by replacing the wild-type amino acid residue with a different amino acid. In some preferred embodiments, replacement is preferred using one of the natural L-amino acids (*i.e.*, Ala, Asn, Asp, Cys, Glu, Gly, Phe, His, Ile, Lys, Leu, Met, Gln, Ser, Thr, Trp, Tyr and Val). In further embodiments, the altered peptide is peptide number 7, comprising a deletion of one or more amino acid residues corresponding to positions 19-33 of SEQ ID NO:1. In still further embodiments, the altered peptide corresponds to peptides 7 and 8, with the sequence QGFKGANVKVAVLDTGIQ (SEQ ID NO:90). Thus, various modifications in the epitopes of interest are provided which provide reduced immunogenicity to the variant subtilisin Carlsberg proteins.

In further embodiments of the invention, a modified wild-type subtilisin Carlsberg protein includes the substitution of an analogous epitope segment from a homologous protease, wherein the analogous epitope segment produces a reduced immunogenic response (*e.g.*, a reduced T-cell response) compared to the epitope which has been substituted in the wild-type parent. For example in some embodiments, peptide number 7 (*i.e.*, corresponding to positions 19-33 of SEQ ID NO:1) is replaced with an analogous segment from another homologous subtilisin such as subtilisin BPN' from B. *amyloliquefaciens* or subtilisin 168 from *B. subtilis*, wherein the analogous segment is not a significant epitope. In some embodiments, the homologous protease is obtained from a prokaryotic organism, while in other embodiments, it is obtained from an eukaryotic organism. Examples of suitable prokaryotic organisms include, but are not limited to such Gram-negative organisms as *E. coli* and *Pseudomonas spp*. and such Gram-positive microorganisms as *Micrococcus spp*. and *Bacillus spp.*

In some embodiments, the epitopes of the wild-type subtilisin Carlsberg proteins are modified by methods well known in the art. (*See e.g.*, Zoller et al., Nucl. Acids Res.,10:6487-6500 [1982]; and Yuckenberg et al., (1991), in McPherson (ed.), Directed Mutagenesis: A Practical Approach, [1991], at pp.27 - 48). As indicated above, these modifications include amino acid residue deletion, substitution, and/or insertion. For example one or more amino acid residues are modified by site-specific amino acid substitutions. Indeed, commercially available mutagenesis kits find use in producing these variant proteins.

In some embodiments amino acid residues identified for substitution, insertion and/or deletion are conserved residues, whereas in other embodiments they are not. In preferred embodiments involving residues which are not conserved, the replacement of one or more amino acids is limited to substitutions which produce a modified peptide with an amino acid sequence that does not correspond to one found in nature. In the case of conserved residues, such replacements do not result in a naturally-occurring sequence.

Cassette mutagenesis also finds use in the present invention to facilitate the construction of the modified proteins of the present invention. According to this method, the naturally-occurring gene encoding the protein is obtained and sequenced in whole or in part. Then, the sequence is scanned for a point at which it is desired to make a mutation (*e.g.*, deletion, insertion or substitution) of one or more amino acids in the encoded protein. The sequences flanking this point are evaluated for the presence of restriction sites for replacing a short segment of the gene with an oligonucleotide pool which when expressed will encode various mutants. Such restriction sites are preferably unique sites within the protein gene so as to facilitate the replacement of the gene segment. However, any convenient restriction site which is not overly redundant in the protein gene finds use with the present invention, provided that the gene fragments generated by restriction digestion can be reassembled in proper sequence. If restriction sites are not present at locations within a convenient distance from the selected point (*e.g.*, from 10 to 15 nucleotides), such sites are generated by substituting nucleotides in the gene such that neither the reading frame nor the amino acids encoded are changed in the final construction. In some embodiments, mutation of the gene in order to change its sequence to conform to the desired sequence is accomplished by M13 primer extension in accord with generally known methods. The task of locating suitable flanking regions and evaluating the needed changes to arrive at two convenient restriction site sequences is made routine by the redundancy of the genetic code, a restriction enzyme map of the gene and the large number of different restriction enzymes. Note that if a convenient flanking restriction site is available, the above method need be used only in connection with the flanking region which does not contain a restriction site. However, it is not intended that the present invention be limited to these means, as other suitable methods known to those in the art find use in the present invention.

In some embodiments, once the DNA (either naturally-occurring or recombinant) is cloned, the restriction sites flanking the positions to be mutated are digested with the cognate restriction enzymes and a plurality of end termini-complementary oligonucleotide cassettes are ligated into the gene. The mutagenesis is simplified by this method because all of the oligonucleotides can be synthesized so as to have the same restriction sites, and no synthetic linkers are necessary to create the restriction sites.

When a peptide comprising the altered epitope is analyzed in the assay of the present invention, it preferably results in lesser T-cell proliferation than the peptide comprising the wild-type subtilisin. More preferably when altered, the epitope produces less than three times the baseline T-cell proliferation, preferably less than two times the baseline T-cell proliferation and most preferably less than or substantially equal to the baseline T-cell proliferation in a sample.

In some embodiments, the wild-type subtilisin Carlsberg proteins and modified proteins thereof are screened

for proteolytic activity according to methods well known in the art. Such methods include, but are not limited to the pNA assay and the dimethyl casein (DMC) assay method (Rothgeb et al., J. Am Oil Chem. Soc., 65:806 [1988]).

Application of recombinant DNA technology facilitates the rapid manipulation of protein or peptide sequences by changing the DNA sequence encoding a protein or peptide (*i.e.*, a protein or peptide of interest). Application of this strategy to the gene coding for a modified subtilisin Carlsberg protein, such as modified ALCALASE@ enzyme facilitates changing the sequence of the epitopes, such that they are no longer capable of activating $CD4^+$ T-cells. In preferred embodiments, these changes reduce the propensity of a subtilisin Carlsberg to produce an antibody-binding antibodies (Bab) and/or neutralizing antibodies (Nab) response in humans. Thus, described herein are compositions and methods for the identification of $CD4^+$ T-cell epitopes in the ALCALASE@ enzyme protein sequence and production of peptides which are no longer capable of initiating the $CD4^+$T-cell response which, when incorporated through recombinant DNA technology, into the ALCALASE® enzyme, are contemplated to reduce the capability of ALCALASE® enzyme to initiate the production of antibodies.

Therefore, in certain embodiments, a DNA sequence encoding a modified wild-type subtilisin Carlsberg as defined in the claims (particularly a modified ALCALASE® enzyme) is introduced into a host cell via an expression vector capable of replicating within the host cell. Those of skill in the art are well aware of suitable vectors for use in host cells, such as *Bacillus* host cells (*See e.g.* Harwood and Cutting (eds.), Molecular Biological Methods for Bacillus, John Wiley & Sons, (1990), at page 92). Transformation techniques are further described in Chang and Cohen, Mol. Gen. Genet., 168: 11-115 [1979]; and Smith et al., Appl. and Env. Microbiol., 51:634 [1986]). In some embodiments, the DNA sequence is directly introduced into a host cell, without insertion into an expression vector. Such methods are well known in the art and include but are not limited to calcium chloride precipitation, electroporation, naked DNA, etc.

In some embodiments, the modified wild-type subtilisin Carlsberg proteins of the present invention are further isolated and/or purified. This is accomplished by separation techniques known in the art, including but not limited to, ion exchange chromatography, affinity chromatography, hydrophobic separation, precipitation, filtration, microfiltration, gel electrophoresis, and any other suitable method. In additional embodiments, once the protein is isolated and/or purified, further constituents are added to the modified proteins to provide compositions of interest.

There are numerous applications for subtilisin Carlsberg proteins, including ALCALASE® enzyme, including but limited to liquid and powered detergents, textile treatment formulations, conventional cleaning compositions and personal care compositions. It is readily understood that the subtilisins which include the modified epitopes of the present invention find use for any purpose in which ALCALASE® enzyme finds use.

## EXPERIMENTAL

The following Examples serve to illustrate certain preferred embodiments and aspects of the present invention and are not to be construed as limiting the scope thereof.

In the experimental disclosure which follows, the following abbreviations apply: eq (equivalents); M (Molar); μM (micromolar); N (Normal); mol (moles); mmol (millimoles); μmol (micromoles); nmol (nanomoles); g (grams); mg (milligrams); kg (kilograms); μg (micrograms); L (liters); ml (milliliters); μl (microliters); cm (centimeters); mm (millimeters); μm (micrometers); nm (nanometers); ° C. (degrees Centigrade); h (hours); min (minutes); sec (seconds); msec (milliseconds); xg (times gravity); Ci (Curies); OD (optical density); Dulbecco's phosphate buffered solution (DPBS); HEPES (N-[2-Hydroxyethyl]piperazine-N-[2-ethanesulfonic acid]); HBS (HEPES buffered saline); SDS (sodium dodecylsulfate); Tris-HCl (tris[Hydroxymethyl]aminomethane-hydrochloride); Klenow (DNA polymerase I large (Klenow) fragment); rpm (revolutions per minute); EGTA (ethylene glycol-bis(β-aminoethyl ether) N, N, N', N'-tetraacetic acid); EDTA (ethylenediaminetetracetic acid); ATCC (American Type Culture Collection, Rockville, MD); Cedar Lane (Cedar Lane Laboratories, Ontario, Canada); Gibco/Life Technologies (Gibco/Life Technologies, Grand Island, NY); Sigma (Sigma Chemical Co., St. Louis, MO); Pharmacia (Pharmacia Biotech, Piscataway, NJ); Procter & Gamble (Procter and Gamble, Cincinnati, OH); Genencor (Genencor International, Palo Alto, CA); Endogen (Endogen, Woburn, MA); Cedarlane (Cedarlane, Toronto, Canada); Dynal (Dynal, Norway); Novo (Novo Industries A/S, Copenhagen, Denmark); Biosynthesis (Biosynthesis, Louisville, TX); TriLux Beta, (TriLux Beta, Wallac, Finland); DuPont/NEN (DuPont/NEN Research Products, Boston, MA); TomTec (Hamden, CT); and Stratagene (Stratagene, La Jolla, CA).

## EXAMPLE 1

### Preparation of Cells Used in the Assay System for the Identification of Peptide T-Cell Epitopes in ALCALASE® Using Human T-Cells

Fresh human peripheral blood cells were collected from 92 humans of unknown exposure status to ALCALASE® enzyme. These cells were tested to determine antigenic epitopes in ALCALASE®, as described in Example 3.

Peripheral mononuclear blood cells (stored at room temperature, no older than 24 hours) were prepared for

use as follows: Approximately 30 mls of a solution of buffy coat preparation from one unit of whole blood was brought to 50 ml with Dulbecco's phosphate buffered solution (DPBS) and split into two tubes. The samples were underlaid with 12.5 ml of room temperature Lymphoprep density separation media (Nycomed; density 1.077 g/ml). The tubes were centrifuged for thirty minutes at 600 x gravity (g). The interface of the two phases was collected, pooled and washed in DPBS. The cell density of the resultant solution was measured by hemocytometer, as known in the art. Viability was measured by trypan blue exclusion, as known in the art.

From the resulting solution, a differentiated dendritic cell culture was prepared from the peripheral blood mononuclear cell sample having a density of $10^8$ cells per 75 ml culture flask in a solution as described below:

(1) 50 ml of serum free AIM V media (Gibco/BRL) was supplemented with a 1:100 dilution beta-mercaptoethanol (Gibco/BRL). The flasks were laid flat for two hours at 37°C in 5% $CO_2$ to allow adherence of monocytes to the flask wall;

(2) Differentiation of the monocyte cells to dendritic cells was performed as follows: nonadherent cells were removed and the resultant adherent cells (monocytes) combined with 30 ml of AIM V, 800 units/ml of GM-CSF (Endogen) and 500 units/ml of IL-4 (Endogen); the resulting mixture was cultured for 5 days at 37°C in 5% $CO_2$. After the five days of incubation, the cytokine TNF$\alpha$ (Endogen) was added to 0.2 units/ml, and the cytokine IL-1$\alpha$ (Endogen) was added to a final concentration of 50 units/ml and the mixture incubated at 37°C in 5% $CO_2$ for two more days.

(3) On the seventh day, mitomycin C was added to a concentration of 50 micrograms/ml in 100 mM EDTA-containing phosphate buffered saline (PBS) to stop growth of the now differentiated dendritic cell culture. The solution was incubated for 60 minutes at 37°C in 5% $CO_2$. Dendritic cells were dislodged from the plastic surface by gently tapping the flask. Dendritic cells were then centrifuged at 600 x g for 5 minutes, washed in DPBS and counted as described above.

(4) The prepared dendritic cells were placed into a 96-well round bottom plate at a concentration of $2x10^4$ cells/well in 100 microliter total volume of AIM V media, per well.

$CD4^+$ T cells were prepared from frozen aliquots of the peripheral blood cell samples used to prepare the dendritic cells, using reagents provided by the Dynal $CD4^+$ T-cell enrichment kit (Dynal). The resultant $CD4^+$ cell solution was centrifuged, resuspended in AIM V media and the cell density was determined using methods known in the art. The $CD4^+$T-cell suspension was then resuspended to a count of $2x10^6$ cells/ml in AIM V media to facilitate efficient manipulation of 96-well plates.

## EXAMPLE 2

### Identification of T-Cell Epitopes in ALCALASE® for Use in the Assay System

Peptides for use in the assay described in Example 3 were prepared based upon the full-length amino acid sequence (SEQ ID NO:1) of ALCALASE® enzyme, 15mers comprising the entire sequence of ALCALASE® were synthetically prepared. Consecutive peptides overlapped by 12 amino acids. A total of 88 peptides (SEQ ID NOS: 2-89) were created, the sequences of which are provided in Figure 2.

Peptide antigens were prepared as a 2 mg/ml stock solutions in DMSO. First, 0.5 microliters of the stock solution were placed in each well of the 96-well plate in which the differentiated dendritic cells were previously placed. Then, 100 microliters of the diluted $CD4^+$ T-cell solution as prepared above, were added to each well. Useful controls included diluted DMSO blanks, and tetanus toxoid positive controls.

The final concentrations in each well, at 20 microliter total volume were as follows:

$2x10^4$ $CD4^+$ T-cells
$2x10^5$ dendritic cells (R:S of 10:1)
5 $\mu$M peptide

## EXAMPLE 3

### Assay for the Identification of Peptide T-Cell Epitopes in ALCALASE® Enzyme Using Human T-Cells

Once the assay reagents (*i.e.*, cells, peptides, etc.) were prepared and distributed into the 96-well plates, the assays were conducted. Controls included dendritic cells plus CD4+ T-cells alone (with DMSO carrier) and with tetanus toxoid (Wyeth-Ayerst, Philadelphia, PA), at approximately 5 Lf/mL.

Cultures were incubated at 37°C in 5% $CO_2$ for 5 days. Tritiated thymidine (NEN) was added at 0.5 microCi/well. The cultures were harvested and assessed for incorporation the next day, using the Wallac TriBeta scintillation

detection system.

All of the tests were performed at least in duplicate. All of the tests reported displayed robust positive control responses to the antigen tetanus toxoid. Responses were averaged within each experiment, then normalized to the baseline response. A positive event (*i.e.*, a proliferative response) was recorded if the response was at least 2.95 times the baseline response.

The immunogenic responses (*i.e.*, T-cell proliferation) to the prepared peptides from ALCALASE® enzyme were tallied for all 92 donors and are illustrated in Figure 3. Six significant epitopes were identified. Peptides identified as being of particular interest include peptides 1, 7 and 8, 14, 29 and 39, as indicated in Figures 2 and 3. These peptides correspond to the following sequences:

| Peptide # | Sequence | SEQ ID NO: |
|---|---|---|
| 1 | AQTVPYGIPLIKADK | 2 |
| 7-8 | QGFKGANVKVAVLDTGIQ | 90 |
| 14 | PDLNVVGGASFVAGE | 15 |
| 29 | PSVSLYAVKVLNSSG | 30 |
| 39 | TNGMDVINMSLGGPS | 40 |

The overall background rate of responses to this peptide set was 2.35 ± 2.56%, for the donors tested. In the above Table, peptide 7-8 is a continuous sequence of 18 amino acids (peptide #7, please the 3 unique amino acids contributed by peptide #8). This combination of peptides 7 and 8 was combined due to the good responses observed for both peptides.

## EXAMPLE 4

### Assay of Variant Peptides

Peptide numbers 7 and 29 are selected for further analysis. Sets of altered peptides are constructed based on the sequences of peptide number 7 and peptide number 29, wherein the amino acid residues are modified from the parent sequence. This can be accomplished by a commercial vendor such as Mimostopes (San Diego). An alanine scan is performed for each peptide (*See e.g.*, Harris, et al., Immunol., 84:555-561 [1995]; and Maillere et al., Mol. Immunol., 32:1073-1080 [1995]). The assay is performed as described in Example 3, utilizing the altered peptides on a set of donor samples. Proliferative responses are collated.

In one embodiment, an altered peptide is considered useful for creating a hypoallergenic protein molecule (*i.e.*, a modified subtilisin protein according to the invention) if at least one and preferably all three of the following criteria are met: (1) all donors who respond with a stimulation index (SI) of about greater than 2.95 to the parent peptide respond to the altered peptide with an SI of about 1.0 or less; (2) all donors who respond weakly to the parent peptide (with an SI greater than about 1.0 but less than about 2.95 ) respond to the altered peptide with an SI of about 1.0 or less; and (3) all non-responders to the parent peptide are also non-responders to the altered peptide. SI is a measure of the T-cell profilerative response of a peptide compared to a control peptide. A SI is calculated for each donor for each peptide.

## EXAMPLE 5

### HLA Association with an Epitope Peptide Number

**[0192]** The HLA-DR and DQ expression of all the donors tested in both rounds of assay testing described above are assessed using a commercially available PCR-based HLA typing kit (Bio-Synthesis). In some embodiments, the phenotypic frequencies of individual HLA-DR and -DQ antigens among responders and non-responders to a peptide number are tested using a chi-squared analysis with 1 degree of freedom. The increased or decreased likelihood of reacting to an epitope corresponding to the peptide number is calculated wherever the HLA antigen in question is present in both responding and non-responding donor samples and the corresponding epitope is considered an HLA associated epitope.

The magnitude of the proliferative response to an individual peptide in responders and non-responders expressing epitope-associated HLA alleles may also be analyzed. An "individual responder to the peptide" is defined by a stimulation index of greater than 2.95. It is contemplated that the proliferative response in donors who express an epitope associated with HLA alleles would be higher than in peptide responders who do not express the associated allele.

From the above, described herein are methods and compositions for the identification of T-cell epitopes in wild-type subtilisin Carlsberg, such as ALCALASE®. Once antigenic epitopes are identified, the epitopes are modified as

desired, and the peptide sequences of the modified epitopes incorporated into a wild-type subtilisin Carlsberg, so that the modified sequence is no longer capable of initiating the $CD4^{+}$T-cell response or wherein the $CD4^{+}$T -cell response is significantly reduced in comparison to the wild-type parent. In particular, the present invention provides means, including methods and compositions suitable for reducing the immunogenicity of ALCALASE®.

## EXAMPLE 6

### Hydrolysis of Dimethyl Casein ("DMC") by Mutant Variant Subtilisin

Mutant variant subtilisins, isolated and purified by the methods described herein, are analyzed for their ability to hydrolyze a commercial synthetic substrate, di-methyl casein (Sigma C-9801). A 5 mg/ml DMC substrate solution is prepared in the appropriate buffer (5 mg/ml DMC, 0.005% (w/w) Tween 80® (polyoxyethylene sorbitan mono-oleate, Sigma P-1754)). Appropriate DMC substrate buffers are prepared (*e.g.*, 50 mM sodium acetate for pH 5.5; 50 mM N-tris(hydroxymethyl)methyl-2-aminoethane sulfonic acid ("TES") for pH 6.5; 50 mM piperazine-N-N'-bis-2-ethane sulfonic acid ("PIPES") for pH 7.5; and 50 mM Tris for pH 8.5). To begin testing, 200 μl of the desired pH substrate are placed into the wells of a microtiter plate (*e.g.*, a 96 well plate) and pre-incubated at 37° C for twenty minutes prior to enzyme addition. A 2,4,6-trinitrobenzene sulfonate salt ("TNBS") color reaction method is used to determine activity on a Spectra Max 250 spectrophotometer. This assay measures the enzymatic hydrolysis of DMC into peptides containing free amino groups. These amino groups react with 2,4,6-trinitro-benzene sulfonic acid to form a yellow colored complex.

Thus, the more deeply colored the reaction, the more activity is measured. The TNBS detection assay can be performed on the supernatant after two hours of incubation at 37° C. A 1 mg/ml solution of TNBS is prepared in a solution containing 2.4 g NaOH, 45.4 g $Na_2B_4)_7 \cdot 10H_2O$ dissolved by heating in 1000ml. From this solution, 60 μl are aliquoted into a 96-well microtiter plate. Then, 10 μl of the incubated enzyme solution described above is added to each well and mixed for 20 minutes at room temperature. Then, 20 μl of $NaH_2PO_4$ solution (70.4 g $NaH_2PO_4 \cdot H_2O$ and 1.2 g $Na_2SO_3$ in 2000 ml) are mixed for 1 minute in the wells to stop the reaction and the absorbance at 405 nm in a SpectraMax 250 spectrophotometer is determined. A blank (same TNBS solution, but without the enzyme) is also be prepared and tested. The hydrolysis is measured by the following formula:

$$\text{Absorbance}_{405}\text{ (Enzyme solution)} - \text{Absorbance}_{405}\text{ (without enzyme)}$$

at varying enzyme concentrations (0, 2.5, 5, 7.5, and 10 ppm). The comparative ability of the mutant variants to hydrolyze such substrate versus subtilisins from a known mutant variant (*e.g.* a characterized mutant subtilisin) can be determined in this manner.

## EXAMPLE 7

### Hydrolysis of Collagen, Elastin, and Keratin by Variant Subtilisin Carlsberg Enzymes

Mutant variant subtilisin Carlsberg enzymes isolated and purified by the methods described above, are often analyzed for their ability to hydrolyze commercial substrates, for example bovine collagen (Sigma C-9879), bovine elastin (Sigma E-1625), and/or bovine keratin (ICN Biomedical 902111). A 5 mg/ml substrate solution is prepared (in 0.005% Tween 80® ). Each substrate is prepared in the appropriate pH as known in the art (*e.g.*, pH 5.5, 6.5, 7.5, and 8.5). To test, 1.5 ml of the each substrate is transferred into 24-well Costar plate at 37° C. The plates are pre-incubated at 37° C for twenty minutes prior to enzyme addition. A TNBS detection assay as described above is performed on the supernatant after two hours of incubation at 37 ° C.

It is contemplated that these assays will find use in demonstrating the comparative ability of the mutant variants to hydrolyze such substrates versus subtilisins from a known mutant variant. In most case, it is contemplated that the mutated enzymes will typically show significant hydrolysis of collagen, elastin and keratin substrates at different pHs and different enzyme concentrations, as compared to each other and wild-type enzyme.

## EXAMPLE 8

### Thermal Stability of Protein Variants in Piperazine-N-N'-bis-2-ethane sulfonic acid ("PIPES") Buffer

In these experiments, the thermal stability of the protein (*e.g.*, subtilisin Carlsberg) variants in PIPES is determined. Typically, these determinations are conducted using a PCR thermocycler of the type Stratagene Robocycler.

The stability of 5.0 ppm enzyme 5.0 ppm enzyme (*e.g.*, the mutants of interest and control mutant enzyme(s)) are tested at five timepoints (*e.g.*, 5, 10, 20, 40, and 60 minutes) at pH 6.5, for each temperature. For example, the samples are tested at two degree intervals ranging from 42 - 56° C, and at every other degree at temperatures ranging from 42-56°C, in the PCR thermocycler gradient. In these experiments, a 50mM PIPES buffer is prepared (50 mM PIPES, 0.005% Tween 80®). Typically, the pH is adjusted to 6.5. However, it is not intended that the present invention be limited to this particular method, as various methods are known in the art to determine the thermal stability of enzymes.

Samples are assayed using standard succinyl-ala-ala-pro-phe-para-nitro anilide ("SAAPFpNA") assay (*See e.g.*, Delmar, Anal. Biochem., 94:316-320 [1979]; and Achtstetter, Arch. Biochem. Biophys., 207:445-54 [1981]), at pH 6.5, and at 25 °C. The samples are diluted to about 300 milliOD/minute. The thermal stability is typically expressed as enzyme half-life (min) as determined by:

$$H.L. = \ln 2 / slope,$$

wherein the slope is the slope of curve of rate v. time for each temperature.

By using these means, the stability of mutant variants can be readily compared relative the control mutant enzymes and/or wild-type enzyme.

## EXAMPLE 9

### Thermal Stability of Subtilisin Carlsberg Variants in N-tris(Hydroxymethyl)methyl-2-Aminoethanesulfonic acid ("TES")

In these experiments, the thermal stability of the variants in TES is determined. As described above, 5.0 ppm enzyme (*e.g.*, the mutants of interest and the controls) are tested at five timepoints (*e.g.*, 5, 10, 20, 40, and 60 minutes) at pH 6.5, for each temperature. For example, the samples are tested at two degree intervals ranging from 42 - 56° C, and at every other degree at temperatures ranging from 42-56°C, in the PCR thermocycler gradient. A TES buffer is prepared by mixing 50mM TES (Sigma T 1375), 0.005% Tween 80®. Typically, the pH is adjusted to 6.5.

Thermal stability of the variants can be determined as activity of the residual variant as measured using a succinyl-ala-ala-pro-phe-para-nitroanilide ("AAPFpNA") as known in the art, using reagents such as Sigma no. S-7388 (mol. wt. 624.6 g/mole) (*See e.g.*, Delmar et al., Anal. Biochem., 94:316-320 [1979]; and Achtstetter, Arch.Biochem.Biophys., 207:445-454 [1981]), tested at pH 6.5, and at a temperature of 25 °C. The (yellow) p-nitronanilide (pNA) formed in the reaction is measured spectrophotometrically at 410 nm: .$\varepsilon_M$ =8,480 $M_{-1}$. cm $_{-1}$, () with a SpectraMax 250 spectrophotometer, the samples being diluted to about 300mOD/min. The thermal stability is expressed as enzyme half-life (min) as described above. As indicated above, these experiments provide means to compare the stability of the variant enzyme preparations with the control mutant enzyme(s) and/or wild-type enzyme.

## EXAMPLE 10

### Stability of Subtilisin Carlsberg Variants in Bodywash Solutions and Other Personal Care Products

The stability of various subtilisin Carlsberg variants is measured using the following protocol.

### Method to Measure Solution Stability

In these experiments, subtilisin Carlsberg and mutant variants are tested in at least two studies, with the first study involving testing for 30 minutes at 45° C, and the second involving testing for 30 minutes at 50° C. For these tests, 50/50 (w/w) bodywash solutions are prepared by mixing a commercially available bodywash (*e.g.*, the bodywash sold under the trademark ZEST ®, from Procter & Gamble), with deionized water. The pH of the buffer blend is approximately 6.8.

The enzymes to be tested are diluted such that their final enzyme concentration in a 50 w/w % BodyWash: deionized water solution produces a change in $OD_{405}$ of 0.5 to 1.0 when 10μl of the enzyme/body wash solution is assayed using SAAPFpNA assay endpoint method. Once the amount of dilution is ascertained, 200 μl of the diluted mixture is placed into 96 well microtiter plate wells. The plate are sealed and placed in a water bath at 40° C, for one study, and at 50° C, for the second study. The plates are removed from the water bath after the desired length of time (*e.g.*, 30 or 45 minutes) and 10 μl samples assayed by the endpoint method. The percent of activity remaining is calculated as 100 times the final activity divided by the initial activity.

In some experiments, the variants including the specific residues determined by the assay of the earlier described example show an increased amount of enzymatic activity remaining and thus have a broader thermal stability than the controls. For example, at 50° C, some variant compounds have a greater percentage activity remaining whereas the control mutant enzyme and/or the wild-type enzyme without the stabilizing residue variants have a lower percentage of activity remaining. In some experiments, all enzymes have enhanced stability in the presence of bodywash at 50°, but control mutant enzyme-[epitopic variants] with different stability variants have even better stability.

Indeed, there are numerous applications in which the variant subtilisin Carlsberg enzymes of the present invention that have reduced immunogenicity find use. In addition to detergents and other cleaning preparations, the variant subtilisin Carlsberg enzymes having reduced immunogenicity also find use in personal care products. The following tables provide the compositions of various products suitable for use in testing. In these tables, the term "minors" encompasses pH modifiers, preservatives, viscosity modifiers, and perfumes. In these tables, the amounts represent approximate weight percent (as provided by the manufacturer), unless otherwise indicated, and are not intended to indicate significant digits.

| MOISTURISING BODYWASH | pH = 7 |
|---|---|
| RAW MATERIAL | Amount |
| Deionized Water | QS |
| Glycerin | 4.0 |
| PEG-6 Caprylic/Capric Glycerides | 4.0 |
| Palm Kernal Fatty acids | 3.0 |
| Sodium Laureth-3 Sulphate | 45.0 |
| Cocamide MEA | 3.0 |
| Sodium Lauroamphoacetate | 25.0 |
| Soybean Oil | 10.0 |
| Polyquaternium-10 (JR30M) | 0.70 |
| Protease | 1000ppm |

| BODYWASH | pH 6.5 | pH 7 | pH 8.5 |
|---|---|---|---|
| RAW MATERIAL | Amount | Amount | Amount |
| Deionized water | QS | QS | QS |
| Sodium Laureth Sulphate | 12 | 15 | 8 |
| Cocamidopropyl Betaine | 8 | 10 | 15 |
| APG Glucoside (Plantacare 2000[1]) | 0 | 2 | 1 |
| Polyquaternium-10 (JR30M) | 0.25 | 0 | 0 |
| Polyquaternium-7 (Mackam 55). | 0 | 0 | 0.7 |
| Protease | 250ppm | 500ppm | 1000ppm |
| 1 - Cognis | | | |

| BODY LOTION | pH 7 | pH 7 | pH 7.5 | pH 7 |
|---|---|---|---|---|
| RAW MATERIAL | Amount | Amount | Amount | Amount |
| DEIONISED WATER | QS | QS | QS | QS |
| GLYCERINE | 8 | 8 | 10 | 12 |
| ISOHEXADECANE | 3 | 3 | 3 | 6 |
| NIACINAMIDE | 0 | 3 | 5 | 6 |
| ISOPROPYL ISOSTEARATE | 3 | 3 | 3 | 3 |
| Polyacrylamide, Isoparaffin, Laureth-7 (Sepigel 305[2]) | 3 | 3 | 3 | 3 |
| PETROLATUM | 4 | 4 | 4 | 2 |

(continued)

| BODY LOTION | pH 7 | pH 7 | pH 7.5 | pH 7 |
|---|---|---|---|---|
| RAW MATERIAL | Amount | Amount | Amount | Amount |
| NYLON 12 | 2 | 2 | 2.5 | 2.5 |
| DIMETHICONE (DC1403[4]) | 2 | 2 | 2.5 | 2.5 |
| SUCROSE POLYCOTTONSEED OIL | 1.5 | 1.5 | 1.5 | 1.5 |
| Stearyl Alcohol 97% | 1 | 1 | 1 | 1 |
| D PANTHENOL | 1 | 1 | 1 | 1 |
| DL-alphaTOCOPHEROL ACETATE | 1 | 1 | 1 | 1 |
| Cetyl Alcohol 95% | 0.5 | 0.5 | 0.5 | 1 |
| BEHYNYL ALCOHOL | 1 | 1 | 1 | 0.5 |
| EMULGADE PL 68/50 | 0.4 | 0.4 | 0.5 | 0.5 |
| STEARIC ACID | 0.15 | 0.15 | 0.15 | 0.15 |
| Peg-100-stearate (MYRJ 59[1]) | 0.15 | 0.15 | 0.15 | 0.15 |
| Protease | 50ppm | 50ppm | 250ppm | 1000ppm |

1 - Uniqema
2 - Seppic
4 - Dow Corning

| ULTRA-HIGH MOISTURISING FACIAL CREAM/LOTION | pH 7 | pH 7 |
|---|---|---|
| RAW MATERIAL | Amount | Amount |
| Deionized water | QS | QS |
| Glycerin | 12 | 5 |
| PEG 400[6] | 0 | 10 |
| Niacinamide | 5 | 7 |
| Isohexadecane | 5 | 5 |
| Dimethicone (DC1403[3]) | 3 | 2 |
| Polyacrylamide, Isoparaffin, Laureth-7 (Sepigel 305[1]) | 3 | 3 |
| Isopropyl Isostearate | 2 | 2 |
| Polymethylsilsesquioxane | 2 | 2 |
| Cetyl Alcohol 95% | 1 | 1 |
| Sucrose polycottonseed oil | 1 | 1 |
| D-Panthenol | 1 | 1 |
| Vitamin E (Tocopherol Acetate) | 1 | 1 |
| Stearyl Alcohol 95% | 0.5 | 0.5 |
| Cetearyl Glucoside | 0.5 | 0.5 |
| Titanium dioxide | 0.3 | 0.3 |
| Stearic Acid | 0.15 | 0.15 |
| PEG-100-Stearate (Myrj 59[4]) | 0.15 | 0.15 |
| Protease | 500ppm | 500ppm |

1 - Seppic
3 - Dow Corning
4 - Uniqema
5 - Scher Chemicals
6 - Dow Chemicals

| FACIAL MOISTURIZING CREAM | pH 7 | pH 7 | pH 7.5 |
|---|---|---|---|
| RAW MATERIAL | Amount | Amount | Amount |
| Deionized water | QS | QS | QS |
| Glycerin | 3 | 5 | 10 |
| Petrolatum | 3 | 3 | 0 |
| Cetyl Alcohol 95% | 1.5 | 1.5 | 1 |
| Dimethicone Copolyol (DC 3225C [4]) | 2 | 2 | 2 |
| Isopropyl Palmitate | 1 | 1 | 0.5 |
| Carbomer 954[2] | 0.7 | 0.7 | 0.7 |
| Dimethicone (DC 200/350cs[4]) | 1 | 1 | 1 |
| Stearyl Alcohol 97% | 0.5 | 0.5 | 1 |
| Stearic acid | 0.1 | 0.1 | 0.1 |
| Peg-100-stearate (MYRJ 59[1]) | 0.1 | 0.1 | 0.1 |
| Titanium Dioxide | 0.3 | 0.3 | 0.3 |
| Protease | 50ppm | 250ppm | 1000ppm |
| 1 - Uniqema<br>2 - BF Goodrich<br>4 - Dow Corning | | | |

**EXAMPLE 11**

**Cleaning Compositions**

In addition to the compositions described above, the present invention provides means to develop cleaning compositions having particular characteristics. Indeed, the present invention provides various cleaning compositions that comprise modified proteases (*e.g.*, subtilisin Carlsberg). In particularly preferred embodiments, an effective amount of one or more protease enzymes described above are included in compositions useful for cleaning a variety of surfaces in need of proteinaceous stain removal. Such cleaning compositions include detergent compositions for cleaning hard surfaces; detergent compositions for cleaning fabrics; dishwashing compositions; oral cleaning compositions; and denture cleaning compositions. It is intended that these compositions be provided in any form suitable for the particular intended use. Preferably, the cleaning compositions of the present invention comprise from about 0.0001 % to about 10% of one or more protease enzymes, more preferably from about 0.001 % to about 1 %, and more preferably still from about 0.001% to about 0.1 %. Several examples of various cleaning compositions wherein the protease enzymes find use are discussed in further detail below. All parts, percentages and ratios used herein are by weight unless otherwise specified.

**A. Cleaning Compositions for Hard Surfaces, Dishes, and Fabrics**

The protease enzymes (*e.g.*, the variant subtilisin Carlsberg enzymes) of the present invention find use in any detergent composition where high sudsing and/or good insoluble substrate removal are desired. Thus, the protease enzymes of the present invention find use with various conventional ingredients to provide fully-formulated hard-surface cleaners, dishwashing compositions, fabric laundering compositions and the like. These compositions are suitable for use in any form (*e.g.*, liquid, granules, bars, etc.) acceptable for the particular application. In addition, these compositions are also suitable for use in commercially available "concentrated" detergents which contain as much as 30%-60% by weight of surfactants.

In some embodiments, the cleaning compositions contain various anionic, nonionic, zwitterionic, etc., surfactants. Such surfactants are typically present at levels of from about 0.1 % to about 60%, preferably from about 1% to about 35%, of the compositions. Suitable surfactants include, but are not limited to the conventional $C_{11}$ -$C_{18}$ alkyl benzene sulfonates and primary and random alkyl sulfates, the $C_{10}$ -$C_{18}$ secondary (2,3) alkyl sulfates of the formulas $CH_3$ $(CH_2)$x$(CHOSO_3)$.sup.- M.sup.+)$CH_3$, and $CH_3$ $(CH_2)$y$(CHOSO_3$.sup.- M.sup.+) $CH_2$ $CH_3$, wherein x and (y+1) are integers of at least about 7, preferably at least about 9, and M is a water-solubilizing cation, especially sodium, the $C_{10}$ -$C_{18}$ alkyl alkoxy sulfates (especially EO 1-7 ethoxy sulfates), $C_{10}$ -$C_{18}$ alkyl alkoxy carboxylates (especially the EO 1-7 ethoxycarboxylates), the $C_{10}$ -$C_{18}$ alkyl polyglycosides, and their corresponding sulfated polyglycosides, $C_{12}$ -$C_{18}$ alpha-sulfonated fatty acid esters, $C_{12}$ -$C_{18}$ alkyl and alkyl phenol alkoxylates (especially ethoxylates and mixed ethoxy/

propoxy), $C_{12}$ -$C_{18}$ betaines and sulfobetaines ("sultaines"), $C_{10}$ -$C_{18}$ amine oxides, $C_8$ -$C_{24}$ sarcosinates (especially oleoyl sarcosinate), and the like. The alkyl alkoxy sulfates (AES) and alkyl alkoxy carboxylates (AEC) are preferred herein. Furthermore, use of such surfactants in combination with the aforesaid amine oxide and/or betaine or sultaine surfactants is also preferred, depending on the desires of the formulator. Other conventional useful surfactants are known to those in the art, including, but not limited to the particularly useful surfactants such as the $C_{10}$- $C_{18}$ N-methyl glucamides (*See*, US Pat. No. 5, 194,639).

In some embodiments, the compositions of the present invention comprise member(s) of the class of nonionic surfactants which are condensates of ethylene oxide with a hydrophobic moiety to provide a surfactant having an average hydrophilic-lipophilic balance (HLB) in the range from 5 to 17, preferably from 6 to 14, more preferably from 7 to 12. The hydrophobic (lipophilic) moiety may be aliphatic or aromatic in nature and the length of the polyoxyethylene group which is condensed with any particular hydrophobic group can be readily adjusted to yield a water-soluble compound having the desired degree of balance between hydrophilic and hydrophobic elements. Especially preferred are the $C_9$ -$C_{15}$ primary alcohol ethoxylates (or mixed ethoxy/propoxy) containing 3-8 moles of ethylene oxide per mole of alcohol, particularly the $C_{14}$ -$C_{15}$ primary alcohols containing 6-8 moles of ethylene oxide per mole of alcohol, the $C_{12}$ -$C_{15}$ primary alcohols containing 35 moles of ethylene oxide per mole of alcohol, and mixtures thereof.

A wide variety of other ingredients useful in detergent cleaning compositions find use in the compositions herein, including other active ingredients, carriers, hydrotropes, processing aids, dyes or pigments, solvents for liquid formulations, etc. For an additional increment of sudsing, suds boosters such as the $C_{10}$ -$C_{16}$ alkolamides can be incorporated into the compositions, typically at about 1% to about 10% levels. The $C_{10}$ -$C_{14}$ monoethanol and diethanol amides illustrate a typical class of such suds boosters. Use of such suds boosters with high sudsing adjunct surfactants such as the amine oxides, betaines and sultaines noted above is also advantageous. If desired, soluble magnesium salts such as $MgCl_2$, $MgSO_4$, and the like, can be added at levels of, typically, from about 0.1% to about 2%, to provide additional sudsing.

The liquid detergent compositions herein typically contain water and other solvents as carriers. Low molecular weight primary or secondary alcohols (*e.g.*, methanol, ethanol, propanol, and isopropanol) are suitable. Monohydric alcohols are preferred for solubilizing surfactants, but polyols such as those containing from about 2 to about 6 carbon atoms and from about 2 to about 6 hydroxy groups (*e.g.*, 1,3-propanediol, ethylene glycol, glycerine, and 1,2-propanediol) also find use in the detergents of the present invention. In some embodiments, the compositions contain about 90%, or from about 10% to about 50% of such carriers.

The detergent compositions herein are preferably formulated such that during use in aqueous cleaning operations, the wash water has a pH between about 6.8 and about 11.0. Thus, finished products are typically formulated at this range. Techniques for controlling pH at recommended usage levels include the use of buffers, alkalis, acids, etc., and are well known to those skilled in the art.

When formulating the hard surface cleaning compositions and fabric cleaning compositions of the present invention, the formulator may wish to employ various builders at levels from about 5% to about 50% by weight. Typical builders include the 1-10 micron zeolites, polycarboxylates such as citrate and oxydisuccinates, layered silicates, phosphates, and the like. Other conventional builders are known to those in the art and are suitable for inclusion in the compositions of the present invention.

Likewise, the formulator may wish to employ various additional enzymes, such as cellulases, lipases, amylases, peroxidases, and proteases in such compositions, typically at levels of from about 0.001 % to about 1% by weight. Various detersive and fabric care enzymes are well-known in the laundry detergent art and are suitable for inclusion in the compositions of the present invention.

Various bleaching compounds, such as the percarbonates, perborates and the like, also find use in the compositions of the present invention. These bleaching compounds are typically present at levels from about 1% to about 15% by weight. If desired, such compositions can also contain bleach activators such as tetraacetyl ethylenediamine, nonanoyloxybenzene sulfonate, and the like, which are also known in the art. Usage levels of such compounds typically range from about 1% to about 10% by weight.

Various soil release agents, especially of the anionic oligoester type, various chelating agents, especially the aminophosphonates and ethylenediaminedisuccinates, various clay soil removal agents, especially ethoxylated tetraethylene pentamine, various dispersing agents, especially polyacrylates and polyasparatates, various brighteners, especially anionic brighteners, various dye transfer inhibiting agents, such as polyvinyl pyrrolidone, various suds suppressors, especially silicones and secondary alcohols, various fabric softeners, especially smectite clays and clay floculating polymers (*e.g.*, poly(oxy ethylene)), and the like all find use in the compositions of the present invention, most typically at levels ranging from about 1% to about 35% by weight.

Enzyme stabilizers also find use in the cleaning compositions of the present invention. Such enzyme stabilizers include, but are not limited to propylene glycol (preferably from about 1% to about 10%), sodium formate (preferably from about 0.1% to about 1 %) and calcium formate (preferably from about 0.1% to about 1%).

**1. Hard Surface Cleaning Compositions**

In preferred embodiments, hard surface cleaning compositions of the present invention comprise an effective amount of one or more protease enzymes (*e.g.*, variant subtilisin Carlsberg enzymes), preferably from about 0.0001% to about 10%, more preferably from about 0.001 % to about 5%, more preferably still from about 0.001 % to about 1% by weight of active protease enzyme of the composition. In addition to comprising one or more protease enzymes, such hard surface cleaning compositions typically comprise a surfactant and a water-soluble sequestering builder. However, in certain specialized products such as spray window cleaners, the surfactants are sometimes not used since they may produce a filmy/streaky residue on the glass surface.

The surfactant component, when present, may comprise as little as 0.1 % of the compositions herein, but typically the compositions will contain from about 0.25% to about 10%, more preferably from about 1% to about 5% of surfactant.

Typically the compositions will contain from about 0.5% to about 50% of a detergency builder, preferably from about 1% to about 10%. Preferably, the pH should be in the range of about 8 to 12. Conventional pH adjustment agents such as sodium hydroxide, sodium carbonate or hydrochloric acid can be used if adjustment is necessary.

In some embodiments, at least one solvent is included in the compositions. Useful solvents include, but are not limited to, glycol ethers such as diethyleneglycol monohexyl ether, diethyleneglycol monobutyl ether, ethyleneglycol monobutyl ether, ethyleneglycol monohexyl ether, propyleneglycol monobutyl ether, dipropyleneglycol monobutyl ether, and diols such as 2,2,4-trimethyl-1,3pentanediol and 2-ethyl-1,3-hexanediol. When used, such solvents are typically present at levels of from about 0.5% to about 15%, preferably from about 3% to about 11 %.

Additionally, highly volatile solvents such as isopropanol or ethanol find use in the present compositions, in order to facilitate faster evaporation of the composition from surfaces when the surface is not rinsed after "full strength" application of the composition to the surface. When used, volatile solvents are typically present at levels of from about 2% to about 12% in the compositions.

The hard surface cleaning composition embodiment of the present invention is illustrated by the following nonlimiting examples. In the following examples, reference to "Protease #" in the examples is to the variant useful in the present invention compositions having a reduced immunogenic responding protease variant of percentages of 0.10, 0.20, 0.10, 0.05, 0.03, and 0.02.

| Liquid Hard Surface Cleaning Compositions | | | | | | |
|---|---|---|---|---|---|---|
| Component | Example No. | | | | | |
| | 1 | 2 | 3 | 4 | 5 | 6 |
| EDTA** | | | 2.90 | 2.90 | | |
| Na Citrate | | | | | 2.90 | 2.90 |
| $NaC_{12}$ Alkylbenzene | 1.95 | | 1.95 | | 1.95 | |
| $NaC_{12}$ Alkylsulfate | | 2.20 | | 2.20 | | 2.20 |
| $NaC_{12}$ (ethoxy)*** | | 2.20 | | 2.20 | | 2.20 |
| $C_{12}$ Dimethylamine | | 0.50 | | 0.50 | | 0.50 |
| Na Cumene sulfonate | 1.30 | | 1.30 | | 1.30 | |
| Hexyl Carbitol*** | 6.30 | 6.30 | 6.30 | 6.30 | 6.30 | 6.30 |
| Water**** | Balance to 100% | | | | | |
| ** $Na_4$ Ethylenediamine diacetic acid<br>*** Diethyleneglycol monohexyl ether<br>**** All formulae adjusted to pH 7. | | | | | | |

In some embodiments of the above examples, additional proteases useful in the present invention (*e.g.*, variant subtilisin Carlsberg enzymes) are substituted with substantially similar results. In addition, in some embodiments of the above examples, any combination of the reduced immunogenic proteases useful in the present invention (*e.g.*, variant subtilisin Carlsberg enzymes) is substituted in the above formulations with substantially similar results.

The following Table provides sample compositions suitable for cleaning hard surfaces and removing mildew. The product compositions are typically at approximately pH 7.

| Spray Compositions for Cleaning Hard Surfaces and Removing Household Mildew | | | | | | |
|---|---|---|---|---|---|---|
| Component | Example No. | | | | | |
| | 7 | 8 | 9 | 10 | 11 | 12 |
| Protease # | 0.20 | 0.05 | 0.10 | 0.30 | 0.20 | 0.30 |
| Protease #+14 | | | | | 0.30 | 0.10 |
| Sodium octyl sulfate | 2.00 | 2.00 | 2.00 | 2.00 | 2.00 | 2.00 |
| Sodium dodecyl sulfate | 4.00 | 4.00 | 4.00 | 4.00 | 4.00 | 4.00 |
| NaOH | 0.80 | 0.80 | 0.80 | 0.80 | 0.80 | 0.80 |
| Silicate (Na) | 0.04 | 0.04 | 0.04 | 0.04 | 0.04 | 0.04 |
| Perfume | 0.35 | 0.35 | 0.35 | 0.35 | 0.35 | 0.35 |
| Water | Balance to 100% | | | | | |

In these Examples, any combination of the protease enzymes useful in the present invention (*e.g.*, variant subtilisin Carlsberg enzymes) is substituted in with substantially similar results.

**2. Dishwashing Compositions**

In additional embodiments of the present invention, dishwashing compositions comprising one or more protease enzymes (*e.g.*, mutant subtilisin Carlsberg enzymes) are provided. Preferred dishwashing composition embodiments of the present invention are illustrated below. Proteases are included with percentages at 0.5, 0.4, 0.1, 0.05, 0.03, and 0.02. In these compositions, the product pH is adjusted to 7.

| Dishwashing Compositions | | | | | | |
|---|---|---|---|---|---|---|
| Component | Example No. | | | | | |
| | 13 | 14 | 15 | 16 | 17 | 18 |
| $C_{12}$ -$C_{14}$ N-methyl- | 0.90 | 0.90 | 0.90 | 0.90 | 0.90 | 0.90 |
| $C_{12}$ ethoxy (1) sulfate | 12.0 | 12.0 | 12.0 | 12.0 | 12.0 | 12.0 |
| 2-methyl undecanoic acid | 4.50 | 4.50 | | 4.50 | 4.50 | |
| $C_{12}$ ethoxy (2) carboxylate | 4.50 | 4.50 | 4.50 | 4.50 | 4.50 | 4.50 |
| $C_{12}$ alcohol ethoxylate (4) | 3.00 | 3.00 | 3.00 | 3.00 | 3.00 | 3.00 |
| $C_{12}$ amine oxide | 3.00 | 3.00 | 3.00 | 3.00 | 3.00 | 3.00 |
| Sodium cumene sulfonate | 2.00 | 2.00 | 2.00 | 2.00 | 2.00 | 2.00 |
| Ethanol | 4.00 | 4.00 | 4.00 | 4.00 | 4.00 | 4.00 |
| Mg $Supp^{++}$ ($MgCl_2$) | 0.20 | 0.20 | 0.20 | 0.20 | 0.20 | 0.20 |
| Ca $Supp^{++}$ ($CaCl_2$) | 0.40 | 0.40 | 0.40 | 0.40 | 0.40 | 0.40 |
| Water | Balance to 100% | | | | | |

In some embodiments of the immediately above examples the proteases useful in the present invention (*e.g.*, variant subtilisin Carlsberg enzymes) are substituted in the above formulations, with substantially similar results. Furthermore, in some embodiments of the immediately above examples, any combination of the protease enzymes useful in the present invention (*e.g.*, variant subtilisin Carlsberg enzymes), among others, is substituted in the above formulations with substantially similar results.

| Granular Automatic Dishwashing Compositions | | | |
|---|---|---|---|
| Component | Example | | |
| | A | B | C |
| Citric acid | 15.0 | | |
| Citrate | 4.0 | 29.0 | 15.0 |
| Acrylate/methacrylate | 6.0 | | 6.0 |
| copolymer | | | |
| Acrylic acid maleic acid | | 3.7 | |
| copolymer | | | |
| Dry add carbonate | 9.0 | | 20.0 |
| Alkali metal silicate | 8.5 | 17.0 | 9.0 |
| Paraffin | | 0.5 | |
| Benzotriazole | | 0.3 | |
| Termamyl 60T | 1.5 | 1.5 | 1.0 |
| Protease #4 (4.6% prill) | 1.6 | 1.6 | 1.6 |
| Percarbonate (AvO) | 1.5 | | |
| Perborate monohydrate | | 0.3 | 1.5 |
| Perborate tetrahydrate | | 0.9 | |
| Tetraacetylethylene diamine | 3.8 | 4.4 | |
| Diethylene triamine penta methyphosphonic acid (Mg salt) | 0.13 | 0.13 | 0.13 |
| Alkyl ethoxy sulphate--3x ethoxylated | 3.0 | | |
| Alkyl ethoxy propoxy nonionic | | | |
| surfactant | | | |
| Suds suppressor | 2.0 | | |
| Olin SLF18 nonionic surfactant | | | |
| Sulfate | | | |

In the immediately above formulations a reduced immunogenic protease (*e.g.*, a variant subtilisin Carlsberg enzyme) useful in the present invention is substituted therein with substantially similar results. Also in the immediately above formulations, any combination of the proteases useful in the present invention (*e.g.*, variant subtilisin Carlsberg enzymes) recited herein can be substituted in with substantially similar results.

### 3. Fabric Cleaning Compositions

The present invention further provides fabric cleaning compositions comprising one or more protease enzymes (*e.g.*, variant subtilisin Carlsberg enzymes).

#### a. Granular Fabric Cleaning

The granular fabric cleaning compositions of the present invention contain an effective amount of one or more protease enzymes (*e.g.*, variant subtilisin Carlsberg enzymes), preferably from about 0.001 % to about 10%, more preferably from about 0.005% to about 5%, more preferably from about 0.01% to about 1% by weight of active protease enzyme of the composition. In addition to one or more protease enzymes, the granular fabric cleaning compositions typically comprise at least one surfactant, one or more builders, and, in some cases, a bleaching agent. Granular fabric cleaning composition embodiments of the present invention are illustrated by the following examples.

| Granular Fabric Cleaning Compositions | | | | |
|---|---|---|---|---|
| Component | Example No. | | | |
| | 20 | 21 | 22 | 23 |
| Protease (4% Prill) | 0.10 | 0.20 | 0.03 | 0.05 |
| Protease (4% Prill) | | | 0.02 | 0.05 |
| $C_{13}$ linear alkyl benzene sulfonate | 22.0 | 22.0 | 22.0 | 22.0 |
| Phosphate (as sodium tripolyphosphates) | 23.0 | 23.0 | 23.0 | 23.0 |
| Sodium carbonate | 23.0 | 23.0 | 23.0 | 23.0 |
| Sodium silicate | 12.0 | 14.0 | 14.0 | 14.0 |
| Zeolite | 8.20 | 8.20 | 8.20 | 8.20 |
| Chelant (diethylaenetriamine-pentaacetic acid) | 0.40 | 0.40 | 0.40 | 0.40 |
| Sodium sulfate | 5.50 | 5.50 | 5.50 | 5.50 |
| Water | Balance to 100% | | | |

In the immediately above formulations a reduced immunogenic protease useful in the present invention (*e.g.*, variant subtilisin Carlsberg enzymes) is substituted therein with substantially similar results. Also in the immediately above formulations, any combination of the proteases (*e.g.*, variant subtilisin Carlsberg enzymes) useful in the present invention recited herein can be substituted in with substantially similar results.

| Granular Fabric Cleaning Composition | | | | |
|---|---|---|---|---|
| Component | Example No. | | | |
| | 24 | 25 | 26 | 27 |
| Protease #(4% Prill) | 0.10 | 0.20 | 0.03 | 0.05 |
| Protease # +1 (4% Prill) | | | 0.02 | 0.05 |
| $C_{12}$ alkyl benzene sulfonate | 12.0 | 12.0 | 12.0 | 12.0 |
| Zeolite A (1-10 pm) | 26.0 | 26.0 | 26.0 | 26.0 |
| 2-butyl octanoic acid | 4.0 | 4.0 | 4.0 | 4.0 |
| $C_{12}$ -$C_{14}$ secondary (2,3) | 5.0 | 5.0 | 5.0 | 5.0 |
| Sodium citrate | 5.0 | 5.0 | 5.0 | 5.0 |
| Optical brightener | 0.10 | 0.10 | 0.10 | 0.10 |
| Sodium sulfate | 17.0 | 17.0 | 17.0 | 17.0 |
| Fillers, water, minors | Balance to 100% | | | |

In the immediately above formulations a reduced immunogenic protease (*e.g.*, variant subtilisin Carlsberg) useful in the present invention is substituted therein with substantially similar results. Also in the immediately above formulations, any combination of the proteases useful in the present invention (*e.g.*, variant subtilisin Carlsberg enzymes) can be substituted in with substantially similar results.

| Granular Fabric Cleaning Compositions | | |
|---|---|---|
| Component | Example No. | |
| | 28 | 29 |
| Linear alkyl benzene sulphonate | 11.4 | 10.7 |

(continued)

| Granular Fabric Cleaning Compositions | | |
|---|---|---|
| Component | Example No. | |
| | 28 | 29 |
| Tallow alkyl sulphate | 1.8 | 2.4 |
| $C_{14-15}$ alkyl sulphate | 3.0 | 3.10 |
| $C_{14-15}$ alcohol 7 times ethoxylated | 4.0 | 4.0 |
| Tallow alcohol 11 times ethoxylated | 1.8 | 1.8 |
| Dispersant | 0.07 | 0.1 |
| Silicone fluid | 0.80 | 0.80 |
| Trisodium citrate | 14.0 | 15.0 |
| Citric acid | 3.0 | 2.5 |
| Zeolite | 32.5 | 32.1 |
| Maleic acid acrylic acid copolymer | 5.0 | 5.9 |
| Diethylene triamine penta methylene | 1.0 | 0.20 |
| Protease # (4% Prill) | 0.30 | 0.30 |
| Lipase | 0.36 | 0.40 |
| Amylase | 0.30 | 0.30 |
| Sodium silicate | 2.0 | 2.5 |
| Sodium sulphate | 3.5 | 5.2 |
| Polyvinyl pyrrolidone | 0.3 | 0.5 |
| Perborate | 0.5 | 1 |
| Phenol sulphonate | 0.1 | 0.2 |
| Peroxidase | 0.1 | 0.1 |
| Minors | Up to 100 | |

| Granular Fabric Cleaning Compositions | | |
|---|---|---|
| Component | Example No. | |
| | 30 | 31 |
| Sodium linear $C_{12}$ alkyl benzene sulphonate | 6.5 | 8.0 |
| Sodium sulphate | 15.0 | 18.0 |
| Zeolite | 26.0 | 22.0 |
| Sodium nitrilotriacetate | 5.0 | 5.0 |
| Polyvinyl pyrrolidone | 0.5 | 0.7 |
| Tetraacetylethylene diamine | 3.0 | 3.0 |
| Boric acid | 4.0 | |
| Perborate | 0.5 | 1 |
| Phenol sulphonate | 0.1 | 0.2 |
| Protease #4 (4% Prill) | 0.4 | 0.4 |

(continued)

| Granular Fabric Cleaning Compositions | | |
|---|---|---|
| Component | Example No. | |
| | 30 | 31 |
| Fillers (*e.g.*, silicates, carbonates, perfumes) | Up to 100 | |

In additional embodiments, compact granular fabric cleaning compositions such as the following are provided. The ingredients are provided in terms of the weight percent. Composition 1: alkyl sulphate (8.0), alkyl ethoxy sulphate (2.0), a mixture of C25 and C45 alcohol 3 and 7 times ethoxylated (6.0), polyhydroxy fatty acid amide (2.5), Zeolite (17.0), layered silicate/citrate (16.0), carbonate (7.0), maleic acid acrylic acid copolymer (5.0), soil release polymer (0.4), carboxymethyl cellulose (0.4), poly(4-vinylpyridine)-N-oxide (0.1), copolymer of vinylimidazole and vinylpyrrolidone (0.1), PEG-2000 (0.2), protease # (4% Prill) (0.5), lipase (0.2), cellulase (0.2), tetracetylethylene diamine (6.0), percarbonate (22.0), ethylene diamine disuccinic acid (0.3), suds suppressor (3.5), disodium-4,4'-bis(2-morpholino-4-anilino-s-triazin-6-ylamino)stilbene-2,2'-disulphonate (0.25), Disodium-4,4'-bis(2-sulfostyril)biphenyl (0.05), and a combination of water, perfume and minors (up to 100).

In an alternative granular fabric cleaning composition, the following ingredients are provided. The ingredients are provided in terms of the weight percent. Composition 2: linear alkyl benzene sulphonate (7.6), $C_{16}$ -$C_{18}$ alkyl sulfate (1.3), $C_{14-15}$ alcohol 7 times ethoxyiated (4.0), coco-alkyl-dimethyl hydroxyethyl ammonium chloride (1.4), dispersant (0.07), silicone fluid (0.8), trisodium citrate (5.0), Zeolite 4A (15.0), maleic acid acrylic acid copolymer (4.0), diethylene triamine penta methylene phosphonic acid (0.4), perborate (15.0), tetraacetylethylene diamine (5.0), smectite clay (10.0), poly (oxy ethylene) (MW 300,000) (0.3), protease # (4% Prill) (0.4), lipase (0.2), amylase (0.3), cellulase (0.2), sodium silicate (3.0), sodium carbonate (10.0), carboxymethyl cellulose (0.2), brighteners (0.2), and a mixture of water, perfume and minors (up to 100).

In yet another alternative granular fabric cleaning composition, the following ingredients are provided. The ingredients are provided in terms of the weight percent. Composition 2: linear alkyl benzene (6.92), tallow alkyl sulfate (2.05), $C_{14-15}$ alcohol 7 times ethoxylated (4.4), $C_{12-15}$ alkyl ethoxy sulfate - 3 times ethoxylated (0.16), Zeolite (20.2), citrate (5.5), carbonate (15.4), silicate (3.0), maleic acid acrylic acid copolymer (4.0), carboxymethyl cellulase (0.31), soil release polymer (0.30), protease # (4% Prill) (0.2), lipase (0.36), cellulase (0.13), perborate tetrahydrate (11.64), perborate monohydrate (8.7), tetraacetylethylene diamine (5.0), diethylene tramine penta methyl phosphonic acid (0.38), magnesium sulfate (0.40), brightener (0.19), a mixture of perfume, silicone, and suds suppressors (0.85), and minors (up to 100).

In the immediately above formulations a reduced immunogenic protease (*e.g.*, a variant subtilisin Carlsberg enzyme) useful in the present invention is substituted therein with substantially similar results. Also in the immediately above formulations, any combination of the proteases useful in the present invention (*e.g.*, variant subtilisin Carlsberg enzymes) can be substituted in with substantially similar results.

### b. Liquid Fabric Cleaning Compositions

Liquid fabric cleaning compositions of the present invention comprise an effective amount of one or more protease enzymes (*e.g.*, variant subtilisin Carlsberg enzymes), preferably from about 0.0001% to about 10%, more preferably from about 0.001% to about 1%, and most preferably from about 0.001 % to about 0.1 %, by weight of active protease enzyme of the composition. Such liquid fabric cleaning compositions typically additionally comprise an anionic surfactant, a fatty acid, a water-soluble detergency builder and water. The liquid fabric cleaning composition embodiment of the present invention is illustrated by the following examples.

| Liquid Fabric Cleaning Compositions | | | | | |
|---|---|---|---|---|---|
| Component | Example No. | | | | |
| | 35 | 36 | 37 | 38 | 39 |
| Protease # | 0.05 | 0.03 | 0.30 | 0.03 | 0.10 |
| Protease # +1 | | | | 0.01 | 0.20 |
| $C_{12}$ -$C_{14}$ alkyl sulfate, Na | 20.0 | 20.0 | 20.0 | 20.0 | 20.0 |
| 2-butyl octanoic acid | 5.0 | 5.0 | 5.0 | 5.0 | 5.0 |

(continued)

| Liquid Fabric Cleaning Compositions | | | | | |
|---|---|---|---|---|---|
| Component | Example No. | | | | |
| | 35 | 36 | 37 | 38 | 39 |
| Sodium citrate | 1.0 | 1.0 | 1.0 | 1.0 | 1.0 |
| $C_{10}$ alcohol ethoxylate (3) | 13.0 | 13.0 | 13.0 | 13.0 | 13.0 |
| Monethanolamine | 2.5 | 2.5 | 2.5 | 2.5 | 2.5 |
| Water/propylene glycol/ethanol | Balance to 100% (100:1:1) | | | | |

In the immediately above formulations a reduced immunogenic protease useful in the present invention (*e.g.*, a variant subtilisin Carlsberg enzyme) is substituted therein with substantially similar results. Also in the immediately above formulations, any combination of the proteases useful in the present invention (*e.g.*, variant subtilisin Carlsberg enzymes) can be substituted in with substantially similar results.

| Liquid Fabric Cleaning Compositions | | |
|---|---|---|
| Component | Example No. | |
| | 40 | 41 |
| $C_{12-14}$ alkyl succinic acid | 3.0 | 8.0 |
| Citric acid monohydrate | 10.0 | 15.0 |
| Sodium $C_{12-15}$ alkyl sulphate | 8.0 | 8.0 |
| Sodium sulfate of $C_{12-15}$ alcohol 2 times ethoxylated | | 3.0 |
| $C_{12-15}$ alcohol 7 times ethoxylated | | 8.0 |
| $C_{12-15}$ alcohol 5 times ethoxylated | 8.0 | |
| Diethylene triamine penta (methylene phosphonic acid) | 0.2 | |
| Oleic acid | 1.8 | |
| Ethanol | 4.0 | 4.0 |
| Propanediol | 2.0 | 2.0 |
| Protease # | 0.2 | 0.2 |
| Polyvinyl pyrrolidone | 1.0 | 2.0 |
| Suds suppressor | 0.15 | 0.15 |
| NaOH | up to pH 7.5 | |
| Perborate | 0.5 | 1.0 |
| Phenol sulphonate | 0.1 | 0.2 |
| Peroxidase | 0.4 | 0.1 |
| Water and minors | Up to 100 | |

In the immediately above formulations a reduced immunogenic protease useful in the present invention (*e.g.*, a variant subtilisin Carlsberg enzyme) is substituted therein with substantially similar results. Also in the immediately above formulations, any combination of the proteases useful in the present invention (*e.g.*, variant subtilisin Carlsberg enzymes) can be substituted in with substantially similar results.

**c. Bar Fabric Cleaning Compositions**

Bar fabric cleaning compositions of the present invention suitable for hand-washing soiled fabrics contain an

effective amount of one or more protease enzymes (*e.g.*, variant subtilisin Carlsberg enzymes), preferably from about 0.001% to about 10%, more preferably from about 0.01 % to about 1 % by weight of the composition. The bar fabric cleaning composition embodiments of the present invention is illustrated by the following examples.

| Bar Fabric Cleaning Composition | | | | |
|---|---|---|---|---|
| **Component** | **Example No.** | | | |
| | **42** | **43** | **44** | **45** |
| Protease # | 0.3 | | 0.1 | 0.02 |
| Protease # +1 | | | 0.4 | 0.03 |
| $C_{12}$-$C_{16}$ alkyl sulfate, Na | 20.0 | 20.0 | 20.0 | 20.0 |
| $C_{12}$ -$C_{14}$-N-methyl glucamide | 5.0 | 5.0 | 5.0 | 5.0 |
| $C_{11}$-$C_{13}$ alkyl benzene sulfonate, Na | 10.0 | 10.0 | 10.0 | 10.0 |
| Sodium carbonate | 25.0 | 25.0 | 25.0 | 25.0 |
| Sodium pyrophosphate | 7.0 | 7.0 | 7.0 | 7.0 |
| Sodium tripolyphosphate | 7.0 | 7.0 | 7.0 | 7.0 |
| Zeolite A (0.1-10 μm) | 5.0 | 5.0 | 5.0 | 5.0 |
| Carboxymethylcellulose | 0.2 | 0.2 | 0.2 | 0.2 |
| Polyacrylate (MW 1400) | 0.2 | 0.2 | 0.2 | 0.2 |
| Coconut monethanolamide | 5.0 | 5.0 | 5.0 | 5.0 |
| Brightener, perfume | 0.2 | 0.2 | 0.2 | 0.2 |
| $CaSO_4$ | 1.0 | 1.0 | 1.0 | 1.0 |
| $MgSO_4$ | 1.0 | 1.0 | 1.0 | 1.0 |
| Water | 4.0 | 4.0 | 4.0 | 4.0 |
| Fillers (*e.g.*, CaCO3, talc, clay, silicates, etc.) | Balance to 100% | | | |

In the immediately above formulations a reduced immunogenic protease useful in the present invention (*e.g.*, a variant subtilisin Carlsberg enzyme) is substituted therein with substantially similar results. Also in the immediately above formulations, any combination of the proteases useful in the present invention (*e.g.*, variant subtilisin Carlsberg enzymes) can be substituted in with substantially similar results.

**B. Additional Cleaning Compositions**

In addition to the hard surface cleaning, dishwashing and fabric cleaning compositions discussed above, one or more protease enzymes (*e.g.*, variant subtilisin Carlsberg enzymes) find use as components of various other cleaning compositions where hydrolysis of an insoluble substrate is desired. Such additional cleaning compositions include, but are not limited to oral cleaning compositions, denture cleaning compositions, and contact lens cleaning compositions, as well as other personal care cleaning compositions.

**1. Oral Cleaning Compositions**

In additional embodiments of the present invention, pharmaceutically-acceptable amounts of one or more protease enzymes (*e.g.*, variant subtilisin Carlsberg enzymes) are included in compositions useful for removing proteinaceous stains from teeth or dentures. Preferably, oral cleaning compositions of the present invention comprise from about 0.0001 % to about 20% of one or more protease enzymes, more preferably from about 0.001% to about 10%, more preferably still from about 0.01% to about 5%, by weight of the composition, and a pharmaceutically-acceptable carrier. Typically, the pharmaceutically-acceptable oral cleaning carrier components of the oral cleaning components of the oral cleaning compositions will generally comprise from about 50% to about 99.99%, preferably from about 65% to about 99.99%, more preferably from about 65% to about 99%, by weight of the composition.

The pharmaceutically-acceptable carrier components and optional components which may be included in the oral cleaning compositions of the present invention are well known to those skilled in the art. A wide variety of composition types, carrier components and optional components useful in the oral cleaning compositions are disclosed in US Pat. No. 5,096,700; US Pat. No. 5,028,414; and US Pat. No. 5,028,415. Oral cleaning composition embodiments of the present invention are illustrated by the following examples.

| Oral Dentrifice Cleaning Composition | | | | |
|---|---|---|---|---|
| **Component** | **Example No.** | | | |
| | **46** | **47** | **48** | **49** |
| Protease # | 2.0 | 3.5 | 1.5 | 2.0 |
| Sorbitol (70% aq. soln.) | 35.0 | 35.0 | 35.0 | 35.0 |
| PEG-6* | 1.0 | 1.0 | 1.0 | 1.0 |
| Silica dental abrasive** | 20.0 | 20.0 | 20.0 | 20.0 |
| Sodium fluoride | 0.243 | 0.243 | 0.243 | 0.243 |
| Titanium oxide | 0.5 | 0.5 | 0.5 | 0.5 |
| Sodium saccharin | 0.286 | 0.286 | 0.286 | 0.286 |
| Sodium alkyl sulfate (27.9%) | 4.0 | 4.0 | 4.0 | 4.0 |
| Flavor | 1.04 | 1.04 | 1.04 | 1.04 |
| Carboxyvinyl polymer*** | 0.30 | 0.30 | 0.30 | 0.30 |
| Carrageenan**** | 0.8 | 0.8 | 0.8 | 0.8 |
| Water | Balance to 100% | | | |

*PEG-6--Polyethylene glycol, having MW of 600
**Precipitated silica identified as Zeodent 119 (J.M. Huber).
***Carbopol (B.F. Goodrich Chemical Co.)
****Iota carrageenan (Hercules Chemical Co.).

In the immediately above formulations a reduced immunogenic protease useful in the present invention (*e.g.*, a variant subtilisin Carlsberg enzyme) is substituted therein with substantially similar results. Also in the immediately above formulations, any combination of the proteases useful in the present invention (*e.g.*, variant subtilisin Carlsberg enzymes) can be substituted in with substantially similar results.

| Mouthwash Compositions | | | | |
|---|---|---|---|---|
| **Component** | **Example No.** | | | |
| | **50** | **51** | **52** | **53** |
| Protease # | 3.0 | 7.5 | 1.0 | 5.0 |
| SDA 40 Alcohol | 8.0 | 8.0 | 8.0 | 8.0 |
| Flavor | 0.08 | 0.08 | 0.08 | 0.08 |
| Emulsifier | 0.08 | 0.08 | 0.08 | 0.08 |
| Sodium fluoride | 0.05 | 0.05 | 0.05 | 0.05 |
| Glycerin | 10.0 | 10.0 | 10.0 | 10.0 |
| Sweetener | 0.02 | 0.02 | 0.02 | 0.02 |
| Benzoic acid | 0.05 | 0.05 | 0.05 | 0.05 |
| NaOH | 0.20 | 0.20 | 0.20 | 0.20 |
| Dye | 0.04 | 0.04 | 0.04 | 0.04 |

(continued)

| Mouthwash Compositions | | | | |
|---|---|---|---|---|
| Component | Example No. | | | |
| | 50 | 51 | 52 | 53 |
| Water | Balance to 100% | | | |

In the immediately above formulations, a reduced immunogenic protease useful in the present invention (*e.g.*, a variant subtilisin Carlsberg enzyme) is substituted therein with substantially similar results. Also in the immediately above formulations, any combination of the proteases useful in the present invention (*e.g.*, variant subtilisin Carlsberg enzymes) can be substituted in with substantially similar results.

| Lozenge Compositions | | | | |
|---|---|---|---|---|
| Component | Example No. | | | |
| | 54 | 55 | 56 | 57 |
| Protease # | 0.01 | 0.03 | 0.10 | 0.02 |
| Sorbitol | 17.50 | 17.50 | 17.50 | 17.50 |
| Mannitol | 17.50 | 17.50 | 17.50 | 17.50 |
| Starch | 13.60 | 13.60 | 13.60 | 13.60 |
| Sweetener | 1.20 | 1.20 | 1.20 | 1.20 |
| Flavor | 11.7 | 11.7 | 11.7 | 11.7 |
| Color | 0.10 | 0.10 | 0.10 | 0.10 |
| Corn syrup | Balance to 100% | | | |

In the immediately above formulations a reduced immunogenic protease useful in the present invention (*e.g.*, a variant subtilisin Carlsberg enzyme) is substituted therein with substantially similar results. Also in the immediately above formulations, any combination of the proteases useful in the present invention (*e.g.*, variant subtilisin Carlsberg enzymes) can be substituted in with substantially similar results.

| Chewing Gum Compositions | | | | |
|---|---|---|---|---|
| Component | Example No. | | | |
| | 58 | 59 | 60 | 61 |
| Protease # | 0.03 | 0.02 | 0.10 | 0.05 |
| Sorbitol crystals | 38.44 | 38.4 | 38.4 | 38.4 |
| Paloja-T gum base* | 20.0 | 20.0 | 20.0 | 20.0 |
| Sorbitol (70% aq. soln.) | 22.0 | 22.0 | 22.0 | 22.0 |
| Mannitol | 10.0 | 10.0 | 10.0 | 10.0 |
| Glycerine | 7.56 | 7.56 | 7.56 | 7.56 |
| Flavor | 1.0 | 1.0 | 1.0 | 1.0 |
| Corn syrup | Balance to 100% | | | |
| *Supplied by L.A. Dreyfus Co. | | | | |

In the immediately above formulations a reduced immunogenic protease useful in the present invention (*e.g.*, a variant subtilisin Carlsberg enzyme) is substituted therein with substantially similar results, Also in the immediately above formulations, any combination of the proteases useful in the present invention (*e.g.*, variant subtilisin Carlsberg

enzymes) can be substituted in with substantially similar results.

**2. Denture Cleaning Compositions**

In yet additional embodiments, the present invention provides various denture cleaning compositions for cleaning dentures outside of the oral cavity comprise one or more protease enzymes (*e.g.*, variant subtilisin Carlsberg enzymes). Such denture cleaning compositions comprise an effective amount of one or more protease enzymes (*e.g.*, variant subtilisin Carlsberg enzymes), preferably from about 0.0001 % to about 50% of one or more protease enzymes, more preferably from about 0.001 % to about 35%, more preferably still from about 0.01% to about 20%, by weight of the composition, and a denture cleansing carrier. Various denture cleansing composition formats such as effervescent tablets and the like are well known in the art (*See e.g.*, US Patent No. 5,055,305), and are generally appropriate for incorporation of one or more protease enzymes for removing proteinaceous stains from dentures.

The denture cleaning composition embodiments of the present invention is illustrated by the following examples.

| **Two-Layer Effervescent Denture Cleansing Table Composition** | | | | |
|---|---|---|---|---|
| **Component** | **Example No.** | | | |
| | **62** | **63** | **64** | **65** |
| Acidic Layer: | | | | |
| Protease # | 1.0 | 1.5 | 0.01 | 0.05 |
| Tartaric acid | 24.0 | 24.0 | 24.0 | 24.0 |
| Sodium carbonate | 4.0 | 4.0 | 4.0 | 4.0 |
| Sulphamic acid | 10.0 | 10.0 | 10.0 | 10.0 |
| PEG 20,000 | 4.0 | 4.0 | 4.0 | 4.0 |
| Sodium bicarbonate | 24.5 | 24.5 | 24.5 | 24.5 |
| Potassium persulfate | 15.0 | 15.0 | 15.0 | 15.0 |
| Sodium acid pyrophosphate | 7.0 | 7.0 | 7.0 | 7.0 |
| Pyrogenic silica | 2.0 | 2.0 | 2.0 | 2.0 |
| Tetracetylethylene diamine | 7.0 | 7.0 | 7.0 | 7.0 |
| Ricinoleylsulfosuccinate | 0.5 | 0.5 | 0.5 | 0.5 |
| Flavor | 1.0 | 1.0 | 1.0 | 1.0 |
| | | | | |
| Alkaline Layer: | | | | |
| Sodium perborate monohydrate | 32.0 | 32.0 | 32.0 | 32.0 |
| Sodium bicarbonate | 19.0 | 19.0 | 19.0 | 19.0 |
| EDTA | 3.0 | 3.0 | 3.0 | 3.0 |
| Sodium tripolyphosphate | 12.0 | 12.0 | 12.0 | 12.0 |
| PEG 20,000 | 2.0 | 2.0 | 2.0 | 2.0 |
| Potassium persulfate | 26.0 | 26.0 | 26.0 | 26.0 |
| Sodium carbonate | 2.0 | 2.0 | 2.0 | 2.0 |
| Pyrogenic silica | 2.0 | 2.0 | 2.0 | 2.0 |
| Dye/flavor | 2.0 | 2.0 | 2.0 | 2.0 |

In the immediately above formulations a reduced immunogenic protease useful in the present invention (*e.g.*, a variant subtilisin Carlsberg enzyme) is substituted therein with substantially similar results. Also in the immediately above formulations, any combination of the proteases useful in the present invention (*e.g.*, variant subtilisin Carlsberg

enzymes) can be substituted in with substantially similar results.

### 3. Personal Cleansing Compositions

In additional embodiments of the present invention, personal cleaning compositions for cleaning the skin comprise one or more of the protease enzymes (*e.g.*, variant subtilisin Carlsberg enzymes). Such compositions typically comprise from about 0.001 % to about 5% protease enzyme (*e.g.*, variant subtilisin Carlsberg enzymes), preferably from about 0.005% to about 2%, and most preferably from about 0.01% to about 0.8% by weight of the composition. Preferred personal cleansing compositions into which can be included protease enzymes as described herein include, but are not limited to those described in US Patent Application Ser. Nos. 08/121,623 and 08/121,624. Although various compositions are contemplated by the present invention, one liquid personal cleaning composition containing a soap component includes (weight %): soap (K or Na) (15.00), 30% laurate, 30% myristate, 25% palmitate, 15% stearate, fatty acids (above ratios) (4.50), Na lauryl sarcosinate (6.00), sodium laureth-3 sulfate (0.66), cocamidopropylbetaine (1.33), glycerine (15.00), propylene glycol (9.00), polyquaternium 10 (0.80), ethylene glycol distearate (EDTA) (1.50), propylparaben (0.10), methylparaben (0.20), protease # (0.10), KOH or NaOH (if necessary to adjust pH), calcium sulfate (3), acetic acid (3), and water (balance to 100).

In another embodiment, personal cleansing bars are provided by the present invention. Although various compositions are contemplated by the present invention, one bar personal cleaning composition containing a soap component includes (weight sodium cocoyl isethionate (47.20), sodium cetearyl sulfate (9.14), paraffin (9.05), sodium soap (*in situ*) (3.67), sodium isethionate (5.51), sodium chloride (0.45), titanium dioxide (0.4), trisodium EDTA (0.1), trisodium etidronate (0.1), perfume (1.20), $Na_2SO_4$ (0.87), protease # (0.10), and a mixture of water and minors (balance to 100).

In the immediately above formulations a reduced immunogenic protease useful in the present invention (*e.g.*, a variant subtilisin Carlsberg enzyme) is substituted therein with substantially similar results. Also in the immediately above formulations, any combination of the proteases useful in the present invention (*e.g.*, variant subtilisin Carlsberg enzymes) can be substituted in with substantially similar results.

## EXAMPLE 12

### Wash Performance Test

The wash performance of the variants useful in the present invention compositions may be evaluated by any suitable means known in the art. One suitable method for measuring the removal of stain from EMPA 116(blood/milk/carbon black on cotton) cloth swatches (Testfabrics, Inc., Middlesex, N.J. 07030) is described in this Example.

Six EMPA 116 swatches, cut to 3.times.41/2 inches with pinked edges, are placed in each pot of a Model 7243S Terg-O-Tometer (United States Testing Co., Inc., Hoboken, N.J.) containing 1000 ml of water, 15 gpg hardness (Ca++:Mg++::3:1::w:w), 7 g of detergent, and enzyme as appropriate. The detergent base is WFK1 detergent from wfk-Testgewebe GmbH, (Krefeld, Germany) and has the following components (% of final formulation): Zeolite A (25%), sodium sulfate (25%), soda ash (10%), linear alkylbenzenesulfonate (8.8%), alcohol ethoxylate (7-8 EO) (4.5%), sodium soap (3%), and sodium silicate ($SiO_2$ :$Na_2$ O::3.3:1)(3%).

To this base detergent, the following additions are made (% of final formulation): sodium perborate monohydrate (13%), copolymer (Sokalan CP5) (4%), TAED (Mykon ATC Green) (3%), enzyme (0.5%), and whitener (Tinopal AMS-GX) (0.2%).

Sodium perborate monohydrate can be obtained from various commercial sources, including Degussa Corporation, Ridgefield-Park. Sokalan CP5 is obtained from BASF Corporation, Parsippany, N.J. Mykon ATC Green (TAED, tetraacetylethylenediamine) can be obtained from Warwick International, Limited, England. T inopal AMS GX can be obtained from Ciba-Geigy Corporation, Greensboro, N.C.

In one suitable testing method, six EMPA 116 swatches are washed in detergent with enzyme for 30 min at 60° C, rinsed twice for 5 minutes each time in 1000 ml water.

Enzymes are added at final concentrations of 0.05 to 1 ppm for standard curves, and 0.25 ppm for routine analyses. Swatches are dried and pressed, and the reflectance from the swatches is measured using the L value on the L*a*b* scale of a Minolta Chroma Meter, Model CR-200 (Minolta Corporation, Ramsey, NJ.). In some embodiments, the performance of the test enzyme is reported as a percentage of the performance of *B. amyloliquefaciens* (BPN') protease and is calculated by dividing the amount of *B. amyloliquefaciens* (BPN') protease by the amount of variant protease (*e.g.*, variant subtilisin Carlsberg enzyme) that is needed to provide the same stain removal performance.times. 100.

## EXAMPLE 13

### Variant Subtilisin Carlsberg Stability in a Liquid Detergent Formulation

This example provides a means for comparison of protease (*e.g.*, variant subtilisin Carlsberg enzyme) stability toward inactivation in a liquid detergent formulation with *Bacillus amyloliquefaciens* subtilisin and its variant enzymes. As other methods find use with the present invention, it is not intended that the present invention be limited to this method.

In this method, the detergent formulation for the study is a commercially available laundry detergent (*e.g.*, Tide® Ultra liquid laundry detergent (Proctor & Gamble)). In some embodiments, heat treatment of the detergent formulation is necessary to inactivate *in-situ* protease. This is accomplished by incubating the detergent at 96°C. for a period of 4.5 hours. Concentrated preparations of the *B. amyloliquefaciens* subtilisin and variant (*e.g.*, variant subtilisin Carlsberg enzymes) to be tested, in the range of 20 grams/liter enzyme, are then added to the heat-treated detergent, at room-temperature to a final concentration of 0.3 grams/liter enzyme in the detergent formulation. The heat-treated detergent with protease added is then incubated in a water bath at 50°C. Aliquots are removed from the incubation tubes at 0, 24, 46, 76, and 112 hour time intervals and assayed for enzyme activity by addition to a 1 cm cuvette containing 1.2 mM of the synthetic peptide substrate suc-Ala-Ala-Pro-phe-p-nitroanilide dissolved in 0.1 M Tris-HCL buffer, pH 8.6, and at 25°C. The initial linear reaction velocity is followed spectrophotometrically by monitoring the absorbance of the reaction product p-nitroaniline at 410 nm as a function of time. In preferred embodiments, the preferred variant(s) are observed to have significantly greater stability towards inactivation than the native *B. amyloliquefaciens* enzyme. Estimated half-lives for inactivation in the laundry detergent formulation for the two enzymes are determined under the specified test conditions.

Various modifications and variations of the described method and system of the invention will be apparent to those skilled in the art. Although the invention has been described in connection with specific preferred embodiments, it should be understood that the invention should not be unduly limited to such specific embodiments. Indeed, various modifications of the described modes for carrying out the invention which that are obvious to those skilled in molecular biology, immunology, formulations, and/or related fields are intended to be within the scope of the present invention.

### Claims

1. A method of reducing the immunogenicity of a subtilisin having at least 80% sequence identity to SEQ ID NO:1 by modifying at least one T-cell epitope of said subtilisin selected from the group consisting of SEQ ID NO:2, SEQ ID NO:90, SEQ ID NO:15, SEQ ID NO:30, and SEQ ID NO:40.

2. The method of claim 1, wherein the subtilisin has at least 90% sequence identity to SEQ ID NO:1.

3. The method of claim 1, wherein the subtilisin has the sequence of SEQ ID NO:1.

4. The method of any one of the preceding claims, comprising the steps of:

   (a) obtaining the gene encoding the subtilisin;
   (b) modifying the DNA sequence of the gene;
   (c) cloning the modified DNA sequence encoding a modified subtilisin into an expression vector;
   (d) transforming a host cell with the expression vector; and
   (e) expressing the modified subtilisin.

5. The method of any one of the preceding claims, wherein said subtilisin is modified by substitution of one or more amino acids in at least one of said epitopes.

6. The method of any one of the preceding claims, wherein said subtilisin is modified by deletion of one or more amino acids in at least one of said epitopes.

7. The method of any one of the preceding claims, wherein said subtilisin is modified by addition of an amino acid to at least one of said epitopes,

8. The method of any one of the preceding claims, wherein said modified subtilisin is expressed in an organism within the genus *Bacillus.*

9. A modified subtilisin having at least 80% identity to SEQ ID NO:1, wherein said subtilisin comprises at least one alteration in at least one epitope comprising an amino acid sequence selected from the group consisting of SEQ ID NO:2, SEQ ID NO:90, SEQ ID NO:15, and SEQ ID NO:30, whereby the immunogenic response produced by said modified subtilisin is less than said immunogenic response produced by wild-type subtilisin,

10. The modified subtilisin of claim 9, wherein the subtilisin has at least 90% sequence identity to SEQ ID NO:1.

11. The modified subtilisin of Claim 9, wherein at least one of said epitopes is modified by substitution of one or more amino acids.

12. The modified subtilisin of Claim 9,wherein at least one of said epitopes is modified by deletion of one or more amino acids.

13. The modified subtilisin of Claim 9, wherein at least one of said epitopes is modified by addition of an amino acid.

14. The modified subtilisin of Claim 9, wherein said modified subtilisin is expressed in an organism within the genus *Bacillus.*

15. An isolated nucleic acid encoding the modified subtilisin of Claim 9,.

16. An expression vector comprising the nucleic acid of Claim 16.

17. A host cell transformed with the expression vector of Claim 17.

18. A composition selected from the group consisting of cleaning compositions, personal care compositions, and healthcare compositions, comprising the modified subtilisin of Claim 9.

19. A peptide having a sequence consisting of SEQ ID NO:2, SEQ ID NO:90, SEQ ID NO:15, or SEQ ID NO:30.

**Patentansprüche**

1. Verfahren zur Verringerung der Immunogenität eines Subtilisins mit einer Sequenzidentität mit Seq.-ID Nr. 1 von zumindest 80 % durch Modifizierung zumindest eines T-Zellen-Epitops des Subtilisins, das aus der aus Seq.-ID Nr. 2, Seq.-ID Nr. 90, Seq.-ID Nr. 15, Seq.-ID Nr. 30 und Seq.-ID Nr. 40 bestehenden Gruppe ausgewählt wird.

2. Verfahren nach Anspruch 1, worin das Subtilisin eine Sequenzidentität mit Seq.-ID Nr. 1 von zumindest 90 % aufweist.

3. Verfahren nach Anspruch 1, worin das Subtilisin die Sequenz von Seq.-ID Nr. 1 aufweist.

4. Verfahren nach einem der vorangegangenen Ansprüche, umfassend die folgenden Schritte:

   (a) das Erhalten des Gens, das für das Subtilisin kodiert;
   (b) das Modifizieren der DNA-Sequenz des Gens;
   (c) das Klonieren der modifizierten DNA-Sequenz, die für ein modifiziertes Subtilisin kodiert, in einen Expressionsvektor;
   (d) das Transformieren einer Wirtszelle mit dem Expressionsvektor, und
   (e) das Exprimieren des modifizierten Subtilisins.

5. Verfahren nach einem der vorangegangenen Ansprüche, worin das Subtilisin durch Substitution einer oder mehrerer Aminosäuren in zumindest einem der Epitope modifiziert wird.

6. Verfahren nach einem der vorangegangenen Ansprüche, worin das Subtilisin durch Deletion einer oder mehrerer Aminosäuren in zumindest einem der Epitope modifiziert wird.

7. Verfahren nach einem der vorangegangenen Ansprüche, worin das Subtilisin durch Addition einer Aminosäure an zumindest eines der Epitope modifiziert wird.

8. Verfahren nach einem der vorangegangenen Ansprüche, worin das modifizierte Subtilisin in einem Organismus des Genus Bacillus exprimiert wird.

9. Modifiziertes Subtilisin mit einer Identität mit Seq.-ID Nr. 1 von zumindest 80 %, worin das Subtilisin zumindest eine Veränderung in zumindest einem Epitop umfasst, das eine Aminosäuresequenz umfasst, die aus der aus Seq.-ID Nr. 2, Seq.-ID Nr. 90, Seq.-ID Nr. 15 und Seq.-ID Nr. 30 bestehenden Gruppe ausgewählt ist, wodurch die vom modifizierten Subtilisin produzierte immunogene Reaktion schwächer ist als die vom Wildtyp-Subtilisin produzierte immunogene Reaktion.

10. Modifiziertes Subtilisin nach Anspruch 9, worin das Subtilisin eine Sequenzidentität mit Seq.-ID Nr. 1 von zumindest 90 % aufweist.

11. Modifiziertes Subtilisin nach Anspruch 9, worin zumindest eines der Epitope durch Substitution einer oder mehrerer Aminosäuren modifiziert ist.

12. Modifiziertes Subtilisin nach Anspruch 9, worin zumindest eines der Epitope durch Deletion einer oder mehrerer Aminosäuren modifiziert ist.

13. Modifiziertes Subtilisin nach Anspruch 9, worin zumindest eines der Epitope durch Addition einer Aminosäure modifiziert ist.

14. Modifiziertes Subtilisin nach Anspruch 9, worin das modifizierte Subtilisin in einem Organismus des Genus Bacillus exprimiert ist.

15. Isolierte Nucleinsäure, die für ein modifiziertes Subtilisin nach Anspruch 9 kodiert.

16. Expressionsvektor, der eine Nucleinsäure nach Anspruch 16 umfasst.

17. Wirtszelle, die mit einem Expressionsvektor nach Anspruch 17 transformiert ist.

18. Zusammensetzung, die aus der aus Reinigungs-, Körperpflege- und Arzneistoffzusammensetzungen bestehenden Gruppe ausgewählt ist und ein modifiziertes Subtilisin nach Anspruch 9 umfasst.

19. Peptid mit einer aus Seq.-ID Nr. 2, Seq.-ID Nr. 90, Seq.-ID Nr. 15 oder Seq.-ID Nr. 30 bestehenden Sequenz.

## Revendications

1. Méthode pour réduire l'immunogénicité d'une subtilisine ayant au moins 80% d'identité de séquences à la SEQ ID NO:1 en modifiant au moins un épitope à cellule-T de ladite subtilisine sélectionné dans le groupe consistant en SEQ ID NO:2, SEQ ID NO:90, SEQ ID NO:15, SEQ ID NO:30 et SEQ ID NO:40.

2. Méthode selon la revendication 1, dans laquelle la subtilisine a au moins 90% d'identité de séquence à la SEQ ID NO:1.

3. Méthode selon la revendication 1, dans laquelle la subtilisine a la séquence de SEQ ID NO:1.

4. Méthode selon l'une quelconque des revendications précédentes, comprenant les étapes de:

    (a)obtenir le gène codant pour la subtilisine;
    (b) modifier la séquence d'ADN du gène;
    (c) cloner la séquence d'ADN modifiée codant pour une substilisine modifiée en un vecteur d'expression;
    (d) transformer une cellule hôte avec le vecteur d'expression; et
    (e) exprimer la subtilisine modifiée.

5. Méthode selon l'une quelconque des revendications précédentes, dans laquelle ladite subtilisine est modifiée par la substitution d'un ou de plusieurs acides aminés dans au moins un desdits épitopes.

6. Méthode selon l'une quelconque des revendications précédentes, dans laquelle ladite subtilisine est modifiée par délétion d'un ou de plusieurs acides aminés dans au moins un desdits épitopes.

7. Méthode selon l'une quelconque des revendications précédentes, dans laquelle ladite substilisine est modifiée par l'addition d'un acide aminé à au moins un desdits épitopes.

8. Méthode selon l'une quelconque des revendications précédentes, dans laquelle ladite subtilisine modifiée est exprimée dans un organisme dans le genre *Bacillus.*

9. Subtilisine modifiée ayant au moins 80% d'identité avec SEQ ID NO:1, où ladite subtilisine comprend au moins une modification dans au moins un épitope comprenant une séquence d'acides aminés sélectionnée dans le groupe consistant en SEQ ID NO:2, SEQ ID NO:90, SEQ ID NO:15 et SEQ ID NO:30, par quoi la réponse immunogénique produite par ladite substilisine modifiée est inférieure à ladite réponse immunogénique produite par la substilisine de type sauvage.

10. Subtilisine modifiée selon la revendication 9, où la substilisine possède au moins 90% d'identité de séquence avec la SEQ ID NO:1.

11. Subtilisine modifiée selon la revendication 9, dans laquelle au moins un desdits épitopes est modifié par substitution d'un ou de plusieurs acides aminés.

12. Subtilisine modifiée selon la revendication 9, dans laquelle au moins un desdits épitopes est modifié par la délétion d'un ou de plusieurs acides aminés.

13. Subtilisine modifiée selon la revendication 9, dans laquelle au moins un desdits épitopes est modifié par l'addition d'un acide aminé.

14. Subtilisine modifiée selon la revendication 9, dans laquelle ladite substilisine modifiée est exprimée dans un organisme dans le genre *Bacillus.*

15. Acide nucléique isolé codant pour la subtilisine modifiée de la revendication 9.

16. Vecteur d'expression comprenant l'acide nucléique de la revendication 16.

17. Cellule hôte transformée avec le vecteur d'expression de la revendication 17.

18. Composition sélectionnée dans le groupe consistant en compositions de nettoyage, compositions de soins corporels et composition de soins de santé, comprenant la substilisine modifiée de la revendication 9.

19. Peptide ayant une séquence consistant en SEQ ID NO:2, SEQ ID NO:90, SEQ ID NO:15 ou SEQ ID NO:30.

**FIGURE 1**

1 mmrkksfwlg mltafmlvft mafsdsasaa qpaknvekdy ivgfksgvkt asvkkdiike
61 sggkvdkqfr iinaakakld kealkevknd pdvayveedh vahalaqtvp ygiplikadk
121 vqaqgfkgan vkvavldtgi qashpdlnvv ggasfvagea yntdgnghgt hvagtvaald
181 nttgvlgvap svslyavkvl nssgsgtysg ivsgiewatt ngmdvinmsl ggpsgstamk
241 qavdnayarg vvvvaaagns gssgntntig ypakydsvia vgavdsnsnr
asfssvgael
301 evmapgagvy styptstyat lngtsmasph vagaaalils khpnlsasqv rnrlsstaty
361 lgssfyygkg linveaaaq (SEQ ID NO:1)

## FIGURE 2A

88 Peptides based on the sequence provided in Figure 1 (SEQ ID NO: 1) wherein the amino terminus is printed to the left

| Peptide # | Sequence | SEQ ID NO: |
|---|---|---|
| 1 | AQTVPYGIPLIKADK | 2 |
| 2 | VPYGIPLIKADKVQA | 3 |
| 3 | GIPLIKADKVQAQGF | 4 |
| 4 | LIKADKVQAQGFKGA | 5 |
| 5 | ADKVQAQGFKGANVK | 6 |
| 6 | VQAQGFKGANVKVAV | 7 |
| 7 | QGFKGANVKVAVLDT | 8 |
| 8 | KGANVKVAVLDTGIQ | 9 |
| 9 | NVKVAVLDTGIQASH | 10 |
| 10 | VAVLDTGIQASHPDL | 11 |
| 11 | LDTGIQASHPDLNVV | 12 |
| 12 | GIQASHPDLNVVGGA | 13 |
| 13 | ASHPDLNVVGGASFV | 14 |
| 14 | PDLNVVGGASFVAGE | 15 |
| 15 | NVVGGASFVAGEAYN | 16 |
| 16 | GGASFVAGEAYNTDG | 17 |
| 17 | SFVAGEAYNTDGNGH | 18 |
| 18 | AGEAYNTDGNGHGTH | 19 |
| 19 | AYNTDGNGHGTHVAG | 20 |
| 20 | TDGNGHGTHVAGTVA | 21 |
| 21 | NGHGTHVAGTVAALD | 22 |
| 22 | GTHVAGTVAALDNTT | 23 |
| 23 | VAGTVAALDNTTGVL | 24 |
| 24 | TVAALDNTTGVLGVA | 25 |
| 25 | ALDNTTGVLGVAPSV | 26 |
| 26 | NTTGVLGVAPSVSLY | 27 |
| 27 | GVLGVAPSVSLYAVK | 28 |
| 28 | GVAPSVSLYAVKVLN | 29 |
| 29 | PSVSLYAVKVLNSSG | 30 |
| 30 | SLYAVKVLNSSGSGT | 31 |
| 31 | AVKVLNSSGSGTYSG | 32 |
| 32 | VLNSSGSGTYSGIVS | 33 |
| 33 | SSGSGTYSGIVSGIE | 34 |
| 34 | SGTYSGIVSGIEWAT | 35 |
| 35 | YSGIVSGIEWATTNG | 36 |
| 36 | IVSGIEWATTNGMDV | 37 |

# FIGURE 2B

| | | |
|---|---|---|
| 37 | GIEWATTNGMDVINM | 38 |
| 38 | WATTNGMDVINMSLG | 39 |
| 39 | TNGMDVINMSLGGPS | 40 |
| 40 | MDVINMSLGGPSGST | 41 |
| 41 | NMSLGGPSGSTAMK | 42 |
| 42 | SLGGPSGSTAMKQAV | 43 |
| 43 | GPSGSTAMKQAVDNA | 44 |
| 44 | GSTAMKQAVDNAYAR | 45 |
| 45 | AMKQAVDNAYARGVV | 46 |
| 46 | QAVDNAYARGVVVVA | 47 |
| 47 | DNAYARGVVVVAAAG | 48 |
| 48 | YARGVVVVAAAGNSG | 49 |
| 49 | GVVVVAAAGNSGSSG | 50 |
| 50 | VVAAAGNSGSSGNTN | 51 |
| 51 | AAGNSGSSGNTNTIG | 52 |
| 52 | NSGSSGNTNTIGYPA | 53 |
| 53 | SSGNTNTIGYPAKYD | 54 |
| 54 | NTNTIGYPAKYDSVI | 55 |
| 55 | TIGYPAKYDSVIAVG | 56 |
| 56 | PAKYDSVIAVGAVD | 57 |
| 57 | KYDSVIAVGAVDSNS | 58 |
| 58 | SVIAVGAVDSNSNRA | 59 |
| 59 | AVGAVDSNSNRASFS | 60 |
| 60 | AVDSNSNRASFSSVG | 61 |
| 61 | SNSNRASFSSVGAEL | 62 |
| 62 | NRASFSSVGAELEVM | 63 |
| 63 | SFSSVGAELEVMAPG | 64 |
| 64 | SVGAELEVMAPGAGV | 65 |
| 65 | AELEVMAPGAGVYST | 66 |
| 66 | EVMAPGAGVYSTYPT | 67 |
| 67 | APGAGVYSTYPTSTY | 68 |
| 68 | AGVYSTYPTSTYATL | 69 |
| 69 | YSTYPTSTYATLNGT | 70 |
| 70 | YPTSTYATLNGTSMA | 71 |
| 71 | STYATLNGTSMASPH | 72 |
| 72 | ATLNGTSMASPHVAG | 73 |
| 73 | NGTSMASPHVAGAAA | 74 |
| 74 | SMASPHVAGAAALIL | 75 |
| 75 | SPHVAGAAALILSKH | 76 |
| 76 | VAGAAALILSKHPNL | 77 |
| 77 | AAALILSKHPNLSAS | 78 |
| 78 | LILSKHPNLSASQVR | 79 |
| 79 | SKHPNLSASQVRNRL | 80 |

**FIGURE 2C**

| | | |
|---|---|---|
| 80 | PNLSASQVRNRLSST | 81 |
| 81 | SASQVRNRLSSTATY | 82 |
| 82 | QVRNRLSSTATYLGS | 83 |
| 83 | NRLSSTATYLGSSFY | 84 |
| 84 | SSTATYLGSSFYYGK | 85 |
| 85 | ATYLGSSFYYGKGLI | 86 |
| 86 | LGSSFYYGKGLINVE | 87 |
| 87 | SFYYGKGLINVEAAA | 88 |
| 88 | FYYGKGLINVEAAQ | 89 |

Fig. 3

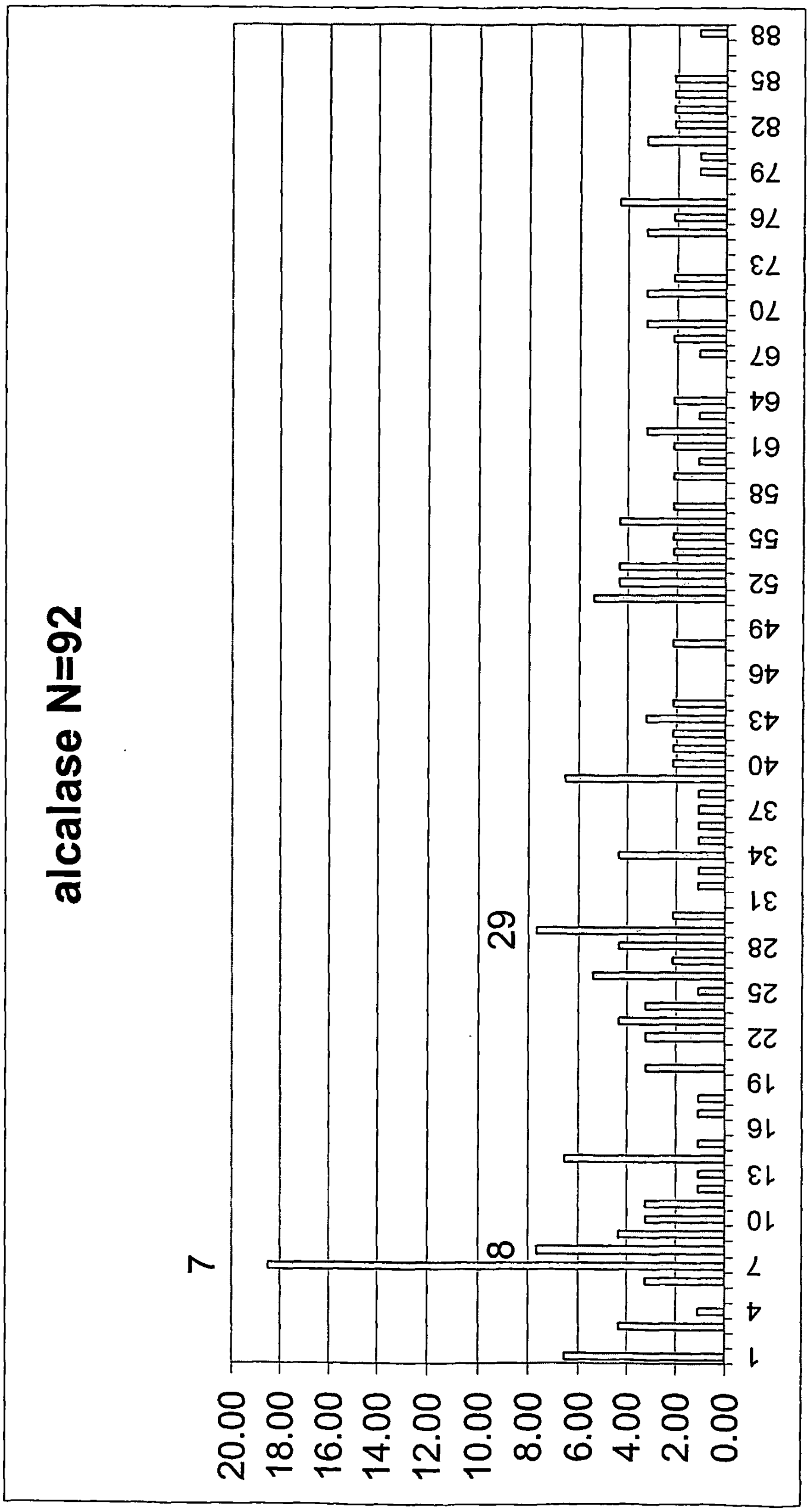
alcalase N=92
7
8
29
20.00
18.00
16.00
14.00
12.00
10.00
8.00
6.00
4.00
2.00
0.00
1
4
7
10
13
16
19
22
25
28
31
34
37
40
43
46
49
52
55
58
61
64
67
70
73
76
79
82
85
88

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9210755 A
- WO 9410191 A
- WO 9617929 A
- WO 9949056 A
- WO 0107578 A
- WO 9953038 A
- WO 9953078 A
- WO 02069232 A
- US 4760025 A
- US RE34606 E
- US 5204015 A
- US 5185258 A
- EP 0328299 A
- WO 8906279 A
- US 5264366 A
- US 4404128 A
- US 4261868 A
- US 216034 A
- EP 134267 A
- US 4533359 A
- EP 344259 A
- WO 9822085 A
- MW 200600
- MW 4252025
- WO 9603964 A
- WO 0024372 A
- WO 9616636 A
- US 4076663 A
- US 5011681 A
- US 4421769 A
- US 3755560 A
- US 3929678 A
- US 4152416 A
- US 4387089 A
- US 5087372 A
- US 5073371 A
- US 5073372 A
- US 4937370 A
- US 4999186 A
- US 5972316 A
- US 5968485 A
- US 5935556 A
- US 5827508 A
- WO 0006110 A
- US 4663157 A
- EP 0680745 A
- US 5487884 A
- WO 9534280 A
- WO 9523780 A
- US 5194639 A
- US 5096700 A
- US 5028414 A
- US 5028415 A
- US 5055305 A
- US 121623 A
- US 08121624 A


### Non-patent literature cited in the description


- **Strickler et al.** *J. Immunother.,* 2000, vol. 23, 654-660
- **Herman et al.** *J. Immunol.,* 1999, vol. 163, 6275-6282
- **Sonderstrup et al.** *Immunol. Rev.,* 1999, vol. 172, 335-343
- **Taneja ; David.** *Immunol. Rev.,* 1999, vol. 169, 67-79
- **Taurog et al.** *Immunol. Rev.,* 1999, vol. 169, 209-223
- **Cosgrove et al.** *Cell,* 1991, vol. 66, 1051-1066
- **Grusby et al.** *Proc. Natl. Acad. Sci.,* 1993, vol. 90, 3913-3917
- **Singleton ; Sainsbury.** Dictionary of Microbiology and Molecular Biology. John Wiley and Sons, 1994
- **Hale ; Marham.** The Harper Collins Dictionary of Biology. Harper Perennial, 1991
- **Jacobs et al.** *Nucleic Acids Res.,* 1985, vol. 13, 8913-8926
- **Smith et al.** *J. Biol. Chem.,* 1968, vol. 243, 2184-2191
- **Moeller.** *Immunol. Rev.,* 1987, vol. 98, 187
- **Smith ; Waterman.** *Adv. Appl. Math.,* 1981, vol. 2, 482
- **Needleman ; Wunsch.** *J. Mol. Biol.,* 1970, vol. 48, 443
- **Pearson ; Lipman.** *Proc. Natl. Acad. Sci. USA,* 1988, vol. 85, 2444
- **Devereux et al.** *Nucl. Acid Res.,* 1984, vol. 12, 387-395
- **Feng ; Doolittle.** *J. Mol. Evol.,* 1987, vol. 35, 351-360

- **Higgins ; Sharp.** *CABIOS,* 1989, vol. 5, 151-153
- **Altschul et al.** *J. Mol. Biol.,* 1990, vol. 215, 403-410
- **Karlin et al.** *Proc. Natl. Acad. Sci. USA,* 1993, vol. 90, 5873-5787
- **Altschul et al.** *Meth. Enzymol.,* 1996, vol. 266, 460-480
- **Henikoff ; Henikoff.** *Proc. Natl. Acad. Sci. USA,* 1989, vol. 89, 10915
- **Henikoff et al.** *Proc. Natl. Acad Sci. USA,* 1989, vol. 89, 10915
- **Karin et al.** *Proc. Natl Acad. Sci USA,* 1993, vol. 90, 5873
- **Higgins et al.** *Gene,* 1988, vol. 73, 237-244
- **Pearson et al.** *Proc. Natl. Acad. Sci. USA,* 1988, vol. 85, 2444-2448
- **Chang ; Cohen.** *Mol. Gen. Genet.,* 1979, vol. 168, 111-115
- **Smith et al.** *Appl. and Env. Microbiol.,* 1986, vol. 51, 634
- **Ferrari et al.** Genetics. Plenum Publishing Corporation, 1989, 57-72
- **Sagarin.** Cosmetics, Science and Technology. 1972, vol. 1, 32-43
- *Fed. Reg.,* 25 August 1978, vol. 43 (166), 38206-38269
- CTFA International Cosmetic Ingredient Dictionary and Handbook. The Cosmetic, Toiletry, and Fragrance Association, Inc, 1997, vol. 2, 1672
- Sunscreens: Development. Evaluation, and Regulatory Aspects. Marcel Dekker, Inc, 1990
- **Segarin et al.** Cosmetics Science and Technology. 189
- CTFA International Cosmetic Ingredient Dictionary. 1995, 1026-281103
- **Sayre et al.** *J. Soc. Cosmet. Chem.,* 1990, vol. 41, 103-109
- **Zoller et al.** *Nucl. Acids Res.,* 1982, vol. 10, 6487-6500
- **Yuckenberg et al.** Directed Mutagenesis: A Practical Approach. 1991, 27-48
- **Rothgeb et al.** *J. Am Oil Chem. Soc.,* 1988, vol. 65, 806
- Molecular Biological Methods for Bacillus. John Wiley & Sons, 1990, 92
- **Chang ; Cohen.** *Mol. Gen. Genet.,* 1979, vol. 168, 11-115
- **Harris et al.** *Immunol.,* 1995, vol. 84, 555-561
- **Maillere et al.** *Mol. Immunol.,* 1995, vol. 32, 1073-1080
- **Delmar.** *Anal. Biochem.,* 1979, vol. 94, 316-320
- **Achtstetter.** *Arch. Biochem. Biophys.,* 1981, vol. 207, 445-54
- **Delmar et al.** *Anal. Biochem.,* 1979, vol. 94, 316-320
- **Achtstetter.** *Arch.Biochem.Biophys.,* 1981, vol. 207, 445-454